# EUROPEAN PATENT SPECIFICATION

(11) **EP 2 326 172 B1**
(45) Date of publication and mention of the grant of the patent: **22.10.2014**
(21) Application number: 09806944.6
(22) Date of filing: 07.08.2009
(51) Int. Cl.: A01N 37/18

(54) **ANTIFUNGAL AGENTS**
ANTIMYKOTIKA
AGENTS ANTIFONGIQUES

(30) Priority: 12.08.2008 US 136106 P
(43) Date of publication of application: 01.06.2011
(73) Proprietor: Scynexis, Inc., Durham, NC 27713 (US)
(72) Inventor: GREENLEE, Mark, L., Rahway, NJ 07065 (US); WILKENING, Robert, Rahway, NJ 07065 (US); APGAR, James, Rahway, NJ 07065 (US); WILDONGER, Kenneth, James, Rahway, NJ 07065 (US); MENG, Dongfang, Rahway, NJ 07065 (US); PARKER, Dann, L, Jr., Rahway, NJ 07065 (US)
(74) Representative: Harris, Jennifer Lucy
(86) International application number: PCT/US2009/004557
(87) International publication number: WO 2010/019203

(56) References cited:
- WO-A1-97/27860
- WO-A1-2007/127012
- WO-A2-03/086271
- WO-A2-2007/126900
- RUGE ET AL.: 'Current state of three-dimensional characterisation of antifungal targets and its use for molecular modelling in drug design' INTEMATIONAL JOUMAL OF ANTIMICROBIAL AGENTS vol. 26, 2005, pages 427 - 441, XP025325958

## Description

The claimed subject matter was made as a result of activities undertaken within the scope of a joint research agreement between Merck & Co., Inc. and Scynexis, Inc.

### FIELD OF THE INVENTION

The claimed subject matter relates to novel compounds and pharmaceutically acceptable salts, hydrates and prodrugs thereof, compositions containing such compounds, synthesis of such compounds, and use of such compounds as antifungal agents and/or inhibitors of (1,3)-β-D-glucan synthesis. The compounds described herein are derivatives of enfumafungin. The novel compounds of this disclosure, their pharmaceutically acceptable salts, hydrates and prodrugs, and compositions comprising such compounds, salts, hydrates and/or prodrugs, are useful for treating and/or preventing antifungal infections and associated diseases and conditions.

### BACKGROUND OF THE INVENTION

Fungal infection is a major healthcare problem, and the incidence of hospital-acquired fungal diseases continues to rise. Severe systemic fungal infection in hospitals (such as candidiasis, aspergillosis, histoplasmosis, blastomycosis and coccidioidomycosis) is commonly seen in neutropaenic patients following chemotherapy and in other oncology patients with immune suppression, in patients who are immune-compromised due to Acquired Immune Deficiency Syndrome (AIDS) caused by HIV infection, and in patients in intensive care. Systemic fungal infections cause ~25% of infection-related deaths in leukaemics. Infections due to Candida species are the fourth most important cause of nosocomial bloodstream infection. Serious fungal infections may cause 5-10% of deaths in patients undergoing lung, pancreas or liver transplantation. Treatment failures are still very common with all systemic mycoses. Secondary resistance also arises. Thus, there remains an increasing need for effective new therapy against mycotic infections.

Enfumafungin is a hemiacetal triterpene glycoside that is produced in fermentations of a *Hormonema spp.* associated with living leaves of *Juniperus communis* (U.S. Pat. No. 5,756,472; Pelaez et al., Systematic and Applied Microbiology, 23:333-343, 2000; Schwartz et al., JACS, 122:4882-4886, 2000; Schwartz, R.E., Expert Opinion on Therapeutic Patents, 11(11):1761-1772, 2001). Enfumafungin is one of the several triterpene glycosides that have *in vitro* antifungal activities. The mode of the antifungal action of enfumafungin and other antifungal triterpenoid glycosides was determined to be the inhibition of fungal cell wall glucan synthesis by their specific action on (1,3)-β-D-glucan synthase (Onishi et al., Antimicrobial Agents and Chemotherapy, 44:368-377, 2000; Pelaez et al., Systematic and Applied Microbiology, 23:333-343, 2000). 1,3-β-D-Glucan synthase remains an attractive target for antifungal drug action because it is present in many pathogenic fungi which affords broad antifungal spectrum and there is no mammalian counterpart and as such, these compounds have little or no mechanism-based toxicity.

Various enfumafungin derivatives have been disclosed, e.g., in International Patent Publication Nos. WO 2007/126900 and WO 2007/127012.

### SUMMARY OF THE INVENTION

The present invention relates to enfumafungin derivatives. These compounds, or pharmaceutically acceptable salts thereof, are useful in the inhibition of (1,3)-β-D-glucan synthase and are useful in the prevention or treatment of mycotic infections caused by one or more of various pathogens including, but are not limited to, *Aspergillus, Cryptococcus, Candida, Mucor, Actinomyces, Histoplasma, Dermatophyte, Malassezia, Fusarium,* and *Pneumocystis carinii.* In particular, the present invention includes a compound of Formula **I**, or a pharmaceutically acceptable salt thereof: 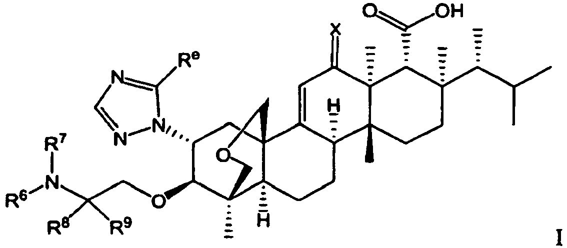 wherein:
X is O or H, H;
R^{e} is C(O)NR^{f}R^{g} or a 6-membered ring heteroaryl group containing 1 or 2 nitrogen atoms wherein the heteroaryl group is optionally mono-substituted on a ring carbon with fluoro or chloro or on a ring nitrogen with oxygen;
R^{f}, R^{g}, R⁶ and R⁷ are each independently hydrogen or C₁-C₃ alkyl;
R⁸ is C₁-C₄ alkyl, C₃-C₄ cycloalkyl or C₄-C₅ cycloalkyl-alkyl;
R⁹ is methyl or ethyl;
R⁸ and R⁹ are optionally taken together to form a 6-membered saturated ring containing 1 oxygen atom.

These compounds are potent antifungal agents useful against pathogens associated with human and agricultural fungal infections.

Additional aspects of the invention relate to compositions comprising the compounds of the invention, optionally in the presence of a second therapeutic agent In addition, aspects of the invention relate to methods of preparing a compound of the invention, to methods of preparing compositions of the invention, to methods of treating or preventing fungal infection in patients using a compound of the invention, and to methods of controlling fungal infection in patients using a compound of the invention.

Other embodiments, aspects and features of the present invention are either further described in or will be apparent from the ensuing description, examples and appended claims.

### DETAILED DESCRIPTION OF THE INVENTION

The present invention relates to compounds of Formula (I), and pharmaceutically acceptable salts thereof: 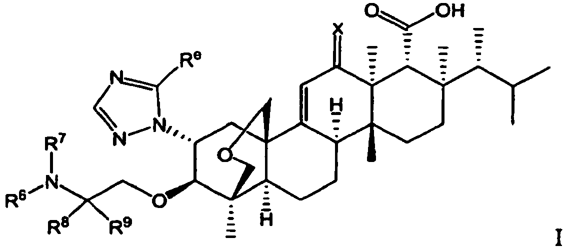 wherein:
X is O or H, H;
R^{e} is C(O)NR^{f}R^{g} or a 6-membered ring heteroaryl group containing 1 or 2 nitrogen atoms wherein the heteroaryl group is optionally mono-substituted on a ring carbon with fluoro or chloro or on a ring nitrogen with oxygen;
R^{f}, R^{g}, R⁶ and R⁷ are each independently hydrogen or C₁-C₃ alkyl;
R⁸ is C₁-C₄ alkyl, C₃-C₄ cycloalkyl or C₄-C₅ cycloalkyl-alkyl;
R⁹ is methyl or ethyl;
R⁸ and R⁹ are optionally taken together to form a 6-membered saturated ring containing 1 oxygen atom.

The present invention also relates to compounds of Formula (Ia), and pharmaceutically acceptable salts thereof: 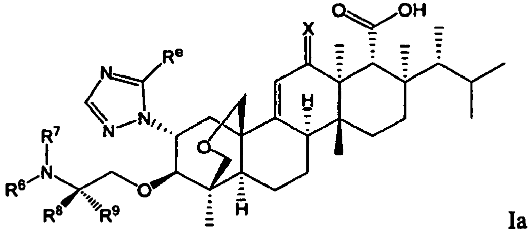 wherein the substituents are as provided for the general formula I.

In a first embodiment, X is H, H, and the other substituents are as provided for the general formula I.

In a second embodiment, R^{e} is either pyridyl or pyrimidinyl optionally mono-substituted on a ring carbon with fluoro or chloro or on a ring nitrogen with oxygen, and the other substituents are as provided in the first embodiment or the general formula I.

In a third embodiment, R^{e} is 4-pyridyl and the other substituents are as provided in the first embodiment or the general formula I.

In a fourth embodiment, R^{e} is C(O)NH₂ or C(O)NH(C₁-C₃ alkyl) and the other substituents are as provided in the first embodiment or the general formula I.

In a fifth embodiment, R⁸ is C₁-C₄ alkyl and R⁹ is methyl; and the other substituents are as provided in the first to fourth embodiments or the general formula I.

In a sixth embodiment, R⁸ is t-butyl, R⁹ is methyl; and the other substituents are as provided in the first to fourth embodiments or the general formula I.

In a seventh embodiment, R⁶ and R⁷ are each independently hydrogen or methyl and the other substituents are as provided in the first to sixth embodiments or the general formula I.

In another embodiment of the invention, the compound of the invention is selected from the exemplary species depicted in Examples 37 through 262 shown below (as the free base or a pharmaceutically acceptable salt thereof).

Other embodiments of the present invention include the following (where reference to a compound of formula (I) encompasses the various embodiments and aspects described above, as well as their pharmaceutically acceptable salts):
(a) A composition comprising a compound of Formula (I) and a carrier, adjuvant, or vehicle;
(b) A pharmaceutical composition comprising a compound of Formula (I) and a pharmaceutically acceptable carrier, adjuvant, or vehicle;
(c) The pharmaceutical composition of (b), further comprising a second therapeutic agent;
(d) The pharmaceutical composition of (c), wherein the second therapeutic agent is an azole, a polyene, a purine or pyrimidine nucleotide inhibitor, a pneumocandin or echinocandin derivative, a protein elongation factor inhibitor, a chitin inhibitor, a mannan inhibitor, a bactericidal/permeability-inducing (BPI) protein product, or an immunomodulating agent;
(e) The pharmaceutical composition of (d), wherein the second therapeutic agent is itraconazole, ketoconazole, miconazole, fluconazole, voriconazole, posaconazole, amphotericin B, flucytosine, anidulafungin, micafungin, or caspofungin;
(f) A pharmaceutical combination which is (1) a compound of Formula (I) and (2) a second therapeutic agent, wherein the compound of Formula (I) and the second therapeutic agent are each employed in an amount that renders the combination effective for treating or preventing fungal/bacterial infections;
(g) The combination of (f), wherein the second therapeutic agent is an azole, a polyene, a purine or pyrimidine nucleotide inhibitor, a pneumocandin or echinocandin derivative, a protein elongation factor inhibitor, a chitin inhibitor, a mannan inhibitor, a bactericidal/permeability-inducing (BPI) protein product, or an immunomodulating agent;
(h) The combination of (g), wherein the second therapeutic agent is itraconazole, ketoconazole, miconazole, fluconazole, voriconazole, posaconazole, amphotericin B, flucytosine, anidulafungin, micafungin, or caspofungin;
(i) A method of inhibiting (1,3)-β-D-glucan synthase in a subject in need thereof comprising administering to the subject an effective amount of a compound of Formula (I);
(j) A compound of Formula (I) for use in a method of treating or preventing mycotic infections in a subject in need thereof comprising administering to the subject an effective amount of a compound of Formula (I);
(k) The compound of Formula (I) for use in a method of (j), wherein the compound of Formula (I), is administered in combination, either sequentially or concurrently, with a second therapeutic agent effective against fungal/bacterial infections;
(l) The compound of Formula (I) for use in a method of (k), wherein the second therapeutic agent is an azole, a polyene, a purine or pyrimidine nucleotide inhibitor, a pneumocandin or echinocandin derivative, a protein elongation factor inhibitor, a chitin inhibitor, a mannan inhibitor, a bactericidal/permeability-inducing (BPI) protein product, or an immunomodulating agent;
(m) The compound of Formula (I) for use in a method of (1), wherein the second therapeutic agent is itraconazole, ketoconazole, miconazole, fluconazole, voriconazole, posaconazole, amphotericin B, flucytosine, anidulafungin, micafungin, or caspofungin;
(n) A pharmaceutical composition of (b), (c), (d), or (e) or the combination of (f), (g) or (h) for use in a method of inhibiting (1,3)-β-D-glucan synthase in a subject in need thereof; and
(o) A pharmaceutical composition of (b), (c), (d), or (e) or the combination of (f), (g) or (h) for use in a method of treating or preventing mycotic infections in a subject in need thereof.

The present invention also includes a compound of the present invention (i) for use in, (ii) for use as a medicament for, or (iii) for use in the preparation of a medicament for: (a) inhibiting (1,3)-β-D-glucan synthase in a subject in need thereof, or (b) treating or preventing mycotic infections. In these uses, the compounds of the present invention can optionally be employed in combination, either sequentially or concurrently, with one or more therapeutic agents effective against fungal and/or bacterial infections.

In the embodiments of the compound as provided above, it is to be understood that each embodiment may be combined with one or more other embodiments, to the extent that such a combination provides a stable compound and is consistent with the description of the embodiments. It is further to be understood that the embodiments of compositions and methods provided as (a) through (o) above are understood to include all embodiments of the compounds, including such embodiments as result from combinations of embodiments of the compound.

In addition, it is understood that, in the description of embodiments of the compounds as set forth above, indicated substitutions are included only to the extent that the substitutents provide stable compounds consistent with the definition.

Additional embodiments of the invention include the pharmaceutical compositions, combinations and methods set forth in (a)-(o) above and the uses set forth in the preceding paragraph, wherein the compound of the present invention employed therein is a compound of one of the embodiments or aspects of the compounds described above. In all of these embodiments as well as those described hereinbelow, the compound may optionally be used in the form of a pharmaceutically acceptable salt or hydrate when appropriate.

The present compounds (including pharmaceutical acceptable salt and/or hydrate forms) have antimicrobial (e.g., antifungal) activities against yeasts and fungi, including one or more *of Acremonium, Absidia* (e.g., *Absidia corymbifera), Alternaria, Aspergillus* (e.g., *Aspergillus clavatus, Aspergilius flavus, Aspergillus jumigatus, Aspergillus nidulans, Aspergillus niger, Aspergillus terreus,* and *Aspergillus versicolor), Bipolaris, Blastomyces* (e.g., *Blastomyces dermatitidis), Blastoschizomyces* (e.g., *Blastoschizomyces capitatus), Candida* (e.g., *Candida albicans, Candida glabrata* (*Torulopsis glabrata*), *Candida guilliermondii, Candida kefyr, Candida krusei, Candida lusitaniae, Candida parapsilosis, Candida pseudotropicalis, Candida stellatoidea, Candida tropicalis, Candida utilis, Candida lipolytica, Candida famata* and *Candida rugosa*), *Cladosporium* (e.g., *Cladosporium carrionii* and *Cladosporium trichloides*), *Coccidioides* (e.g., *Coccidioides immitis*), *Cryptococcus* (e.g., *Cryptococcus neojormans*), *Curvularia, Cunninghamella* (e.g., *Cunninghamella elegans*), *Dermatophyte, Exophiala* (e.g., *Exophiala dermatitidis* and *Exophiala spinifera*), *Epidermophyton* (e.g., *Epidermophyton floccosum*), *Fonsecaea* (e.g., *Fonsecaea pedrosoi), Fusarium* (e.g., *Fusarium solani*), *Geotrichum* (e.g., *Geotrichum candiddum* and *Geotrichum clavatum*), *Histoplasma* (e.g., *Histoplasma capsulatum var. capsulatum*), *Malassezia* (e.g., *Malassezia furfur*), *Microsporum* (e.g., *Microsporum canis* and *Microsporum gypseum*), *Mucor, Paracoccidioides* (e.g., *Paracoccidioides brasiliensis*), *Penicillium* (e.g., *Penicillium marneffei*), *Phialophora, Pityrosporum ovale, Pneumocystis* (e.g., *Pneumocystis carinii*), *Pseudallescheria* (e.g., *Pseudallescheria boydii*), *Rhizopus* (e.g., *Rhizopus microsporus var. rhizopodiformis* and *Rhizopus oryzae*), *Saccharomyces* (e.g., *Saccharomyces cerevisiae*), *Scedosporium* (e.g., *Scedosporium apiosperum*), *Scopulariopsis, Sporothrix* (e.g., *Sporothrix schenckii*), *Trichoderma, Trichophyton* (e.g., *Trichophyton mentagrophytes* and *Trichophyton rubrum*), and *Trichosporon* (e.g., *Trichosporon asahii, Trichosporon beigelii* and *Trichosporon cutaneum*). The present compounds are not only useful against organisms causing systemic human pathogenic mycotic infections, but also useful against organisms causing superficial fungal infections such as *Trichoderma sp.* and other *Candida spp.* The compounds of the present invention are particularly effective against *Aspergilius flavus, Aspergillus fumigatus, Candida albicans, Candida parapsilosis, Cryptococcus neoformans, Saccharomyces cerevisiae,* and *Trichophyton mentagrophytes.*

In view of their antifungal activity, compounds of Formula (I) are useful for the treatment and/or prevention of one or more of a variety of superficial, cutaneous, subcutaneous and systemic mycotic infections in skin, eye, hair, nail, oral mucosa, gastrointestinal tract, bronchus, lung, endocardium, brain, meninges, urinary organ, vaginal portion, oral cavity, ophthalmus, systemic, kidney, bronchus, heart, external auditory canal, bone, nasal cavity, paranasal cavity, spleen, liver, hypodermal tissue, lymph duct, gastrointestine, articulation, muscle, tendon, interstitial plasma cell in lung, blood, and so on.

Therefore, compounds of the present invention are useful for preventing and treating one or more of various infectious diseases, such as dermatophytosis (e.g., trichophytosis, ringworm or tinea infections), athletes foot, paronychia, pityriasis versicolor, erythrasma, intertrigo, fungal diaper rash, candida vulvitis, candida balanitis, otitis externa, candidiasis (cutaneous and mucocutaneous), chronic mucocandidiasis (e.g. thrush and vaginal candidiasis), cryptococcosis, geotrichosis, trichosporosis, aspergillosis, penicilliosis, fusariosis, zygomycosis, sporotrichosis, chromomycosis, coccidioidomycosis, histoplasmosis, blastomycosis, paracoccidioidomycosis, pseudallescheriosis, mycetoma, mycotic keratitis, otomycosis, pneumocystosis, and fungemia. The present compounds may also be used as prophylactic agents to prevent systemic and topical fungal infections. Use as prophylactic agents may, for example, be appropriate as part of a selective gut decontamination regimen in the prevention of infection in immuno-compromised patients (e.g. AIDS patients, patients receiving cancer therapy or transplant patients). Prevention of fungal overgrowth during antibiotic treatment may also be desirable in some disease syndromes or iatrogenic states.

Examples of azoles that may be used in combination with the present compounds include, but are not limited to, fluconazole, voriconazole, itraconazole, ketoconazole, miconazole, ravuconazole, detoconazole, clotrimazole, and posaconazole. Examples of polyenes that may be used in combination with the present compounds include, but are not limited to, amphotericin B, nystatin, liposamal and lipid forms thereof such as ABELCET, AMBISOME, and AMPHOCIL. Examples of purine or pyrimidine nucleotide inhibitors that may be used in combination with the present compounds include, but are not limited to, flucytosine or polyxins such as nikkomycines, in particular nikkomycine Z or nikkomycine X. Another class of therapeutic agents that may be used in combination with the present compounds includes chitin inhibitors. Examples of elongation factor inhibitors that may be used in combination with the present compounds include, but are not limited to, sordarin and analogs thereof. Examples of pneumocandin or echinocandin derivatives that may be used in combination with the present compounds include, but are not limited to, cilofungin, anidulafungin, micafungin, and caspofungin. Examples of mannan inhibitors that may be used in combination with the present compounds include but are not limited to predamycin. Examples of bactericidal/permeability-inducing (BPI) protein products that may be used in combination with the present compounds include but are not limited to XMP.97 and XMP. 127. Examples of immunomodulators that may be used in combination with the present compounds include, but are not limited to, an interferon, (e.g., IL-1, IL-2, IL-3 and IL-8), defensines, tacrolimus and G-CSF (Granulocyte-colony stimulating factor).

As used herein, the term "alkyl" refers to any linear or branched chain alkyl group having a number of carbon atoms in the specified range. Thus, for example, "C₁₋₆ alkyl" (or "C₁-C₆ alkyl") refers to all of the hexyl alkyl and pentyl alkyl isomers as well as n-, iso-, sec- and t-butyl, n- and isopropyl, ethyl and methyl. As another example, "C₁₋₄ alkyl" refers to n-, iso-, sec- and t-butyl, n- and isopropyl, ethyl and methyl.

The term "cycloalkyl" refers to any cyclic ring of an alkane having a number of carbon atoms in the specified range. Thus, for example, "C₃₋₄, cycloalkyl" (or "C₃-C₄ cycloalkyl") refers to cyclopropyl and cyclobutyl.

The term "cycloalkyl-alkyl" (or equivalently "alkyl-cycloalkyl") as used herein, refers to a system that includes an alkyl portion as described above and also includes a cycloalkyl portion as described above. Attachment to a "cycloalkyl-alkyl" (or "alkyl-cycloalkyl") may be through either the cycloalkyl or the alkyl portion. The specified number of carbon atoms in "cycloalkyl-alkyl" systems refers to the total number of carbon atoms in both the alkyl and the cycloalkyl parts. Examples of C₄-C₅ cycloalkyl-alkyl include but are not limited to methylcyclopropyl, dimethylcyclopropyl, methylcyclobutyl, ethylcyclopropyl, cyclopropylmethyl, cyclopropylethyl and cyclobutylmethyl.

The term "halogen" (or "halo") refers to fluorine, chlorine, bromine and iodine (alternatively referred to as fluoro, chloro, bromo, and iodo).

As used herein, the term "or," as used herein, denotes alternatives that may, where appropriate, be combined.

Unless expressly stated to the contrary, all ranges cited herein are inclusive. For example, a heterocyclic ring described as containing from "1 to 4 heteroatoms" means the ring can contain 1, 2, 3 or 4 heteroatoms. It is also to be understood that any range cited herein includes within its scope all of the sub-ranges within that range. Thus, for example, a heterocyclic ring described as containing from "1 to 4 heteroatoms" is intended to include as aspects thereof, heterocyclic rings containing 2 to 4 heteroatoms, 3 or 4 heteroatoms, 1 to 3 heteroatoms, 2 or 3 heteroatoms, 1 or 2 heteroatoms, 1 heteroatom, 2 heteroatoms, and so forth.

Any of the various cycloalkyl and heterocyclic/heteroaryl rings and ring systems defined herein may be attached to the rest of the compound at any ring atom (i.e., any carbon atom or any heteroatom) provided that a stable compound results. Suitable 5- or 6-membered heteroaromatic rings include, but are not limited to, pyridyl, pyrazinyl, pyrimidinyl, pyridazinyl and triazolyl.

A "stable" compound is a compound which can be prepared and isolated and whose structure and properties remain or can be caused to remain essentially unchanged for a period of time sufficient to allow use of the compound for the purposes described herein (e.g., therapeutic or prophylactic administration to a subject). Reference to a compound also includes stable complexes of the compound such as a stable hydrate.

As a result of the selection of substituents and substituent patterns, certain of the compounds of the present invention can have asymmetric centers and can occur as mixtures of stereoisomers, or as individual diastereomers, or enantiomers. Unless otherwise indicated, all isomeric forms of these compounds, whether isolated or in mixtures, are within the scope of the present invention. Also included within the scope of the present invention are tautomeric forms of the present compounds as depicted.

When any variable occurs more than one time in any constituent or in Formula (I) or in any other formula depicting and describing compounds of the invention, its definition on each occurrence is independent of its definition at every other occurrence. Also, combinations of substituents and/or variables are permissible only if such combinations result in stable compounds.

The term "substituted" includes mono- and poly-substitution by a named substituent to the extent such single and multiple substitution (including multiple substitution at the same site) is chemically allowed. Unless expressly stated to the contrary, substitution by a named substituent is permitted on any atom in a ring (e.g., an aryl, a cycloalkyl, a heteroaryl, or a heterocyclyl) provided such ring substitution is chemically allowed and results in a stable compound.

A bond terminated by a wavy line is used herein to signify the point of attachment of a substituent group or partial structure. This usage is illustrated by the following example:

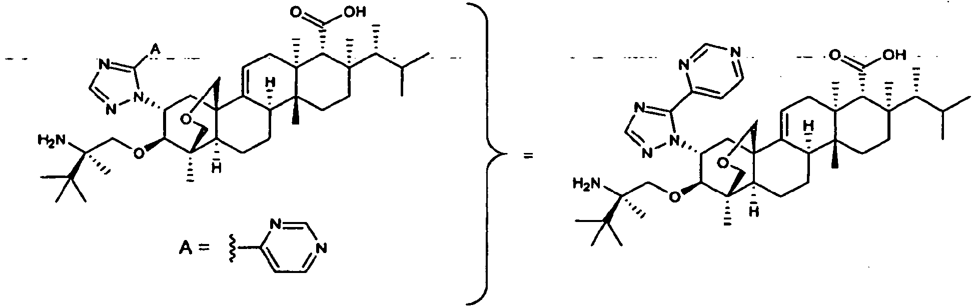

The compounds of this invention are also useful in the preparation and execution of screening assays for antifungal compounds. For example, the compounds of this invention are useful for isolating mutants, which are excellent screening tools for more powerful antifungal compounds.

All compounds of the present invention may be administered in the form of "pharmaceutically acceptable salts" or hydrates as appropriate. Other salts may, however, be useful in the preparation of the compounds according to the invention or of their pharmaceutically acceptable salts. For example, when the compounds of the present invention contain a basic amine group, they may be conveniently isolated as trifluoroacetic acid salts (e.g. following HPLC purification). Conversion of the trifluoroacetic acid salts to other salts, including pharmaceutically acceptable salts, may be accomplished by a number of standard methods known in the art. For example, an appropriate ion exchange resin may be employed to generate the desired salt. Alternatively, conversion of a trifluoroacetic acid salt to the parent free amine may be accomplished by standard methods known in the art (e.g. neutralization with an appropriate inorganic base such as NaHCO₃). Other desired amine salts may then be prepared in a conventional manner by reacting the free base with a suitable organic or inorganic acid. Representative pharmaceutically acceptable quaternary ammonium salts include the following: hydrochloride, sulfate, phosphate, carbonate, acetate, tartrate, citrate, malate, succinate, lactate, stearate, fumarate, hippurate, maleate, gluconate, ascorbate, adipate, gluceptate, glutamate, glucoronate, propionate, benzoate, mesylate, tosylate, oleate, lactobionate, laurylsulfate, besylate, caprylate, isetionate, gentisate, malonate, napsylate, edisylate, pamoate, xinafoate, napadisylate, hydrobromide, nitrate, oxalate, cinnamate, mandelate, undecylenate, and camsylate. Many of the compounds of the invention carry an acidic carboxylic acid moiety, in which case suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

The present invention includes within its scope prodrugs of the compounds of this invention. In general, such prodrugs will be functional derivatives of the compounds of this invention which are readily convertible in vivo into the required compound. Thus, in the methods of treatment of the present invention, the term "administering" shall encompass the treatment of the various conditions described with the compound specifically disclosed or with a compound which converts to the specified compound in vivo after administration to the patient. Conventional procedures for the selection and preparation of suitable prodrug derivatives are described, for example, in "Design of Prodrugs," ed. H. Bundgaard, Elsevier, 1985, which is incorporated by reference herein in its entirety. Metabolites of these compounds include active species produced upon introduction of compounds of this invention into the biological milieu.

The term "administration" and variants thereof (e.g., "administering" a compound) in reference to a compound of the invention mean providing the compound or a prodrug of the compound to the subject in need of treatment. When a compound of the invention or a prodrug thereof is provided in combination with one or more other active agents (e.g., other antifungal/antibacterial agents useful for treating fungal/bacterial infections), "administration" and its variants are each understood to include concurrent and sequential provision of the compound or prodrug and other agents.

As used herein, the term "composition" is intended to encompass a product comprising the specified ingredients, as well as any product which results, directly or indirectly, from combining the specified ingredients.

By "pharmaceutically acceptable" is meant that the ingredients of the pharmaceutical composition must be compatible with each other and not deleterious to the recipient thereof.

The term "subject" (alternatively referred to herein as "patient") as used herein refers to an animal, preferably a mammal, most preferably a human, who has been the object of treatment, observation or experiment.

The term "effective amount" as used herein means that amount of active compound or pharmaceutical agent that elicits the biological or medicinal response in a tissue, system, animal or human that is being sought by a researcher, veterinarian, medical doctor or other clinician. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented or for reducing the likelihood of occurrence. The term also includes herein the amount of active compound sufficient to inhibit (1,3)-β-D-glucan synthase and thereby elicit the response being sought (i.e., an "inhibition effective amount"). When the active compound (i.e., active ingredient) is administered as the salt, references to the amount of active ingredient are to the free acid or free base form of the compound.

For the purpose of inhibiting (1,3)-β-D-glucan synthase or preventing or treating fungal infection, the compounds of the present invention, optionally in the form of a salt or a hydrate, can be administered by any means that produces contact of the active agent with the agent's site of action. They can be administered by conventional means available for use in conjunction with pharmaceuticals, either as individual therapeutic agents or in a combination of therapeutic agents. They can be administered alone, but typically are administered with a pharmaceutical carrier selected on the basis of the chosen route of administration and standard pharmaceutical practice. The compounds of the invention can, for example, be administered by one or more of the following: orally, parenterally (including subcutaneous injections, intravenous, intramuscular, intrastemal injection or infusion techniques), by inhalation (e.g., nasal or buccal inhalation spray, aerosols from metered dose inhalator, and dry powder inhalator), by nebulizer, ocularly, topically, transdermally, or rectally, in the form of a unit dosage of a pharmaceutical composition containing an effective amount of the compound and conventional non-toxic pharmaceutically-acceptable carriers, adjuvants and vehicles. Liquid preparations suitable for oral administration (e.g., suspensions, syrups, elixirs and the like) can be prepared according to techniques known in the art and can employ the usual media such as water, glycols, oils, alcohols and the like. Solid preparations suitable for oral administration (e.g., powders, pills, capsules and tablets) can be prepared according to techniques known in the art and can employ such solid excipients as starches, sugars, kaolin, lubricants, binders, disintegrating agents and the like. Parenteral compositions can be prepared according to techniques known in the art and typically employ sterile water as a carrier and optionally other ingredients, such as a solubility aid. Injectable solutions can be prepared according to methods known in the art wherein the carrier comprises a saline solution, a glucose solution or a solution containing a mixture of saline and glucose. Further description of methods suitable for use in preparing pharmaceutical compositions of the present invention and of ingredients suitable for use in said compositions is provided in Remington's Pharmaceutical Sciences, 20th edition, edited by A. R. Gennaro, Mack Publishing Co., 2000.

The compounds of this invention can be administered, e.g., orally or intravenously, in a dosage range of, for example, 0.001 to 1000 mg/kg of mammal (e.g., human) body weight per day in a single dose or in divided doses. An example of a dosage range is 0.01 to 500 mg/kg body weight per day orally or intravenously in a single dose or in divided doses. Another example of a dosage range is 0.1 to 100 mg/kg body weight per day orally or intravenously in single or divided doses. For oral administration, the compositions can be provided in the form of tablets or capsules containing, for example, 1.0 to 500 milligrams of the active ingredient, particularly 1, 5, 10, 15, 20, 25, 50, 75, 100, 150, 200, 250, 300, 400, and 500 milligrams of the active ingredient for the symptomatic adjustment of the dosage to the patient to be treated. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention also includes processes for making compounds of Formula (I). The compounds of the present invention may be prepared according to the following reaction schemes and examples, or modifications thereof, from starting material enfumafungin. Enfumafungin is a natural product produced from a fungus strain of *Hormonema sp.* (deposited under the Budapest Treaty in the culture collection of the American Type Culture Collection and assigned accession number ATCC 74360) that was isolated from living leaves of an unidentified shrub collected in Navalquejigo, province of Madrid, Spain, as described in U.S. Pat. No. 5,756,472.

The following two examples illustrate the systematic name and numbering conventions employed for the compounds of the present invention.

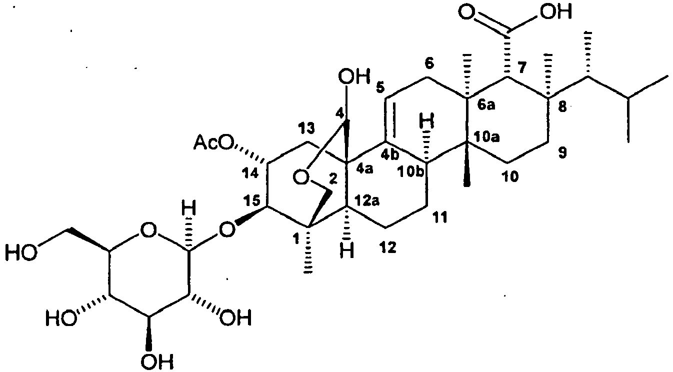

(1*S*,4a*R*,6a*S*,7*R*,8*R*,10a*R*,10b*R*,12a*R*,14*R*,15*R*)-14-(acetyloxy)-8-[(1*R*)-1,2-dimethylpropyl]-15-(β-*D*-glucopyranosyloxy)-1,6,6a,7,8,9,10,10a,10b,11,12,12a-dodecahydro-4-hydroxy-1,6a,8,10a-tetramethyl-4*H*-1,4a-propano-2*H*-phenanthro[1,2-c]pyran-7-carboxylic acid (common name: enfumafungin).

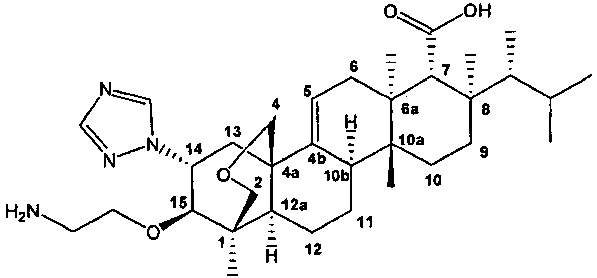

### (1S,4aR,6aS,7R,8R,10aR,10bR,12aR,14R,15R)-15-(aminoethoxy)-8-[(1R)-1,2-dimethylpropyl]-14-(1H-1,2,4-triazol-1-yl)-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid.

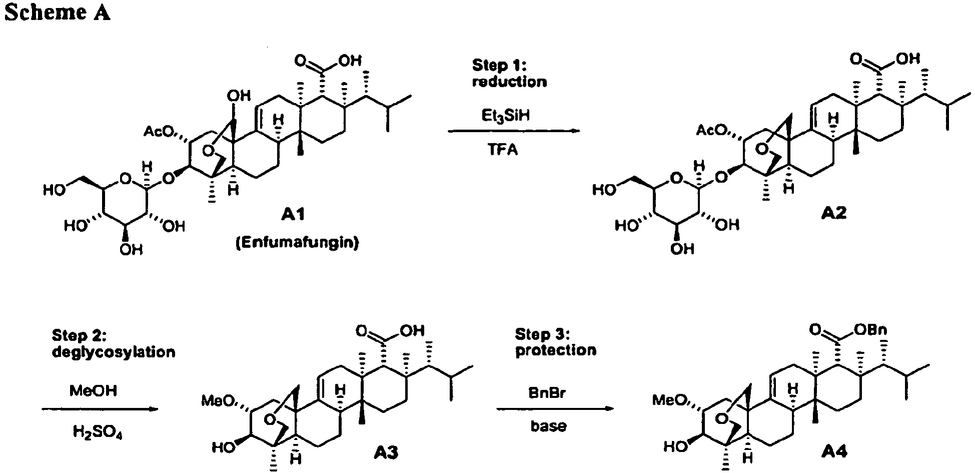

Scheme A illustrates a method for deglycosylation of the natural product enfumafungin and additional modification to prepare the molecule for further elaboration. In a first step, the lactol group of enfumafungin is reduced by treatment with a suitable reducing agent such as triethylsilane under acidic conditions (e.g. trifluoroacetic acid) to give compound **A2**. Removal of the glucose moiety may be accomplished by heating **A2** in methanol in the presence of a strong acid such as sulfuric acid. Under these conditions, the acetoxy group at C14 is also replaced by methoxy to produce the methyl ether compound **A3.** Other methods for deglycosylation of **A2** and related compounds are also known (International Patent Publication No. WO 2007/127012; and Shafiee et al., J. Molecular Catalysis B: Enzymatic, 2001(16), pp. 27-32). Next, selective protection of the carboxylic acid of **A3** may by accomplished by treatment with benzyl bromide in the presence of a suitable base such as sodium bicarbonate or potassium carbonate to give **A4.** Other suitable protecting groups known in the art may also be employed.

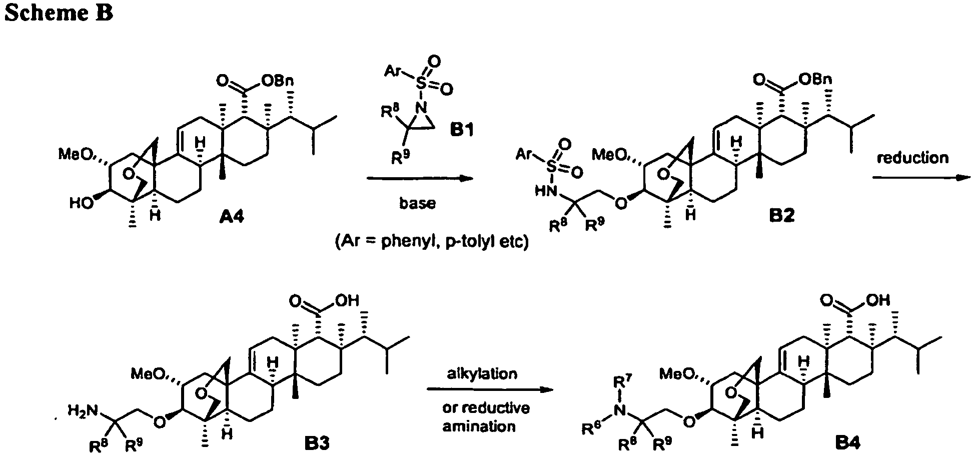

Schemes B to E illustrate methods of introducing the R² substituent on the C15 hydroxy group. Additional methods are also described in International Patent Publication No. WO 2007/127012, herein incorporated by reference in its entirety. In the Schemes the variables R⁶, R⁷, R⁸ and R⁹ are as previously defined or are precursor groups thereto. Additional variables are as defined in the individual schemes. As shown in Scheme B, reaction of **A4** with an *N-*sulfonyl aziridine (**B1**) in the presence of a suitable base such as potassium hydride, sodium hydride or potassium tert-pentylate and optionally in the presence of an appropriate cation complexing agent such as 18-Crown-6 or 15-Crown-5, gives intermediate **B2**. Aziridines **B1** are prepared by methods known in the art (see e.g. Acc. Chem. Res. 2006, 39, 194-206; Tetrahedron 2004, 60, 2701-2743; J. Am. Chem. Soc. 1998, 120, 6844; Org. Lett. 1999, 5, 783-786; Chem. Soc. Rev. 2002, 31, 247; Synthesis 2000, 1347**;** ARKIVOC 2007, 4, 304-311; Tetrahedron: Asymmetry 1997, 8, 1693; Chem. Commun. 2006, 1833-1835) and as exemplified further below. Removal of the *N*-sulfonyl group of **B2** is accomplished by dissolving metal reduction with sodium or lithium in liquid ammonia employing a suitable co-solvent such as dimethoxyethane or tetrahydrofuran. This step also conveniently deprotects the carboxylic acid when it is protected as a benzyl ester, giving intermediate **B3.** It will be appreciated by one skilled in the art that other protecting group strategies may also be employed. Further substitution of the amino group of **B3** may be carried-out at this point by standard methods known in the art such as alkylation or reductive amination to give compound **B4.**

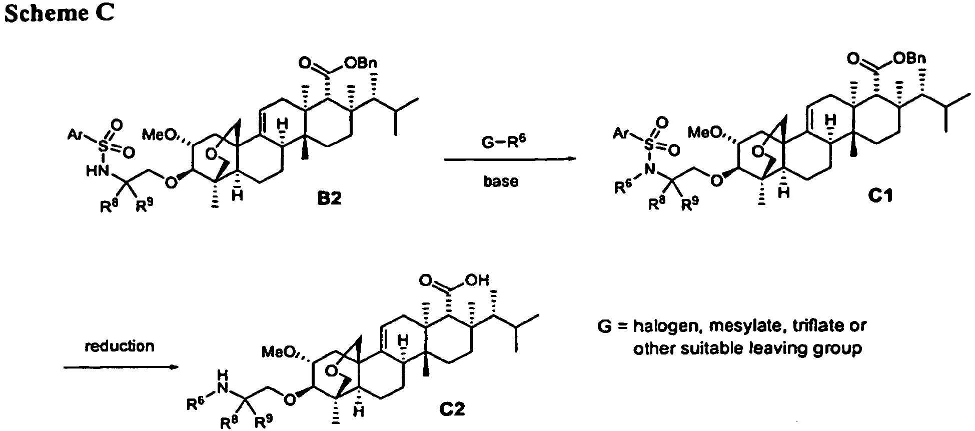

Scheme C illustrates an alternative method of substituting the amino group by alkylating the *N*-sulfonyl intermediate **B2** with an appropriate alkylating agent such as methyl iodide, ethyl iodide or allyl bromide in the presence of a suitable base such as sodium hydride to give **C1**. Dissolving metal reduction as described previously for **B2** then gives **C2.** The synthesis of Scheme C is particularly useful for introducing a single substitution on the aminoether nitrogen. In Scheme C introduction of an R⁶ group is illustrated, but it will be apparent to one skilled in the art that the synthesis would work equally well for introduction of an R⁷ group.

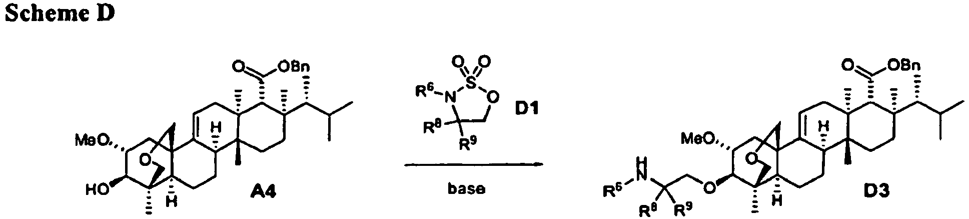

Scheme D describes an additional method for introducing the R² group. Reaction of **A4** with the 5-membered cyclic sulfamidate reagent **D1** gives intermediate **D3.** This reaction is carried-out under conditions analogous to those described in Scheme B for coupling with aziridine **B1.** An acidic aqueous work-up is carried-out which cleaves the initial N-sulfated product to give the amine **D3.** The cyclic sulfamidate reagent **D1** is prepared by methods known in the art (e.g. Tetrahedron 2003, 59, 2581-2616; J. Org. Chem. 2002, 67, 5164-5169) and as exemplified further below. While in Scheme D the synthesis of **D3** with R⁶ substitution on the nitrogen of the aminoether is illustrated, it will be apparent to one skilled in the art that the method would work equally well for the synthesis of a **D3** compound with R⁷ substitution on the nitrogen of the aminoether by employing the appropriately substituted **D1** compound.

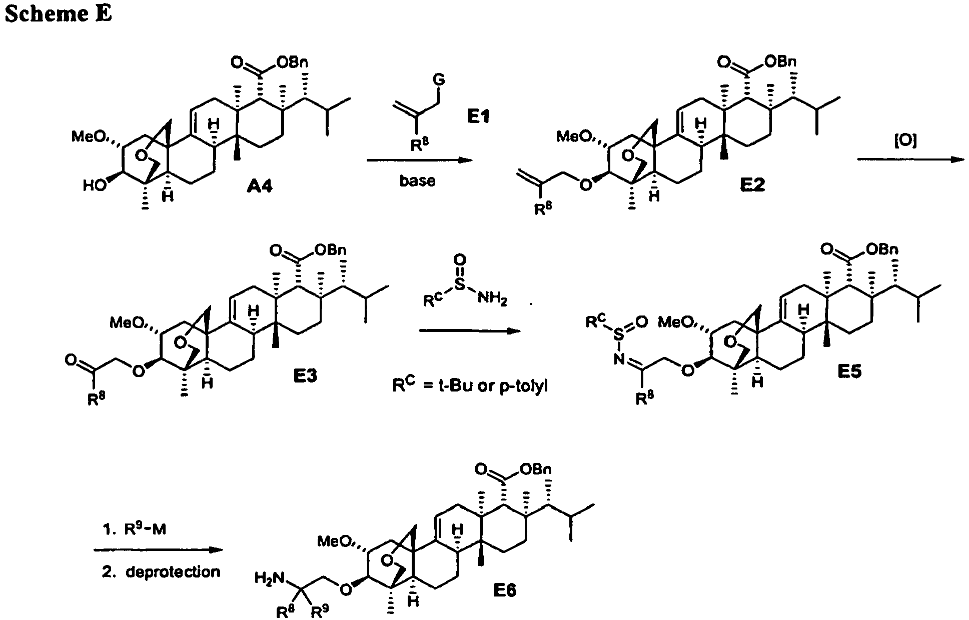

Scheme E illustrates additional methods for introduction of the R² substitution on the C15 hydroxy group. Alkylation **of A4** with an allylic halide or other suitably activated allylic species (**E1**) gives the allylic ether **E2.** Suitable bases for this reaction are sodium hydride or potassium hydride and the like. Oxidative cleavage of the alkenyl group under standard conditions (e.g. OsO₄/NaIO₄) gives the corresponding ketone **E3.** Conversion **of E3** to the sulfinylimine **E5** is accomplished by reaction with an alkyl- or arylsulfinylamide in the presence of a dehydrating agent such as titanium ethoxide. Reaction of **E5** with an alkyllithium reagent (e.g. R⁹Li / Me₃Al) or alkyl Grignard reagent (e.g. R⁹MgBr) followed by acid treatment (e.g. HCl/MeOH) to cleave the N-sulfinyl group gives **E6.** Further substitution of the amino group of **E6** may be carried-out at this point by standard methods known in the art such as alkylation or reductive amination. In one useful variation of this synthesis Scheme, use of an enantiomerically pure alkyl- or arylsulfinylamide reagent for this sequence allows for control of the stereochemistry of the substitution adjacent to the amine in **E6** (see e.g. Acc. Chem. Res. 2002, 35, 984-995). In another useful variation of this Scheme, the roles of R⁸ and R⁹ as illustrated in Scheme E may be reversed.

Schemes G-J illustrate methods for introducing R¹ heterocyclic groups. In the Schemes the various methods are exemplified by starting with intermediate **B4**, but it is understood that the same methods work equally well starting with many other intermediates including but not limited to **B3, C2, D3** and **E6**. In addition, many of the intermediates and final compounds described in International Patent Publication No. WO 2007/127012 may also serve as starting materials for introduction of R¹ heterocyclic groups as described in Schemes G-J. In the Schemes the variables R^{e}, R⁶, R⁷, R⁸ and R⁹ are as previously defined or are precursor groups thereto.

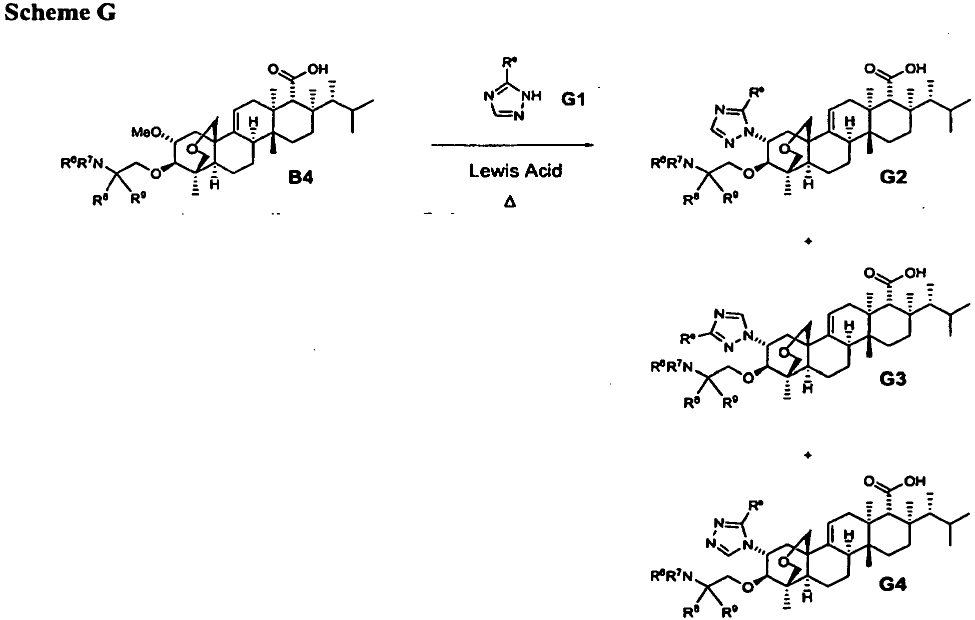

Scheme G illustrates the introduction of a 1,2,4-triazole heterocycle at the C14 position. The displacement reaction between **B4** and the triazole derivative **G1** is promoted by a Lewis acid reagent. Suitable Lewis acid reagents include boron trifluoride diethyl etherate, copper trifluoromethansulfonate, zinc trifluoromethanesulfonate and the like. The reaction is conducted in a non-coordinating aprotic solvent such as 1, 2-dichloroethane at a temperature of between about 20°C and about 100°C. This displacement reaction generally occurs with retention of configuration at C14, possibly due to participation by the proximal bridging ether oxygen.. In addition to the desired compound **G2,** the regioisomeric products **G3** and **G4** may also be formed in this displacement reaction. When undesired isomers are formed, it is often possible and desirable to separate them chromatographically or by other means. The ratio of the isomers may vary depending on the substituent group R^{e}. Depending on the desired R^{e} substituent, the starting triazole compounds **G1** are generally readily available either from commercial sources or through preparation by known literature methods.

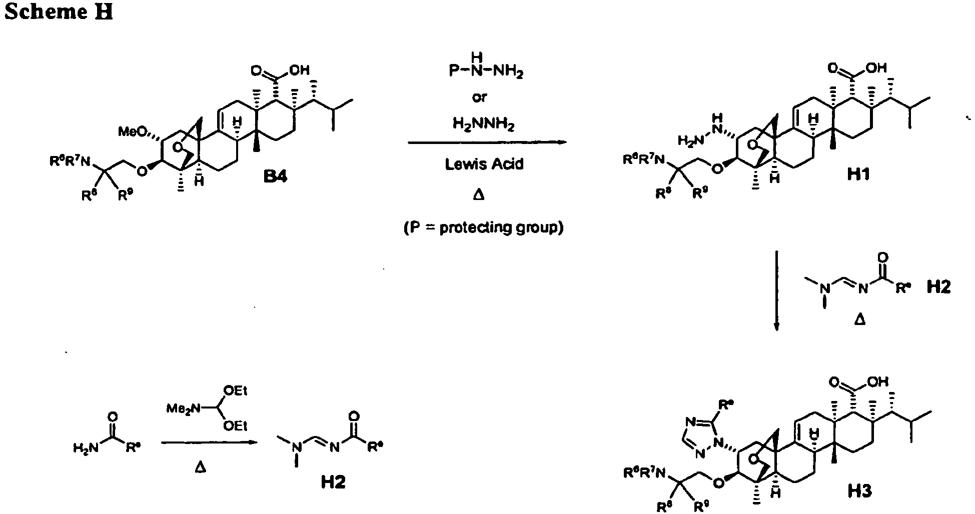

Scheme H illustrates an alternative method for installing a substituted 1,2,4-triazole group. Reaction of intermediate **B4** with anhydrous hydrazine promoted by a Lewis acid such as boron trifluoride diethyl etherate yields the hydrazine intermediate **H1.** The reaction is conducted in a non-coordinating aprotic solvent such as 1,2-dichloroethane at a temperature of between about 20°C and 100°C. Alternatively, a protected hydrazine such as benzyl carbazate (P = benzyloxycarbonyl) or t-butyl carbazate (P = t-butyloxycarbonyl) can be employed in this reaction. With these protecting groups, deprotection occurs under the conditions of the displacement reaction to give **H1** directly with no separate deprotection step being required. Other suitable protecting groups may also be employed which require a separate deprotection step (e.g. phthalimido). Cyclocondensation reaction of **H1** with an acyl amidine derivative **H2** by heating in acetic acid or another suitable solvent at a temperature between about 20°C and about 120°C yields the triazole product **H3.** This method of triazole synthesis is well known in the art (e.g. J. Org. Chem. 1979, 44, 4160-4164; Science of Synthesis, 2004, 13, 603-639). By this method a single triazole regioisomer **H3** is produced. While this method of synthesis is not appropriate for all desired R^{e} groups, it is especially useful for compounds in which R^{e} is heteroaryl. Acyl amidine compounds **H2** are conveniently synthesized as shown in Scheme H by treating the requisite primary amide compound with dimethylformamide diethyl acetal or an equivalent reagent at a temperature between about 20°C and about 120°C (cf., J. Org. Chem. 1979, 44, 4160-4164; J. Am. Chem. Soc. 2006, 128, 16406-16409).

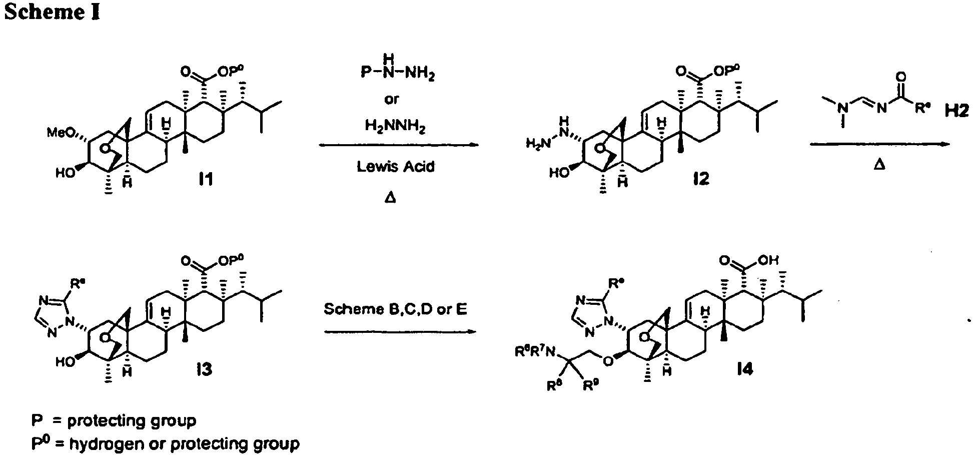

Scheme I describes a variation of the synthesis method of Scheme H which is useful in certain instances. In this case the R¹ heterocyclic group is introduced prior to installation of the R² ether substituent. The starting point for this mode of synthesis is intermediate **I1** which may be synthesized according to Scheme A. Steps 1 and 2 in Scheme I are analogous to Steps 1 and 2 in Scheme H. Thus, reaction of **11** with hydrazine or a protected form of hydrazine promoted by a Lewis acid such as boron trifluoride diethyl etherate gives the hydrazino intermediate **I2.** As described previously, with certain hydrazine protecting groups (e.g. phthalimido) a deprotection step may be required at this stage in the synthetic Scheme. Reaction of **12** with an acyl amidine derivative **H2** as described for step 2 in Scheme H yields the triazole intermediate **I3**. Further elaboration of **I3** to a final compound such as **I4** may then proceed according to the methods described in Schemes B, C, D and E. In Scheme I, protection of the carboxylic acid group may be necessary. The protection step can be conveniently carried-out at the stage of **I1** or **I3.** Various carboxy protecting groups known in the art may be suitable, including benzyl, 4-methoxybenzyl, allyl and the like. Depending on the exact details of the synthesis, a final deprotection step may be required to give compound **I4.**

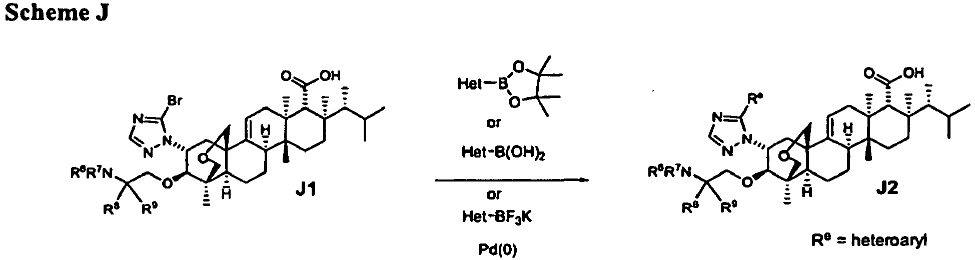

Additional compounds of the present invention may be prepared by further elaboration of a R¹ heterocyclic group once introduced at C14. Examples of this method of synthesis are illustrated in Scheme J. In Scheme J, a bromotriazole compound **J1** is employed as a versatile intermediate. Compound **J1** may be synthesized according to the method of Scheme G. Palladium catalyzed cross-coupling reactions between **J1** and a variety of heteroaryl boronic acid and boronate ester derivatives are possible to give product compounds **J2**. These reactions are generally carried-out in the presence of a base (e.g. cesium carbonate) a palladium catalyst (e.g. palladium (II) acetate) and a phosphine ligand (e.g. 2-dicyclohexylphosphino-2',4',6'-triisopropylbiphenyl) at an elevated temperature (e.g. 50°C to 120°C). Such cross-coupling reactions are well-known in the art and are generically referred to as Suzuki couplings. Many suitable heteroaryl boronic acid and boronate ester derivatives are readily available from commercial sources or are readily prepared by known methods. Other related cross-coupling reactions to give product compounds **J2** are also possible, such as a Stille cross-coupling reaction between **J1** and a heteroaryl stannane derivative or by reaction of **J1** with a heteroaryl trifluoroborate salt (e.g. 5-fluoropyridine-2-trifluoroborate potassium salt).

The antifungal activity of the present compounds can be demonstrated by various assays known in the art, for example, by their glucan synthesis inhibitory activity (IC₅₀), minimum inhibitory concentration (MIC-100) or minimum prominent inhibition (MIC-50) against yeasts and minimum effective concentration (MEC) against filamentous moulds and dermatophytes in a broth microdilution assay, or in vivo anti-*Candida* activity in a mouse (TOKA). Compounds provided in the Examples were generally found to inhibit the growth of *Candida spp.* in the range of <0.03-32 µg/mL or to give an MEC against *Aspergillus fumigatus* in the range of <0.03-32 µg/mL.

### Glucan Synthase Inhibition

The *in vitro* evaluation of glucan synthase inhibitory activity of compounds was measured in a polymerization assay in 96-well format. Each well contained 100 µL of ³H-UDPG at 0.5 mM (6000 to 8000 dpm/nmol), 50 mM HEPES pH 7.5 (Sigma), 10% w/v glycerol (Sigma), 1.5 mg/mL bovine serum albumin (Sigma A 9647. Lot 44H0190), 25 mM KF (Fisher), 1 mM EDTA (Gibco ULTRAPURE), 25 µM GTP-γ-S, enzyme sufficient to give 3 to 6 nmoles incorporation during the 60 min incubation at 22°C, and test compound added from wells in 3-fold serial dilutions in 100% DMSO (1 µL/well). The reaction was stopped by the addition of 100 µL of 20% trichloroacetic acid. Plates were chilled for a minimum of 10 min, and precipitated glucan collected by filtration on GF/C plates (Packard UNIFILTER®-96), washed with 5 cycles of water (about 1 mL/well each cycle) using a Packard FILTERMATE HARVESTER. 40 µL/well scintillation fluid (Packard ULTIMA GOLD TM-XR) was added and the sealed plates counted in a WALLAC BETA counter in top-counting mode at an efficiency of approximately 40%.

Stock solutions were stored at 10 mg/mL in DMSO at -20°C. For each new enzyme preparation, the initial titration performed started at 1 mg/mL, which was prepared by making a 10-fold dilution in DMSO (5 µL to 50 µL). 40 µL of this stock was placed in column 12 of a round-bottomed 96-well microtiter plate. 40 µL DMSO was added to columns 1 to 11 in the same row and ten 3-fold serial dilutions performed, by transferring 20 µL from column 12 to column 11 etc., with 4 mixings before each transfer. No test compound was transferred to from column 2 to column 1. Duplicate aliquots of 1 µL of all 12 dilutions were then transferred to the side walls of a 96-well Bioblock 1.1 mL plate (Fisher brand) to create two rows.

Graphs of the primary data were created in PRISM software (the average of two determinations) using PRISM's curve fitting program (sigmoidal dose response non-linear regression). The amount of compound required to inhibit glucan synthase activity by 50% in this assay (IC₅₀ - ng/mL) was calculated.

Routine analysis was performed with glucan synthase (GS) prepared from *Candida albicans* MY1055 by the following procedure: MY1055 was grown in 10 liters YPD medium (10 g yeast extract, 20 g tryptone, 20 g glucose per liter) with vigorous shaking at 30°C, to early stationary phase. Cells were harvested by centrifugation, the pellet was washed and frozen at -70°C until breakage. Thawed pellets were shaken with an equal volume of breakage buffer (50 mM HEPES pH 7.4, 10% glycerol, 1 mM EDTA, 1 mM PMSF, 1mM DTT) and 4 times their weight of 0.5 mm acid washed glass beads for 2 hours at 4°C. Extent of breakage was assessed visually at 40x magnification. After low speed centrifugation to remove cell debris, the supernatant was centrifuged at 100,000 x g for 60 min. to separate membranes plus ribosomes from cytoplasmic components. Membranes were further washed two additional times with breakage buffer using the same centrifugation conditions and finally suspended in breakage buffer at 25 to 30 mg/mL protein (Biorad) for storage at -70°C. Extraction of GS activity from membranes was performed at a protein concentration of 5 mg/mL in extraction buffer (50mM NaPO₄ pH 7.5, 0.1 M KCl, 0.1M Na citrate, 20% glycerol, 5 µM GTP-γ-S, 1mM DTT, 1 mM PMSF, 3 µg/mL pepstatin) plus 0.25% W1 by gentle mixing at 4°C for 60 min, followed by centrifugation at 100,000 x g for 60 min. After centrifugation, clear supernatant was removed from a pellet consisting of a hard layer usually with small amounts of gelatinous unextracted membranes above it.

Trapping was initiated immediately by 5-fold dilution in trapping buffer (50 mM HEPES pH 7.5, 10 mM KF, 1 mM EDTA, 2 mg/mL BSA) plus 2.5 mM UDPG and 10µM GTP-γ-S. After incubation at 25°C for 60 to 90 minutes, glucan was harvested by low speed centrifugation (3,000 x g, 10 min). The soft pellet was washed 3 times with wash buffer (50 mM HEPES, 20% glycerol, 1 mM EDTA) plus 2.5 mM UDPG and 5 µM GTP-γ-S, once without UDPG, and suspended in about 5 volumes of PE extraction buffer (50 mM HEPES, 30% glycerol, 1 mM EDTA, 20 µM GTP-γ-S, 0.4% CHAPS, 0.08% cholesterol hernisuccinate) using a DOUNCE homogenizer. The suspension was frozen overnight at -70°C, and then centrifuged at 100,000 x g for 10 min. The post-centrifugation supernatant was frozen as aliquots at -70°C for subsequent assays.

### Susceptibility Testing

To each well of a 96-well plate 100 µL of appropriate test medium (example: RPMI-1640 containing 0.165 M MOPS + 3 g/L glutamine w/o sodium bicarbonate or RPMI-1640 containing 0.165 M MOPS + 3 g/L glutamine w/o sodium bicarbonate with 3.2% DMSO or 2X RPMI-1640 containing 0.33 M MOPS + 6 g/L glutamine w/o sodium bicarbonate with 6.4% DMSO for the plates with final concentration of 50% serum) was added.

The test compound was dissolved at concentration of 10 mg/mL in DMSO and diluted 1:78 into appropriate test medium with no DMSO or 1.92% DMSO or 5.12% DMSO. Example: added 25 µL of 10 mg/ml compound stock solution to 1925 µL of RPMI-1640 containing 0.165 M MOPS + 3 g/L glutamine w/o sodium bicarbonate with 1.92% DMSO. The test compound concentration achieved was 128 µg/ml and DMSO concentration of 3.2%. To the first well of each row of appropriate test medium plate 100µL of the compound stock solutions (128 µg/mL) were added. Compounds were serially diluted two-fold across the plate to column 11 (column 12 was the growth control well) and the last 100µL was discarded yielding compound concentrations of 64 to 0.06 µg/mL. For plates with dermatophytes the last 100µL were placed in the first row of a second plate and serial diluted two-fold and yielding compound concentrations of 64-0.00004 µg/mL. Amphotericin B and caspofungin, the control compounds, were prepared as a stock solution of 10 mg/mL in DMSO and prepared in micro-titer plate as stated above for test compounds.

### Yeasts

In the microbroth dilution assay for yeasts, microorganisms *Candida* spp., *Cryptococcus neoformans* (MY2062) and *Saccharomyces cerevisiae* (MY2255) were selected by streaking a yeast culture on SABOURAUD Dextrose Agar (SDA) incubating for 24-48 hours at 35-37°C, thereafter selecting 1 characteristic colony and transferring to a fresh plate and incubating under same conditions. From the regrowth, 3 to 5 colonies were selected and suspended in 5 mL of sterile normal saline (BBL) and adjusted to match the turbidity of a 0.5 McFarland standard using DADE/BEHRING turbidity meter (preferred OD of 0.06 to 0.12). This resulted in a concentration of approximately 1-5 x 10⁶ CFU/mL. The inocula were further diluted 1:1000 into RPMI-1640 containing 0.165 M MOPS + 3 g/L glutamine w/o sodium bicarbonate with 3.2% DMSO. Assay plates previously titrated with test compound in RPMI-1640 containing 0.165 M MOPS + 3 g/L glutamine w/o sodium bicarbonate with 3.2% DMSO were then inoculated with 100 µL/well of this dilution of culture. This resulted in a final organism concentration of 5 x 10² to 2.5 x 10³ CFU/mL and final compound concentrations of 32 to 0.03 µg/mL. In addition *C. albicans* (MY1055) was also tested with heat inactivated (1 hour at 55°C) mouse serum which was filtered twice using 0.22 micron GP EXPRESS PLUS MILLIPORE filtration system. This standardized suspension was diluted 1:1000 into mouse serum. Assay plates previously titrated with drug in 2X RPMI-1640 containing 0.33 M MOPS + 6 g/l glutamine w/o sodium bicarbonate with 6.4% DMSO were then inoculated with 100 µl/well of this dilution of culture. This resulted in a final organism concentration of 5 x 10² to 2.5 x 10³ CFU/mL and final compound concentration of 32 to 0.03 µg/ml and 50% mouse serum. Plates were incubated at 35-37°C and MICs were read at 24 hours for *Candida* and 48 hours for *Cryptococcus neoformans.*

### Filamentous fungi

In the microbroth dilution assay for filamentous fungi *Aspergillus fumigatus* (MF5668) and dermatophyte *Trichophyton mentagrophytes* (MF7004) these microorganisms were grown on Sabouraud Dextrose Agar (SDA) slants at 35-37°C for *Aspergillus fumigatus* and at 30°C for *Trichophyton mentagrophytes* for 7 days prior to use. Inocula for filamentous fungi were prepared by adding 5 mL of sterile normal saline to slant followed by gently scraping the surface of stock slants growth with a sterile DACRON swab suspending the spores (conidia) in saline. Each spore suspension was then transferred to another tube and adjusted to match the turbidity of a 0.5 McFarland standard using the DADE/BEHRING turbidity meter (preferred OD of 0.06-0.09) for *A. fumigatus* and (preferred OD of 0.13-0.17) for dermatophyte *T. mentagrophytes.* This resulted in a concentration of approximately 1-5 x 10⁶ CFU/mL. A spore count was performed on each culture suspension with a hemocytometer to insure the correct inoculum. This standardized suspension for *A. fumigatus* was diluted 1:500 in RPMI-1640 containing 0.165 M MOPS + 3 g/L glutamine w/o sodium bicarbonate with 3.2% DMSO. This standardized suspension for *T. mentagrophytes* was diluted 1:500 in RPMI-1640 containing 0.165 M MOPS + 3 g/L glutamine w/o sodium bicarbonate. Assay plates previously titrated with test compound in either RPMI-1640 containing 0.165 M MOPS + 3 g/L glutamine w/o sodium bicarbonate with 3.2% DMSO or RPMI-1640 containing 0.165 M MOPS + 3 g/L glutamine w/o sodium bicarbonate were then inoculated with 100 µL/well of this dilution. In addition *A. fumigatus* (MF5668) was also tested with heat inactivated human serum which was filtered once using 0.22 micron GP EXPRESS PLUS MILLIPORE filtration system. This standardized suspension was diluted 1:500 in human serum. Assay plates previously titrated with test compound in 2X RPMI-1640 containing 0.33 molar MOPS + 6 g/L glutamine w/o sodium bicarbonate were then inoculated with 100 µl/well of this dilution of culture. Plates were incubated at 35°C and MICs were read at 48 hours for *Aspergillus fumigatus,* and plates incubated at 30°C and MICs were read at 96 hours for Dermatophyte *T. mentagrophytes.*

In the above testing, viable cell counts were performed on 0.5 McFarland samples to verify the CFU/mL. Serial dilutions (1:10) with the 0.5 McFarland were made in saline. One-hundred µl of each dilution (10⁴, 10⁵, 10⁶) was spread onto a SABOURAUD Dextrose Agar (SDA) plates which were then incubated for 24 to 48 or 96 (dermatophytes) hours at 35°C or 30°C. After incubation colonies were counted and recorded. Growth and sterility controls for each organism were also carried out. Column 12 was the growth control and contains no test compound. Row H was not inoculated with organism or test compound and was used as sterility control for each plate.

The minimum inhibitory concentration (MIC-100) for all test compounds is determined to be the lowest concentration of compound at which there was no visible growth as compared to growth control without test compound. The minimum prominent inhibition (MIC-80) in growth is indicated as 80% inhibition in growth compared to growth control without test compound. For *Aspergillus* and dermatophyte *T. mentagrophytes* minimum effective concentration (MEC) was determined as narly morphology of hyphae both macroscopic and microscopic.

### In Vivo Anti-Candida Activity

A disseminated *Candida* infection is induced in DBA/2 mice by the I.V. inoculation of 0.2 mL of a yeast cell suspension containing 3.0 x 10⁴ CFU of *C*. *albicans* MY1055 into the lateral tail vein. Therapy is initiated within 15 to 30 minutes after challenge. Mice are treated with test compound, either (1) I.P., b.i.d. for a total of 2 days, or (2) P.O., b.i.d. for a total of 2 days. For each route of administration and diluent, an appropriate sham-treated control group is included.

Kidneys from euthanized mice (4-5/group) are removed four days after challenge using aseptic techniques, weighed and placed in sterile WHIRL PAK bags containing 5 mL sterile saline. Kidneys are homogenized in the bags, serially diluted in saline and aliquots are plated on SD agar plates. Plates are incubated at 35°C and enumerated after 30 to 48 hours for *C*. *albicans* colony forming units (CFUs). Means from CFU/g of paired kidneys of treated groups are compared to the means from sham-treated controls. Percent sterilization is indicated by the number of mice with no detectable yeast, where the limit of detection (because of the dilution scheme) is 50 yeast cells per pair of kidneys. For data from individual mice where no detectable yeast are recovered from paired kidneys, 9.8 is entered into the MICROSOFT EXCEL spread sheets formula [log₁₀ (5 x raw count)/paired kidney weight)] so that the counts would be one less than the limit of detection (49 cells per pair of kidneys).

Mean log₁₀ yeast CFU/g of paired kidneys are compared to the sham treated controls using Student's t-test (two tailed, unpaired) on MICROSOFT EXCEL. Comparisons are deemed significant at the p = 0.05 level. Mean percent reduction in CFU/g of paired kidneys for treated groups at 4 days following challenge relative to control are computed. A linear trend is typically evident when dose and CFU are both expressed in log₁₀ scale. Inverse regression (2) is subsequently used to estimate ED₉₀ and ED₉₉ values, defined as the doses (mg/kg) that reduced the number of CFU per organ by 90 and 99%, respectively.

Compounds provided in the Examples generally have GS IC₅₀ values less than 500 ng/mL and MIC-100 values against one or more organisms of <0.03-32 µg/mL; however, some compounds may have an IC₅₀ in the range of from about 500 to more than 10,000 ng/mL. Compounds provided in the Examples generally display prominent inhibition of growth in vitro (MIC-50) in the range of <0.03-32 µg/mL and MECs of <0.03-32 µg/mL. As for activity in the disseminated *Candida* infection, useful compounds will lower the number of fungal CFU/g kidney by greater than 1 log₁₀ unit compared to sham treated controls and compounds that lower the CFU/g by 2 log₁₀ units are especially useful.

Example Numbers correspond to the examples described in the Examples section.

| EXAMPLE NUMBER | Candida Albicans GS IC₅₀ (ng/mL) |
|---|---|
| **38** | 13 |
| **40** | 18 |
| **43** | 11 |
| **44** | 3 |
| **46** | 6 |
| **50** | 9 |
| **53** | 31 |
| **89** | 8 |
| **93** | 15 |
| **101** | 13 |
| **112** | 7 |
| **173** | 0.6 |
| **174** | 2 |
| **181** | 8 |
| **182** | 4 |
| **190** | 3 |
| **202** | 0.2 |
| **208** | 0.7 |
| **241** | 1 |
| **256** | 5 |
| **261** | 2 |

The following examples serve only to illustrate the invention and its practice. The examples are not to be construed as limitations on the scope or spirit of the invention.

### Abbreviations

- Boc: t-Butyloxycarbonyl
- Cbz: Benzyloxycarbonyl (also CBz)
- CDCl₃: Deuterio-trichloromethane
- CH₃CN: Acetonitrile
- DCE: Dichloroethane
- DCM: Dichloromethane
- DMAC: Dimethylacetamide
- DMAP: 4-Dimethylaminopyridine
- DMF: Dimethylformamide
- DMSO: Dimethyl sulfoxide
- Et: Ethyl
- EtOAc or EA: Ethyl acetate
- Et₃SiH: Triethylsilane
- H₂: Hydrogen or hydrogen atmosphere
- H₂O: Water
- HOAc: Acetic acid
- HPLC: High pressure liquid chromatography
- H₂SO₄: Sulfuric acid
- HCl: Hydrochloric acid
- K₂CO₃: Potassium carbonate
- LAH: LiAlH₄
- LDA: Lithium diisopropylamide
- MCPBA: meta-Chloroperoxybenzoic acid
- Me: Methyl
- MeOH: Methanol
- MOPS: 3-(N-morpholino)propanesulfonic acid
- NaCl: Sodium chloride
- NaHCO₄: Sodium bicarbonate
- NH₄Cl: Ammonium chloride
- Na₂SO₄: Sodium sulfate
- NMO: 4-methylmorpholine N-oxide
- PMSF: Phenylmethanesulphonylfluoride
- PTAB: Phenyltrimethylammonium tribromide
- RT or r.t.: Room temperature, approximately 25°C
- SiO₂: Silica
- TEA: Triethylamine
- TFA: Trifluoroacetic acid
- THF: Tetrahydofuran
- TLC: Thin layer chromatography
- UDGP: Uridine-diphosphate glucose

### PREPARATION 1

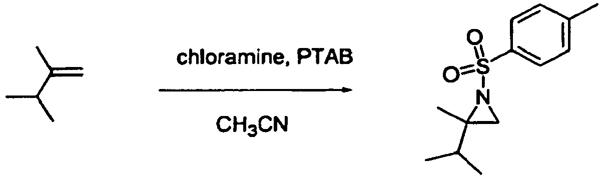

### 2-isopropyl-2-methyl-1-[(4-methylphenyl)sulfonyl]aziridine

To a solution of 2, 3-dimethyl butene (300 ml, 2.42 mol) in 7.8 L of dry acetonitrile was added Chloramine-T (749.9 g, 1.1 eq) portionwise over 90 min. The temperature was maintained at approximately 20 °C. To this reaction mixture was added phenyltrimethylammonium tribromide (91.4 g, 0.1 eq) in 10 g portions over 90 min. The temperature increased to 26 °C during the addition. The reaction mixture was stirred at room temperature for 2 days. The reaction mixture was concentrated down to approximately 15 % of the initial volume and was them filtered, washing the solid with 1 L of acetonitrile. The organic liquid phase was concentrated and the residue dissolved in 2.5 L of EtOAc. The resulting solution was washed twice with water, dried over MgSO₄, and concentrated to give a solid. The crude was purified on a large plug of Celite using gradient elution 5% to 25% EtOAc/ heptanes to afford 317 g of 2-isopropyl-2-methyl-1-[(4-methylphenyl)sulfonyl]aziridine as a solid.

### PREPARATION 2

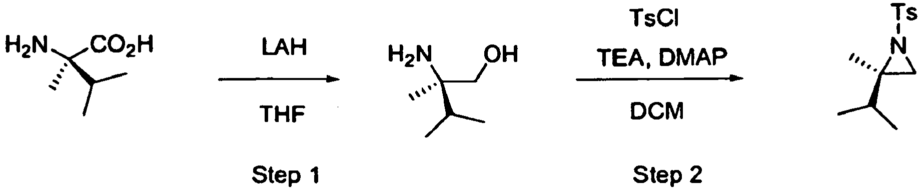

### (2R)-2-isopropyl-2-methyl-1-[(4-methylphenyl)sulfonyl]aziridine

### Step 1

(*R*)-α-methylvaline (8.05 g, 61.4 mmol) was added in small portions to a cold (0°C) solution of LiAlH₄ in THF (1M, 123 mL, 123 mmol), maintaining the reaction temperature below 15°C. The reaction was stirred at 0°C for a few minutes then heated at reflux for 4 h. The reaction mixture was cooled to RT and quenched by addition of sodium sulfate decahydrate / Celite (1:1 by weight) until gas evolution ceased. The reaction mixture was filtered, washing with THF and methanol. The filtrate was concentrated under reduced pressure to provide 4.7 g of amino alcohol as a colorless oil.

### Step 2

To a solution of the amino alcohol product from Step 1 (4.70 g, 40.1 mmol), Et₃N (22.36 mL, 160 mmol) and 4-dimethylaminopyridine (0.490 g, 4.01 mmol) in anhydrous CH₂Cl₂ (200 mL) at 0°C was added p-toluenesulfonyl chloride (22.94 g, 120 mmol) in portions during 10 min. The reaction mixture was stirred at room temperature overnight. The volatiles were evaporated *in vacuo* by rotary evaporation and the residue was partitioned between CH₂Cl₂ and 1N HCl. The organic layer was washed with 1N HCl and dried over Na₂SO₄. The solvent was evaporated and the residue was chromatographed on silica gel using 1:1 CH₂Cl₂/hexane as eluant to remove excess TsCl and then 100% CH₂Cl₂ to elute the product. The title compound was obtained as an off-white solid (5.40 g).

¹H NMR (400 MHz, CDCl₃) δ 0.94 (d, J=6.88 Hz, 3 H), 0.98 (d, J=6.88 Hz, 3 H), 1.49 (quint, J=6.88 Hz, 1 H), 1.59 (s, 3 H), 2.20 (s, 1 H), 2.43 (s, 3 H), 2.60 (s, 1 H), 7.31 (d, J=8.0 Hz, 2 H), 7.83 (d, J=8.0 Hz, 2 H).

### PREPARATION 4

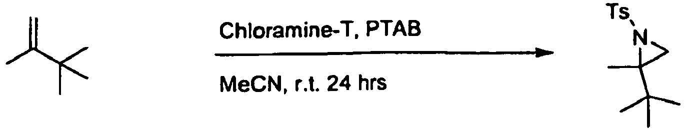

### 2-(1,1-dimethylethyl)-2-methyl-1-[(4-methylphenyl)sulfonyl]aziridine

Chloramine-T trihydrate (10.19g, 36.2 mmol) was placed under high vacuum for 15 hours and the remaining material (8.3g) was suspended in acetonitrile (121 mL) at room temperature under nitrogen. To this suspension was added 2,2, 3-trimethylbut-1-ene (50.6 mL. 362 mmol) followed by phenyltrimethylammonium tribromide (13.6 g, 36.2 mmol) in two roughly equal portions. After twenty hours the reaction mixture was concentrated to half volume and then filtered through a sintered glass funnel. The filtrate was concentrated to half volume again which caused further precipitation. This suspension was filtered washing with acetonitrile and the filtrate concentrated. The resulting material was dissolved/suspended in dichloromethane, filtered and the resulting filtrate was concentrated to an orange oil. This oil was diluted with ethyl acetate and washed with water. The organic phase was dried with MgSO₄, filtered and concentrated. The crude material was purified by column chromatography using a Biotage 65i column eluting with (0-100% EtOAc/hexane) to give 2-(1,1-dimethylethyl)-2-methyl-1-[(4-methylphenyl)sulfonyl]aziridine as a colorless solid (5.2 g).

¹H NMR (CDCl₃, 500 MHz, ppm) δ 0.92 (s, 9H), 1.72 (s, 3H), 2.33 (s, 1H), 2.43 (s, 3H), 2.51 (s, 1H), 7.30 (d, J=8.1 Hz, 2H), 7.83 (d, J=8.1 Hz, 2H)

### PREPARATION 5A

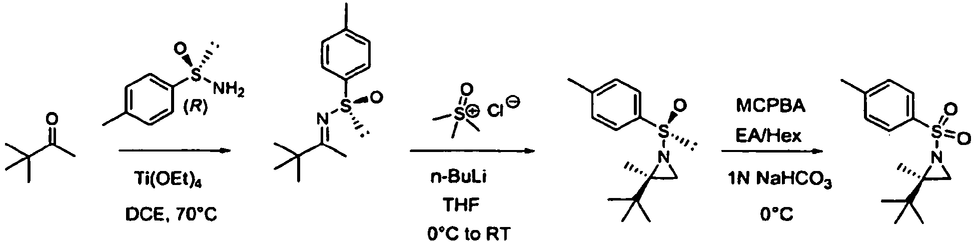

### (2R)-2-(1,1-dimethylethyl)-2-methyl-1-[(4-methylphenyl)sulfonyl]aziridine

### Step 1

To (*R*)-p-toluenesulfinamide (2.00 g, 12.89 nunol) in a 250 mL flask under argon were added dichloroethane (50 mL), t-butylmethylketone (8.1 mL, 64.4 mmol) and Ti(OEt)₄ (13.5 mL, 64.4 mmol). The stirred reaction solution was heated at 70°C overnight. After 21h, the yellow solution was cooled to RT and poured into a vigorously stirred suspension of 15g of Celite in 100 mL of hexane, rinsing the flask with dichloromethane. To the stirred suspension was added 15 mL of H₂O dropwise. After several minutes, the mixture became very thick. Stirring was continued for 5 min. The resulting thick slurry was filtered through a 350 mL coarse sintered filter funnel. The solid was washed twice with 50 mL of 10% dichloromethane /hexane by resuspending the solid by stirring with a spatula and then filtering. The two-phase fitrate was transferred to a separatory funnel and the organic layer was washed with water and brine (sat. acqueous NaCl) and dried over Na₂SO₄. Filtration and concentration by rotary evaporation gave 3.07 g of a yellow oil. Chromatography on an ISCO CombiFlash system (40g silica gel column, 10:90 to 50:50 EA/hex, 20 min gradient, 40 mL/min, detection at 254 nM) gave 2.49 g of a pale yellow oil which solidified upon storage at -20°C.

¹H NMR (CD₂Cl₂, 500MHz, ppm) δ 1.16 (s, 9H), 2.33 (s, 3H), 2.43 (s, 3H), 7.35 (d, J=8.1 Hz), 7.63, (d, J=8.1 Hz).

### Step 2

A mixture of the trimethylsulfoxonium chloride (2.37 g, 18.6 mmol) in THF (35 mL) was sonicated briefly to break-up lumps and then cooled to 0°C and BuLi/hex (2.5 M) was added dropwise. The stirrred reaction mixture was heterogeneous. After 25 min., a solution of the ketimine product from Step 1 (1.45 g, 6.11 mmol) in THF (5+1 mL) was added dropwise to the stirred suspension during 15 min. The resulting white suspension stirred at 0°C for 3h and then allowed to warm to RT overnight. The reaction mixture was quenched with sat. NH₄Cl and partitioned between sat. NH₄Cl and ethyl acetate. The organic phase was washed with water and brine, dried over Na₂SO₄ and evaporated to give 1.539 g of a pale yellow oil.

¹H NMR (CD₂Cl₂, 500MHz, ppm) δ 0.97 (s, 9H), 1.53 (s, 3H), 1.82 (s, 1H), 2.28 (s, 1H), 2.43 (s, 3H), 7.33 (d, J=8.1 Hz), 7.61, (d, J=8.1 Hz).

### Step3

The product fron Step 2 (1.539 g, 6.2 mmol) was dissolved in EA (20 mL) and hexane (40 mL). After addition of 1 M NaHCO₃ solution (30 mL) the two phase reaction mixture was vigorously stirred and cooled to 0°C. Commercial grade MCPBA (2.11 g, ~9 mmol) was added in several portions during 5 min. The reaction was monitored by TLC (30:70 EA/hex). At T = 45 min, the reaction was quenched by addition of 5% Na₂S₂O₃ (30 mL) and the mixture was stirred for several minutes until a negative starch-iodide test was obtained. The reaction mixture was diluted with ethyl acetate and the organic phase was washed with sat. NaHCO₃, H₂O and brine. Drying over Na₂SO₄ and evaporation gave 1.56 g of the title compound as an off-white crystalline solid.

¹H NMR (CD₂Cl₂, 500MHz, ppm) δ 0.94 (s, 9H), 1.71 (s, 3H), 2.37 (s, 1H), 2.46 (s, 3H), 2.51 (s, 1H), 7.36 (d, J=8.1 Hz), 7.82, (d, J=8.1 Hz).

### PREPARATION 5B

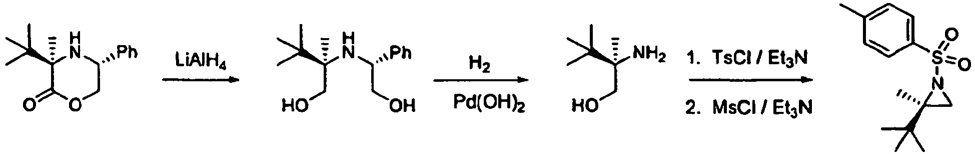

### (2R)-2-(1,1-dimethylethyl)-2-methyl-1-[(4-methylphenyl)sulfonyl]aziridine

### Step 1

To a solution of (3*R*,5*R*)-3-(1,1-dimethylethyl)-3-methyl-5-phenylmorpholin-2-one (Harwood, L. M. et al. *Synlett* **1996,** 1051; 17.3 g, 70 mmol) in THF (1 L) at 0°C was added LiAlH₄ (70 mL of a 2M solution in THF, 140 mmol) dropwise. The mixture was heated at 45°C for 3 h. The reaction mixture was cooled to 0°C and quenched carefully by sequential addition of 6 mL of water, 6 mL of 15% aqueous NaOH, and 18 mL of water. The slurry was stirred vigorously. The solid was removed by suction filtration, and the filter cake was thoroughly washed with ether and CH₂Cl₂. The filtrate was concentrated under reduced pressure to afford the product (17.0 g, 100%) as a viscous oil.

¹H NMR (400 MHz, CDCl₃) δ 0.96 (s, 3 H) 1.02 (s, 9 H) 3.09 (d, *J*=11.32 Hz, 1 H) 3.39 - 3.46 (m, 1 H) 3.46 (d, *J*=11.32 Hz, 1 H) 3.62 (dd, *J*=10.52, 4.71 Hz, 1 H) 4.02 (dd, *J*=9.22, 4.69 Hz, 1 H) 7.08 - 7.44 (m, 5 H).

### Step 2:

To a solution of the product from Step 1 (17.0 g, 70 mmol) in MeOH was added HOAc (5 mL) and palladium hydroxide (5 g of 20 wt% on carbon). The flask was evacuated and filled with hydrogen several times. The suspension was stirred at room temperature under H₂ (balloon, 1 atm) for 3 h. The reaction mixture was filtered through a pad of Celite, the filter cake was washed with additional MeOH, and the filtrate was concentrated under reduced pressure. The residue was purified by flash chromatography, elution with 0-10% MeOH in CH₂Cl₂ with 1% HOAc followed by 100% MeOH with 1% HOAc, to provide 8.84 g of the product as an acetic acid salt. Anhydrous K₂CO₃ (50 g) was added to a solution of the acetate salt (7.03 g, 38.0 mmol) in CH₂Cl₂ (500 mL). The resulting suspension was stirred under nitrogen overnight after which the inorganic salts were removed by suction filtration. The filtrate was concentrated under reduced pressure and the residue was dried azeotropically using PhCH₃ until a constant weight was obtained to give the amino alcohol product (4.98 g, 66% overall).

¹H NMR (400 MHz, CDCl₃) δ 0.93 (s, 9 H) 1.05 (s, 3 H) 3.34 (d, *J*=10.05 Hz, 1 H) 3.45 (d, *J*=10.05 Hz, 1 H).

### Step 3:

A solution of the amino alcohol from Step 2 (4.98 g, 38.0 mmol) in CH₂Cl₂ (200 mL) at 0°C was treated with Et₃N (26 mL, 190 mmol) followed by a solution of *p-*toluenesulfonyl chloride (8.7 g, 45.6 mmol) in CH₂Cl₂ (50 mL) over 40 min. The reaction mixture was stirred at room temperature for 4 d. The mixture was cooled to 0°C after which Et₃N (8.50 mL, 60.8 mmol) and methanesulfonyl chloride (5.88 mL, 76.0 mmol) were added. The mixture was stirred at 0°C for 4 h. The reaction mixture was poured into a 1:1 mixture of saturated aqueous NaCl and water and was extracted with CH₂Cl₂. The organic layer was washed with saturated aqueous NaCl, dried (Na₂SO₄,) filtered, and concentrated under reduced pressure. The residue was purified by flash chromatography, elution with 0 - 50% EtOAc in heptane, to provide the title compound (5.88 g, 58%) as a white solid.

¹H NMR (400 MHz, CDCl₃) δ 0.93 (s, 9 H) 1.73 (s, 3 H) 2.34 (s, 1 H) 2.44 (s, 3 H) 2.52 (s, 1 H) 7.31 (d, *J*=7.96 Hz, 2 H) 7.84 (d, *J*=8.35 Hz, 2 H).

### PREPARATION 9

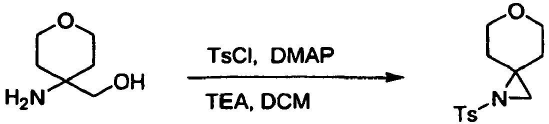

### 1-[(4-methylphenyl)sulfonyl]-6-oxa-1-azaspiro[2.5]octane

A solution of 4-aminotetrahydro-2*H*-pyran-4-methanol (16 g, 122 mmol) in dichloromethane (700 mL) was treated with triethylamine (85 mL, 610 mmol), p-toluenesulfonyl chloride (69.8 g, 366mmol) and DMAP (1490 mg, 12.2 mmol) under nitrogen. After approximately 18 hours at room temperature, the reaction was filtered through a pad of silica gel. The filtrate was concentrated *in vacuo* then purified by chromatography on silica gel (5-20% EtOAc/hexane) to afford intermediate the title compound (12.5g) as a white solid.

¹H NMR (CDCl₃, 500 MHz, ppm) δ 1.92 (m, 2H), 2.08 (m, 2H), 2.47 (s, 3H), 2.52 (s, 2H), 3.76 (m, 2H), 3.99 (m, 2H), 7.35 (d, J=8.0 Hz, 2H), 7.86 (d, J=8.5 Hz, 2H).

### PREPARATION 14

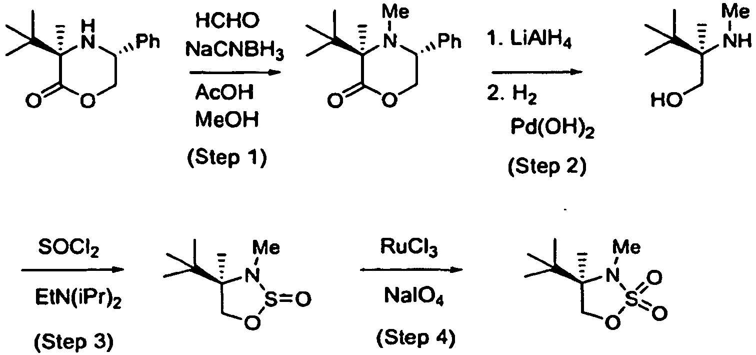

### (4R)-4-(1,1-dimethylethyl)-3.4-dimethyl-1,2,3-oxathiazolidine 2,2-dioxide

### Step 1:

To a solution of compound (3*R*,5*R*)-3-(1,1-dimethylethyl)-3-methyl-5-phenylmorpholin-2-one (100 mg, 0.40 mmol) in MeOH was added formaldehyde (1.40 mL, 37 wt% in water, 16.2 mmol), HOAc (0.14 mL, 2.4 mmol), and sodium cyanoborohydride (100 mg, 1.60 mmol). The reaction mixture was stirred at room temperature overnight, and the solvent was removed under reduced pressure. The residue was partitioned between EtOAc and saturated aqueous NaHCO₃, and the layers were separated. The organic layer was washed with saturated aqueous NaHCO₃, saturated aqueous NaCl, dried (Na₂SO₄), and concentrated under reduce pressure to provide the product (99.7 mg, 95%) as a viscous oil.

¹H NMR (400 MHz, CDCl₃) δ 1.08 (s, 9 H) 1.30 (s, 3 H) 2.28 (s, 3 H) 3.97 (d, *J*=2.54 Hz, 1 H) 4.45 (dd, *J*=10.91, 2.37 Hz, 1 H) 4.89 (dd, *J*=10.88, 3.66 Hz, 1 H) 7.13 - 7.41 (m, 5 H).

### Step 2:

Employing procedures analogous to those described for Steps 1 and 2 of Preparation 5B, the desired amino alcohol was prepared from the product of Step 1.

¹H NMR (400 MHz, CDCl₃) δ 1.08 (s, 9 H) 1.17 (s, 3 H) 1.99 (s, 3 H) 2.63 (s, 3 H) 3.53 (d, *J*=12.35 Hz, 1 H) 3.84 (d, *J*=12.30 Hz, 1 H) 5.32 (br. s., 4 H)

### Step 3:

To a solution of the amino alcohol product from Step 2 (59.4 mg, 0.29 mmol) in CH₂Cl₂ (3 mL) at 0 °C was added N,N-diisopropylethylamine (0.15 mL, 0.87 mmol) and thionyl chloride (21.0 µL, 0.29 mmol), and the resulting solution was stirred at 0 °C for 45 min. The reaction mixture was diluted with CH₂Cl₂ and washed with saturated aqueous NaCl, dried (Na₂SO₄), and concentrated under reduced pressure. The residue was purified by flash chromatography to provide two diastereoisomers (22.1 mg, 40%) as white solids. ISOMER A: ¹H NMR (400 MHz, CDCl₃) δ ppm, 1.05 (s, 9 H) 1.24 (d, *J*=0.63 Hz, 3 H) 2.87 (s, 3 H) 4.12 (d, *J*=8.88 Hz, 1 H) 4.87 (dd, *J*=8.88, 0.68 Hz, 1 H);

ISOMER B: ¹H NMR (400 MHz, CDCl₃) δ ppm 0.92 (s, 9 H) 1.38 (s, 3 H) 2.72 (s, 3 H) 4.42 (d, *J*=9.27 Hz, 1 H) 4.59 (d, *J*=9.23 Hz, 1 H)

### Step 4:

A solution of the two diastereoisomers from Step 3 (15.2 mg, 0.08 mmol) in CH₃CN (0.5 mL) was added to a solution of ruthenium trichloride (1 mg, 0.0008 mmol) and sodium periodate (19 mg, 0.09 mmol) in water (1 mL) and CH₃CN (1 mL). The reaction mixture was stirred at room temperature for 1 h, diluted with EtOAc and washed with water. The aqueous layer was extracted with EtOAc. The combined organic layers were dried (Na₂SO₄), and concentrated under reduced pressure to provide the title compound (13.9 mg, 84%) directly as a white solid.

¹H NMR (400 MHz, CDCl₃) δ 1.00 (s, 9 H) 1.31 (s, 3 H) 2.81 (s, 3 H) 4.03 (d, J=9.32 Hz, 1 H) 4.55 (d, *J*=9.32 Hz, 1 H).

### PREPARATION 15

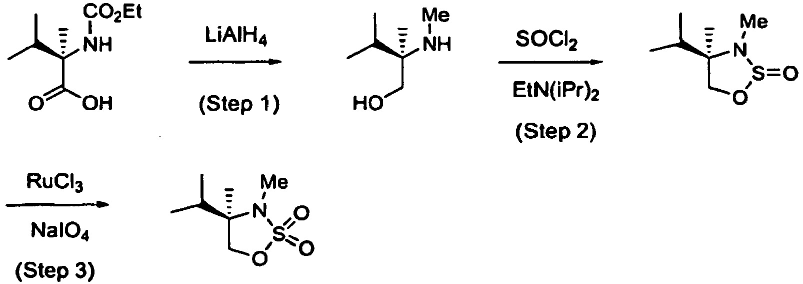

### (4R)-3,4-dimethyl-4-(1-methylethyl)-1,2,3-oxathiazolidine 2,2-dioxide

Starting with (2*R*)-2-{[(ethyloxy)carbonyl]amino}-2,3-dimethylbutanoic acid (prepared as described in J. Org. Chem. 2007, 72, 7469-7472 but employing *D*-tartaric acid for the resolution step) and employing procedures analogous to those described for Preparation 5B Step 1 and Preparation 14 Steps 3 and 4, the title compound was prepared and isolated as a waxy solid.

¹H NMR (400 MHz, CDCl₃) δ: 4.38 (d, *J*=8.8 Hz, 1 H), 4.06 (d, *J*= 8.8 Hz, 1 H), 2.65 (s, 3 H), 1.85 (m, 1 H), 1.32 (s, 3 H), 0.94 (d, *J*= 6.9 Hz, 3 H), 0.89 (d, *J*= 6.9 Hz, 3 H) ppm.

### PREPARATION 20

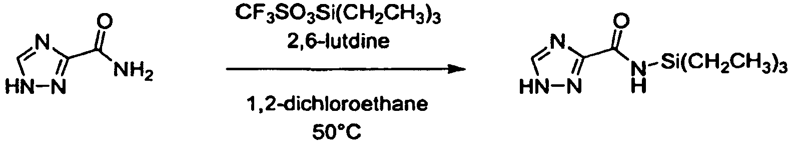

### N-(triethylsilyl)-1H-1,2,4-triazole-3-carboxamide

A stirred mixture of 1*H*-1,2,4-triazole-3-carboxamide (500 mg, 4.46 mmol) in 1,2-dichloroethane (4.46 mL) treated at room temperature with 2,6-lutidine (2.08 mL, 17.84 mmol) and triethylsilyl trifluoromethanesulfonate (3.03 mL, 13.38 mmol) and heated under a nitrogen atmosphere at 50°C. After 4 hours of heating the reaction solution allowed to cool to room temperature and diluted with dichloromethane (25 mL), washed with water (25 mL), 0.5M hydrochloric acid (25 mL), water (25 mL), dried over MgSO₄, filtered, and evaporated to solid. The solid was flash chromatographed (silica gel, 10-65% ethyl acetate:hexane) to give the product as a white solid (683 mg).

¹H NMR (CDCl₃, 500MHz, ppm) 0.89 (q, J=8Hz, 6H, CH₂), 1.03 (t, J=8 Hz, 9H, CH₃), 6.77 (s, 1H, NH), 8.63 (s, 1H, triazole H-5).

LC/MS m/z (positive ion scan) M+1= 227.20

### PREPARATION 21

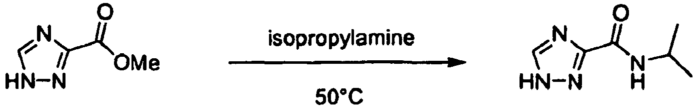

### N-(1-methylethyl)-1H-1,2,4-triazole-3-carboxamide

Methyl 1*H*-1,2,4-triazole-3-carboxylate (3.53 g, 27.8 mmol) and isopropylamine (11 ml, 128 mmol) were combined in a 20 ml vial to give a white suspension. The vial was sealed and heated to 50°C. After 6 days the reaction mixture had become a translucent solid. The vial was cooled to room temperature and unsealed. The solid was dissolved in methanol and transferred to a 200 ml flask. The solvent was evaporated under reduced pressure and the resulting residue was stripped several times with ethanol to removed excess isopropylamine. The residue was stripped with toluene and placed under high vacuum overnight to give the title compound (4.07 g) as a white solid.

¹H NMR (CD₃OD, 600 MHz, ppm) δ 1.25 (d, 6H, 2Me), 4.15-4.22 (m, 1H, CONCH), 8.38 (s, 1H, triazole).

Mass Spectrum: (ESI) *m*/*z* = 155.14 (M+H).

### PREPARATION 26

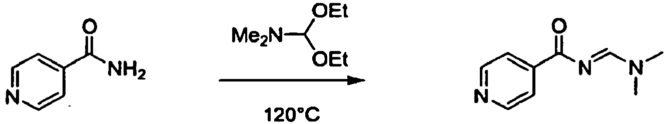

[cf. Y. Lin, et al. J. Org. Chem. 1979, 44, 4160]

### N-[(1E)-(dimethylamino)methylidene)pyridine-4-carboxamide

Isonicotinamide (2.00 g, 16.38 mmol) and N,N-dimethylformamide diethyl acetal (2.8 ml, 16.38 mmol) were combined and heated to 120°C. A short path distillation apparatus was used to collect the ethanol that was liberated during the reaction. The reaction mixture was an orange solution. After 15 minutes, additional N,N-dimethylformamide diethyl acetal (1.0 ml, 5.83 mmol) was added to the reaction mixture. After 1.5 hours, the reaction mixture was cooled to room temperature. The reaction mixture solidified upon cooling and was placed under high vacuum overnight to give the title compound (2.92 g, 16.48 mmol) as a yellow solid.

¹H NMR (CD₃OD, 500 MHz, ppm) δ 3.28 (s, 3H, NMe), 3.29 (s, 3H, NMe), 8.10-8.12 (m, 2H, ArH), 8.66-8.68 (m, 2H, ArH), 8.70 (s, 1H).

Mass Spectrum: (ESI) *m*/*z* = 178.19 (M+H).

### PREPARATION 29

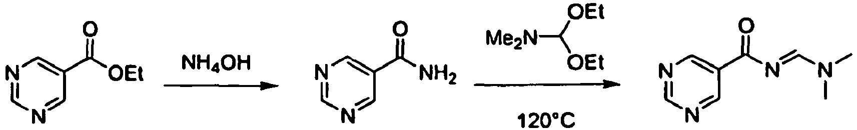

### N-[(1E)-(dimethylamino)methylidene]pyrimidine-5-carboxamide

### Step 1:

Ethyl 5-pyrimidine carboxylate (0.26 ml, 1.982 mmol) and ammonium hydroxide (2.8 ml, 20.13 mmol) were combined in a 25 ml flask to give a hazy solution. The reaction mixture was stirred at room temperature. After 15 minutes, a white precipitate formed, which turned the reaction mixture into a white suspension. After 2.5 hours, LMCS and ¹H NMR showed complete conversion of starting material. The reaction mixture was filtered, rinsing over with water (3 x 1 ml). The filtered solid was air dried overnight to give the product (199.8 mg) as a white solid.

¹H NMR (CD₃OD, 500 MHz, ppm) δ 9.21 (s, 2H), 9.29 (s, 1H).

Mass Spectrum: (ESI) *m*/*z* = 124.12 (M+H).

### Step 2:

The product compound from Step 1 (199.8 mg, 1.623 mmol) and N,N-dimethylformamide diethyl acetal (0.39 ml, 2.276 mmol) were combined and heated to 120°C. A short path distillation apparatus was used to collect the ethanol that was liberated during the reaction. The reaction mixture slowly changed from a yellow suspension to an amber solution. A yellow precipitate formed. After 1.5 hours, the reaction mixture was cooled to room temperature. The reaction mixture solidified upon cooling and was placed under high vacuum for about an hour to give the title compound (284.9 mg) as a yellow solid.

¹H NMR (CD₃OD, 600 MHz, ppm) δ 3.26 (s, 3H, NMe), 3.27 (s, 3H, NMe), 8.70 (s, 1H), 9.23 (s, 1H, ArH), 9.41 (s, 2H, ArH).

Mass Spectrum: (ESI) *m*/*z* = 179.20 (M+H).

### INTERMEDIATE 1

### Benzyl (1S,4aR,6aS,7R,8R,10aR,10bR,12aR,1AR,15R)-8-[(1R)-1,2-dimethylpropyl]-15-hydroxy-14-methoxy-1,6,6a,7,8,9,10,10a,10b,11,12,12a-dodecahydro-1,6a,8,10a-tetramethyl-4H-1,4a-propano-2H-phenanthro[1,2-c]pyran-7-carboxylate

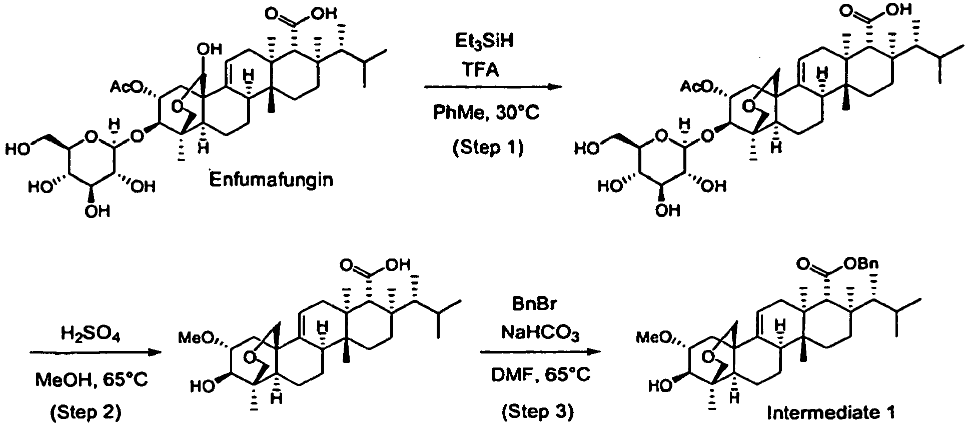

### Step 1:

To a slurry of enfumafungin (90.0 g, 126.9 mmol) in 846 ml of toluene with mechanical stirring at room temperature was added Et₃SiH (202.2 ml, 1269.5 mmol) in one portion. Trifluoroacetic acid (202.4 ml, 2627.8 mmol) was then added dropwise at a rapid rate. Once the trifluoroacetic acid addition was complete, the resulting amber colored solution was allowed to stir at room temperature for 2.5 hours. The TFA/toluene solution was then concentrated to dryness. Fresh toluene (300-500 ml) was added and the mixture was once again concentrated to dryness. The toluene stripping procedure was repeated two additional times. The crude solid was then dried overnight on a high vacuum line to yield 120 g of a purple brown solid. This material was carried on to the next step without additional purification.

### Step 2:

To a solution of the solid from above (120 g crude material, -126.9 mmol) in MeOH (1.27 L) with mechanical stirring, H₂S0₄ (31.2 ml, 585.3 mmol) was added dropwise at a fast rate. Once the addition was complete, the resulting solution was warmed to 65 deg C and was allowed to stir for 4.5 hours. During the course of the reaction a white solid precipitated. The reaction was cooled to room temperature and the white solid was isolated by filtration. The solid was then washed with MeOH (2x200 ml) and CH₃CN (2x200 ml). After drying, 47.91 g white solid was recovered.

Additional material was isolated from the initial filtrate and subsequent washings as follows. The total liquid volume was reduced to 1/3 by evaporation in vacuo. An excess of water was added and a purple white solid precipitated. The solid was filtered, washed with 3:7 MeOH:water (2x100 mL) and CH₃CN (2x100 mL) and dried to give an additional 7.30 g of product as a brownish white solid. The combined yield of product was 55.21 g (86.5%).

### Step 3:

The product from Step 2 (55.21 g, 109.8 mmol), NaHCO₃ (147.5 g, 1756.8 mmol) and benzyl bromide (65.29 ml, 549.0 mmol) were combined in 550 ml DMF with mechanical stirring. The mixture was warmed to 65 deg C and was allowed to stir for 4.5 hours. The DMF was removed in vacuo and the resulting crude material was dissolved in 1 L of 3:2 water/MeOH. The mixture was vigorously stirred for 2-3 hours. During this time a brownish white solid formed. The precipitate was filtered and washed with additional 3:2 water/MeOH (2x250 mL). The solid was then rinsed with heptane and was allowed to air aspirate to initial dryness. The white solid recovered was then transferred to a recrystallizing dish and placed in a vacuum oven at 30°C for four hours to give 52.2 g of white solid.

Additional material was isolated from the water:MeOH and heptane filtrates as follows. The combined solutions were extracted with EtOAc. The combined EtOAc washings were dried over Na₂SO₄ and concentrated to dryness. The resulting material was purified by SiO₂ chromatography (3:7 EtOAc:DCM) to yield an additional 5.42 g of product as a white solid. The total combined yield of Intermediate 1 was 57.6 g (88.5%).

¹H NMR (400MHz, CDCl₃, ppm) δ 0.71-0.74 (m, 6H), 0.78 (d, J=6.83Hz, 3H), 0.80-0.83 (m, 6H), 1.15 (s, 3H), 1.16-1.21 (m, 1H), 1.23 (s, 3H), 1.24-1.29 (m, 2H), 1.32-1.53 (m, 4H), 1.56-1.62 (m, 1H), 1.70-1.81 (m, 3H), 1.87-1.95 (m, 1H), 1.99-2.04 (m, 1H), 2.07-2.16 (m, 1H), 2.30 (d, J=2.25Hz, 1H), 2.40-2.47 (m, 1H), 2.88 (s, 1H), 3.18 (d, J=8.88Hz, 1H), 3.31 (d, J=11.76Hz, 1H), 3.40-3.42 (m, 2H), 3.43 (s, 3H), 3.77 (d, J=11.81Hz, 1H), 4.09-4.19 (m, 1H), 4.98 (d, 1H), 5.12 (d, 1H), 5.39-5.43 (m, 1H), and 7.32-7.39 (m, 5H).

### INTERMEDIATES 4 & 5

Benzyl (1*S*,4a*R*,6a*S*,7*R*,8*R*,10a*R*,10b*R*,12a*R*,14*R*,15*R*)-15-[[(2*R*)-2,3-dimethyl-2-[[(4-methylphenyl)sulfonyl]amino]butyl]oxy]-8-[(1*R*)-1,2-dimethylpropyl]-14-methoxy-1,6,6a,7, 8,9,10,10a,10b,11,12,12a-dodecahydro-1,6a,8,10a-tetramethyl-4*H*-1,4a-propano-2*H-*phenanthro[1,2-c]pyran-7-carboxylate (INTERMEDIATE 4)
and
Benzyl (1*S*,4a*R*,6a*S*,7*R*,8*R*,10a*R*,10b*R*,12a*R*,14*R*,15*R*)-15-[[(2*S*)-2,3-dimethyl-2-[[(4-methylphenyl)sulfonyl]amino]butyl]oxy]-8-[(1*R*)-1,2-dimethylpropyl]-14-methoxy-1,6,6a,7,8,9,10,10a,10b,11,12,12a-dodecahydro-1,6a,8,10a-tetramethyl-4*H*-1,4a-propano-2*H-*phenanthro[1,2-c]pyran-7-carboxylate (INTERMEDIATE 5)

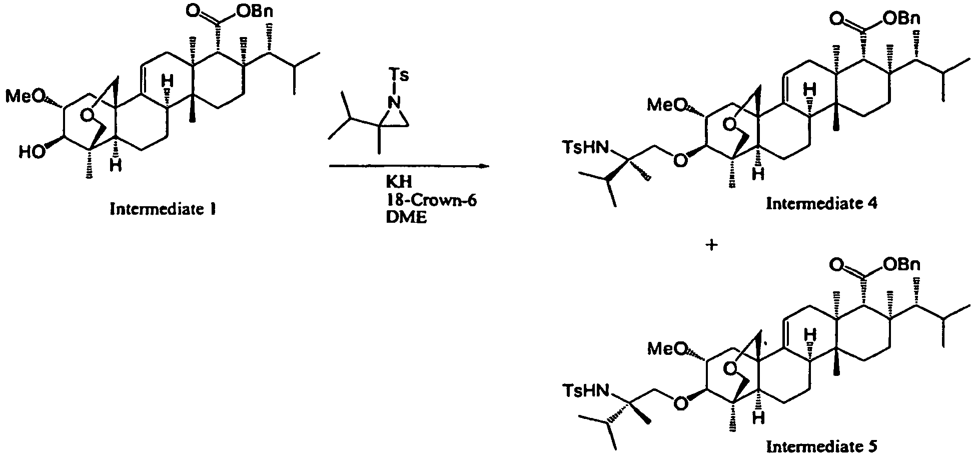

To a stirred solution of Intermediate 1 dissolved in anhydrous dimethoxyethane (400 mL) was added 18-crown-6 (33.7 g, 127.5 mmol) and 2-isopropyl-2-methyl-1-[(4-methylphenyl)sulfonyl]aziridine (21.4 g, 84.6 mmol, 1.66 equiv). The mixture was stirred under nitrogen for 10 min until all solids were dissolved. Potassium hydride (30% in oil, 17.0 g, 127.5 mmol, 2.5 equiv) was added portionwise (ca. 1 g portions) over a period of about 30 minutes. After the completion of the addition, the resulting suspension was stirred at room temperature for about 3 h. The reaction was carefully quenched by the dropwise addition of methanol (40 mL). The reaction mixture was then diluted with water (300 mL) and extracted with EtOAc (300 mL). The organic solution was washed with water (2x200 mL) and dried over anhydrous MgSO₄. The drying agent was removed by filtration and the organic solvent was removed under reduced pressure to afford the desired compound (67.4 g) as a mixture of diastereomers. Separation of the diastereomers was accomplished by chromatography on silica gel (0-15% EtOAc/heptanes) to give the faster eluting isomer, Intermediate 4, and the slower eluting isomer, Intermediate 5.

### INTERMEDIATE 4:

¹H NMR CDCl₃ δ (PPM) 7.81 (d, 1H, ArH); 7.38 (m, ArH); 7.34 (m, ArH); 7.26 (m, ArH); 6.65 (s, NH); 5.44 (m, 1H, H5); 5.12 (d, 2H, CH₂Ar); 4.99 (d, 2H, CH₂Ar); 4.23 (m, 1H, H14); 3.69 (d, 1H); 3.65 (d, 1H); 3.47 (s, 3H, OMe); 3.38 (m); 3.26 (d, 1H); 3.21 (d, 1 H); 2.89 s, 1H, H7), 2.83 (d, 1H); 2.49 (dd, 1H, H13); 2.42 (s, ArMe); 2.12 (m, 1H); 2.02-2.08 (m); 1.18 (s, 3H, Me); 0.95 (d, 3H, Me); 0.93 (s, 3H, Me); 0.88 (d, 3H, Me); 0.82 (d, 3H, Me); 0.78 (d, 3H, Me); 0.73 (d, 3H, Me); 0.72 (s, 3H, Me) and 0.67 (s, 3H, Me).

### INTERMEDIATE 5:

¹H NMR CDCl₃ δ (PPM) 7.77 (d, 1H, ArH); 7.37 (m, ArH); 7.33 (m, ArH); 7.27 (s, ArH); 7.26 (d, ArH); 5.41 (m, 1H, H5); 5.19 (s, NH); 5.11 (d, 2H, CH₂Ar); 4.98 (d, 2H, CH₂Ar); 4.22 (m, 1H, H14); 3.72 (d, 1H); 3.68 (d, 1H); 3.50 (d, 1H); 3.39 m); 3.37 (s, 3H, OMe); 3.30 (d, 1H); 2.89 (s, 1H, H7), 2.82 (d, 1H); 2.42-2.45 (m); 2.41 (s, ArMe); 2.11 (m, 1H); 2.00-2.04 (m); 1.89-1.94 (m); 1.70-1.79 (m); 1.44-1.58 (m); 1.35-1.39 (m); 1.14-1.27 (m); 1.23 (s, 3H, Me); 1.15 (s, 3H, Me); 1.00 (s, 3H, Me); 0.88 (d, 3H, Me); 0.86 (d, 3H, Me); 0.82 (s, 3H, Me); 0.81 (d, 3H, Me); 0.78 (d, 3H, Me); 0.73 (d, 3H, Me) and 0.72 (s, 3H, Me).

### INTERMEDIATE 4

### (ALTERNATIVE SYNTHESIS)

Benzyl (1*S*,4a*R*,6a*S*,7*R*,8*R*,10a*R*,10b*R*,12a*R*,14*R*,15*R*)-15-[[(2*R*)-2,3-dimethyl-2-[[(4-methylphenyl)sulfonyl]amino]butyl]oxy]-8-[(1*R*)-1,2-dimethylpropyl]-14-methoxy-1,6,6a,7,8,9,10,10a,10b,11,12,12a-dodecahydro-1,6a,8,10a-tetramethyl-4*H*-1,4a-propano-2*H-*phenanthro[1,2-c]pyran-7-carboxylate

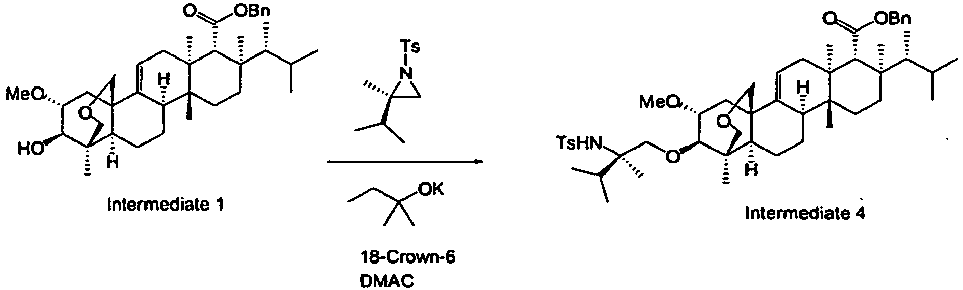

To a solution of Intermediate 1 (8.0g, 13.49mmol) in DMAC (50mL) under a nitrogen atmosphere was added (2*R*)-2-isopropyl-2-methyl-1-[(4-methylphenyl)sulfonyl]aziridine (6.15g, 24.29 mmol) and 18-crown-6 (3.57g, 13.49 mmol). A solution of potassium tert pentoxide in toluene (∼1.7 M, 9.53 mL, 16.19 mmol) was added in one portion. The mixture was stirred at room tempurature for 16 hours and partitioned between EtOAc and 1N HCl. The organic layer was washed with brine and dried over Na₂SO₄. The solvent was evaporated and the residue was chromatographed with an ISCO Combiflash using 15-30% EtOAc/ hexanes as gradient to afford Intermediate 4 as a pale yellow solid. 7.50g

### INTERMEDIATE 6

### (1S,4aR,6aS,7R,8R,10aR,10bR,12aR,14R,15R)-15-[[(2R)-2-amino-2,3-dimethylbutyl]oxy]-8-[(1R)-1,2-dimethylpropyl]-14-methoxy-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

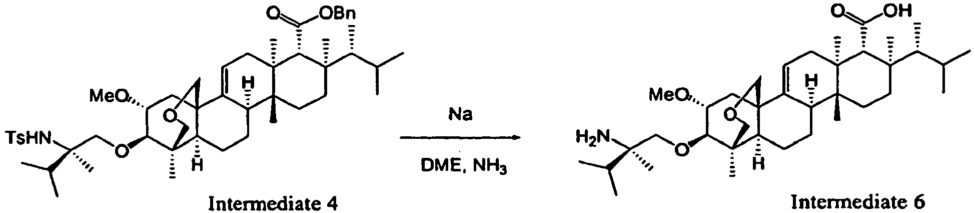

A 500 mL round bottom flask was cooled in a dry ice acetone bath and approximately 100 mL of ammonia was distilled into the flask. The flask was removed from the bath and allowed to warm to reflux. Sodium (5.7 g) was added to give a deep blue solution. DME (15 mL) was added followed by the dropwise addition of Intermediate 4 (5 grams) in DME (20 mL) over 6 minutes. The deep blue color persisted over the addition and the next 1.5 hours. At 1.5 hours, LC/MS analysis of an aliquot showed complete conversion to the product. Workup was as follows: The dropwise addition of methanol (130 mL) (with a stream of nitrogen blown over the surface) produced a heavy white suspension. The nitrogen stream was continued an additional 30 minutes. Ethyl acetate (800 mL) and water (400 mL) were added and the aqueous layer was re-extracted with more ethyl acetate (200 mL). The combined ethyl acetate was dried with magnesium sulfate, filtered and evaporated to give Intermediate 6 as a white solid (3.18 grams). No purification was necessary.

¹H NMR CD3OD δ (PPM) 5.52 (dd, 1H, H5); 4.23 (m, 1H, H14); 3.70 (m); 3.38 (s, 3H, OMe); 3.28-3.34 (m); 2.71 (s, 1 H, H7), 2.54 (dd, 1H, H 13), 2.29 (m); 1.98- 2.08 (m); 1.54- 1.84 (m); 1.44-1.50 (m); 1.34-1.41 (m); 1.27 (s, 3H, Me); 1.19 (s, 3H, Me); 1.15-1.24 (m); 1.10 (s, 3H, Me); 0.99 (d, 3H, Me); 0.96 (d, 3H, Me); 0.89 (d, 3H, Me); 0.83 (d, 3H, Me); 0.79 (s, 3H, Me); 0.77 (d, 3H, Me) and 0.76 (s, 3H, Me).

LC/MS *m*/*z* (positive ion scan) M+1= 602.62

### INTERMEDIATE 8

### (1S,4aR,6aS,7R,8R,10aR,10bR,12aR,14R,15R)-15-[[(2R)-2,3-dimethyl-2-(methylamino)butyl]oxy]-8-[(1R)-1,2-dimethylpropyl]-14-methoxy-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

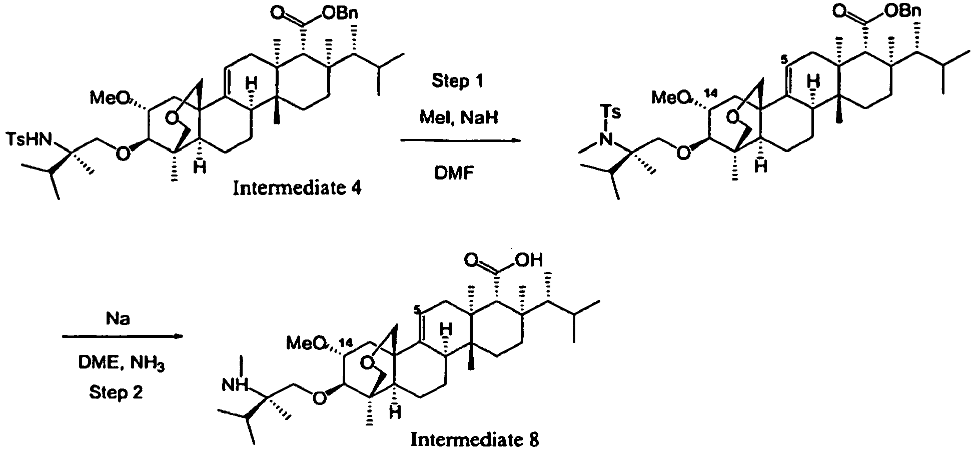

### Step 1

Sodium hydride, a 60% dispersion in mineral oil (52 mg, 1.3 mmol), was added to a suspension of Intermediate 4 (1.1 g, 1.3 mmol) and methyl iodide (0.81 mL, 13 mmol) in anhydrous dimethylformamide (2.6 mL). The suspension was heated in a 50 degree oil bath for 1.5 hours, whereupon additional sodium hydride (47 mg, 1.2 mmol) was added. After an additional 1.5 hours, the mixture was cooled to room temperature, ethyl acetate (50 mL), water (50 mL) and 2N hydrochloric acid (7 mL) were added and the organic layer was washed with water (4 x 50 mL), brine (1 x 20 mL), dried with magnesium sulfate, filtered and evaporated to give 2 as a foam (1.1 grams).

Selected ¹H NMR (CDCl₃, 600MHz, ppm) 2.42 (s, 3H, PhMe), 3.06 (s, 3H, NMe); 3.28 (s, 3H, OMe); 4.14 (m, 1H, H14); 5.00 and 5.14 (2d, 2H, CH₂Ph), 5.22 (dd, 1H, H5), 7.25 (d, 2H, ArH), 7.75 (d, 2H, ArH).

### Step 2

A solution of the product from Step 1 (1.1 g, 1.28mmol) in anhydrous dimethoxyethane (6 mL) was added dropwise over 5 minutes to refluxing ammonia (ca. 20 mL) containing dimethoxyethane (4 mL) and sodium (1.68 g, 73.4 mmol). Additional ammonia (ca. 10 mL) was added and the deep blue colored mixture was stirred an additional 80 minutes. Dropwise addition of methanol (30 mL) produced a heavy white suspension over which a stream of nitrogen was passed for approximately 20 minutes. Ethyl acetate (200 mL) and water (100 mL) were added, the aqueous layer was re-extracted with more ethyl acetate (1x50 mL) and the combined ethyl acetate layers were dried with magnesium sulfate, filtered and evaporated to give Intermediate 8 as a foam (0.8 g).

Selected ¹H NMR (CDCl₃, 600MHz, ppm) 2.64 (s, 3H, NMe); 3.32 (s, 3H, OMe); 4.22 (m, 1H, H14), 5.57 (dd, 1H, H5).

LC/MS m/z (positive ion scan) M+1= 616.60

### INTERMEDIATES 12 & 13

Benzyl (1*S*,4a*R*,6a*S*,7*R*,8*R*,10a*R*,10b*R*,12a*R*,14*R*,15*R)*-8-[(1*R*)-1,2-dimethylpropyl]-14-methoxy-15-[[(2*R*)-2,3,3-trimethyl-2-[[(4-methylphenyl)sulfonyl]amino]butyl]oxy]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carboxylate (INTERMEDIATE 12)
and

Benzyl (1*S*,4a*R*,6a*S*,7*R*,8*R*,10a*R*,10b*R*,12a*R*,14*R*,15*R*)-8-[(1*R*)-1,2-dimethylpropyl]-14-methoxy-15-[[(2*S*)-2,3,3-trimethyl-2-[[(4-methylphenyl)sulfonyl]amino]butyl]oxy]-1,6,6a,7,8,9,10,10a,10b,11,12,12a-dodecahydro-1,6a,8,10a-tetramethyl-4*H-*1,4a-propano-2*H*-phenanthro[1,2-c]pyran-7-carboxylate (INTERMEDIATE 13)

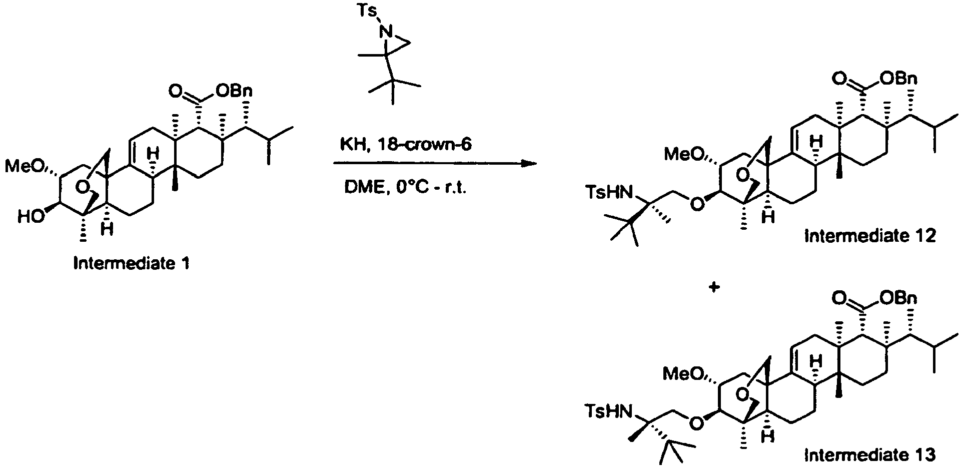

Intermediate 1 (5.00 g, 8.43 mmol), 18-crown-6 (11.15 g, 42.2 mmol), and 2-(1,1-dimethylethyl)-2-methyl-1-[(4-methylphenyl)sulfonyl]aziridine (4.51 g, 8.67 mmol) were dissolved in toluene and concentrated then placed under high vacuum for 1 hour. The resulting mixture was dissolved in dimethoxyethane (84 mL) placed under nitrogen atmosphere and cooled to 0°C. Potassium hydride (30% dispersion in mineral oil, 3.38 g, 25 mmol) was added and the reaction evacuated and charged with nitrogen (repeat evac./charge three times). The reaction was allowed to slowly warm to room temperature and after two hours additional 2-(1,1-dimethylethyl)-2-methyl-1-[(4-methylphenyl)sulfonyl]aziridine (0.43 g, 0,83 mmol) was added. After an additional hour the reaction was quenched by the addition of methanol followed by 1 N aq. HCl. The reaction mixture was partitioned between ethyl acetate and water and the aqueous extracted with ethyl acetate as necessary. The combined organic phase was dried over MgSO₄, filtered then concentrated. The crude product was purified by multiple flash chromatographies on Biotage 65i columns (0-100% ethyl acetate/hexane) which resolved the two diastereomeric products; the faster eluting Intermediate 12 (1.3g) and the slower eluting Intermediate 13 (3.0g).

### INTERMEDIATE 12

¹H NMR (CDCl₃, 500 MHz, ppm) δ 0.63 (s, 3H), 0.71 (s, 3H) 0.72 (d, 3H, partially obscured), 0.78 (d, J=5.8 Hz, 3H), 0.81 (d, J=5.7 Hz, 3H), 0.96 (s, 3H), 1.02 (s, 9H), 1.14 - 1.3 (m), 1.2 (s, 3H), 1.25 (s, 3H), 1.32 - 1.8 (m), 1.92 (m, 1H), 2.04 (m, 1H), 2.12 (m, 1H), 2.41 (s, 3H), 2.52 (m, 1H), 2.72 (d, J=9.0 Hz, 1H), 2.88 (s, 1H), 2.98 (d, J=10.9 Hz, 1H), 3.25 (d, J=11.2 Hz, 1H), 3.35 - 3.4 (m, 2H), 3.49 (s, 3H), 3.63 (d, J=11.9 Hz, 1H), 3.85 (d, J=10.8 Hz, 1H), 4.25 (m, 1H), 4.98 (d, J=12.4 Hz, 1H), 5.12 (d, J=12.4 Hz, 1H), 5.44 (m, 1H), 6.89 (s, 1H), 7.25 (d, 2H, partially obscured), 7.32 - 7.4 (m, 5H), 7.84 (d, J=8.3 Hz, 2H).

### INTERMEDIATE 13

¹H NMR (CDCl₃, 500 MHz, ppm) δ 0.71 (s, 3H), 0.72 (d, 3H, partially obscured), 0.77 (d, J=6.7 Hz, 3H), 0.81 (d, J=6.6Hz, 3H), 0.83 (s, 3H), 0.97 (s, 9H), 1.02 (s, 3H), 1.12 - 1.28 (m), 1.14 (s, 3H), 1.24 (s, 3H), 1.34 - 1.6 (m), 1.68 - 1.8 (m), 1.92 (m, 1H), 2.02 (m, 1H), 2.11 (m, 1H), 2.40 (s, 3H), 2.45 (m, 1H, partially obscured), 2.77 (d, J=8.2 Hz, 1H), 2.87 (s, 1H), 2.96 (d, J=9.6 Hz, 1H), 3.29 (d, J=11.7 Hz, 1H), 3.34 - 3.41 (3d, 3H), 3.35 (s, 3H), 3.6 (d, J=11.6 Hz, 1H), 4.25 (m, 1H), 4.98 (d, J=12.3 Hz, 1H), 5.08 (s, 1H), 5.10 (d, J=12.4 Hz, 1H), 5.41 (m, 1H), 7.25 (d, 2H, partially obscured), 7.3 - 7.4 (m, 5H), 7.78 (d, J=5.8 Hz, 2H).

### INTERMEDIATE 12

### (ALTERNATIVE SYNTHESIS)

### Benzyl (1S,4aR,6aS,7R,8R,10aR,10bR,12aR,14R,15R)-8-[(1R)-1,2-dimethylpropyl]-14-methoxy-15-[[(2R)-2,3,3-trimethyl-2-[[(4-methylphenyl)sulfonyl]amino]butyl]oxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylate

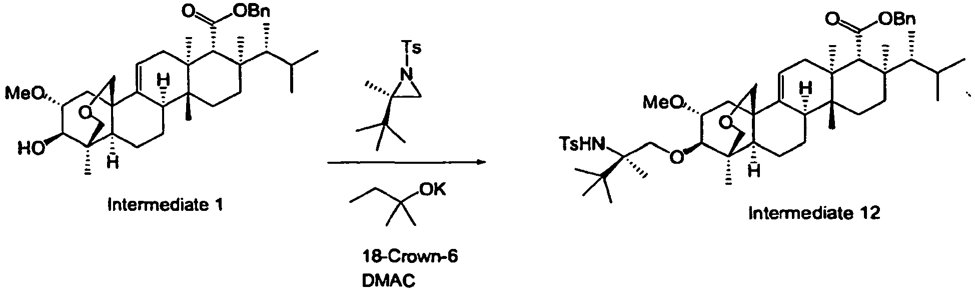

A solution of Intermediate 1 (21.26 g, 35.9 mmol), 18-crown-6 (11.37 g, 43.0 mmol), and (2*R*)-2-(1,1-dimethylethyl)-2-methyl-1-[(4-methylphenyl)sulfonyl]aziridine (9.59 g, 35.9 mmol), in toluene (25 mL) was evaporated under vacuum to azeotropically dry the reagents. The resulting oil was dissolved in N,N-dimethylacetamide (200 mL) and the solution was cooled under nitrogen in an ice bath. To the ice cold stirred solution was added over a 2 minute period a solution of potassium 2-methyl-2-butoxide in toluene (1.7 M, 25.3 mL, 43.0 mmol). The reaction was slowly allowed to warm to room temperature and monitored by TLC. After the reaction was judged complete, the reaction was quenched with 2N hydrochloric acid (22 mL), diluted with dichloromethane (500 mL), and the mixture was washed with water (3 x 300 mL). The organic phase was dried over magnesium sulfate, filtered, and evaporated to an oil which was flash chromatographed (silica gel, 5-60% ethyl acetate:hexane) to give Intermediate 12 as a white solid (24.04 g).

### INTERMEDIATE 14

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-methoxy-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

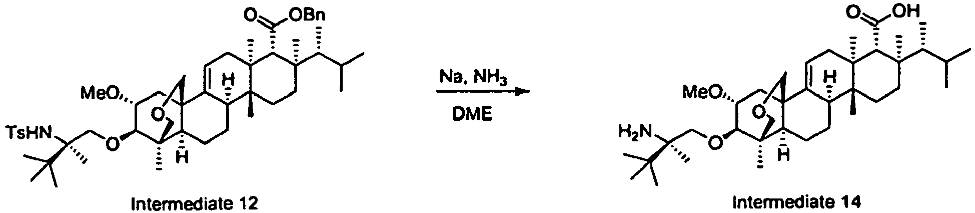

Ammonia (approx. 150 mL) was condensed into a 3-neck flask equipped with a cold-finger condenser and sodium (approx. 5 g, 220 mmol) was added to give a deep blue solution. To this solution was added a solution of intermediate Intermediate 12 (13.3 g, 15.5 mmol) in dimethoxymethane (130 mL) and the reaction was refluxed at -33°C for 1.5 hours. The reaction was quenched by the careful addition of methanol followed by water until the reaction was a white slurry. The solvents were evaporated by a stream of nitrogen overnight. After approximately 18 hours methanol (approx. 50mL) was added and the resulting white slurry/partial solution was stirred for about 10 minutes to ensure that all solids were in suspension (as opposed to fixed to the flask wall.) This mixture was partitioned between ethyl acetate and water and the aqueous extracted twice with ethyl acetate. The combined organic phase was dried over MgSO₄, filtered then concentrated to give Intermediate 14 (9.8 g) as an off-white solid.

¹H NMR (CD₃OD, 500 MHz, ppm) δ 0.77 (s, 3H), 0.78 (d, J=7.4 Hz, 3H), 0.81 (s, 3H), 0.85 (d, J=6.9 Hz, 3H), 0.89 (d, J=6.8 Hz, 3H), 1.05 (s, 9H), 1.19 (s, 3H), 1.20 (s, 3H), 1.32 (s, 3H), 1.18 - 1.36 (m), 1.4 (m, 1 H), 1.48 (m, 1 H), 1.56-1.86 (m). 2.05 (m, 1H), 2.23 (m, 1H), 2.55 (dd, J=13.3 Hz, 6.7 Hz, 1H), 2.71 (s, 1H), 3.25 (m, 1 H), 3.30 (m, 1H, partially obscured), 3.4 (AB, 2H, partially obscured), 3.4 (s, 3H), 3.67 (d, J=11.9 Hz, 1H), 3.74 (d, J=11.3 Hz, 1H), 3.92 (d, J=11.2 Hz, 1H), 4.24 (m, 1H), 5.54 (m, 1H)

m/z = 616.34 (M + H)

### INTERMEDIATE 16

### (1S,4aR,6aS,7R,8R,10aR,10bR,12aR,14R,15R)-8-[(1R)-1,2-dimethylpropyl]-14-methoxy-15-[[(2R)-2,3,3-trimethyl-2-(methylamino)butyl]oxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

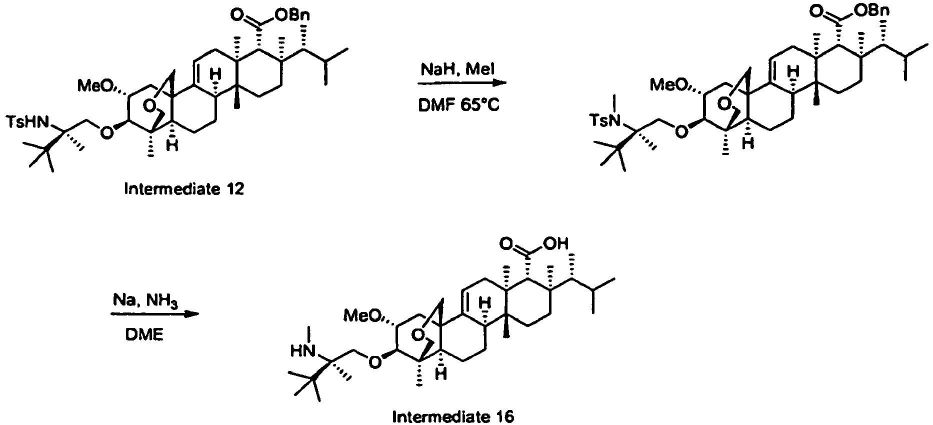

Sodium hydride (60% dispersion in mineral oil, 930 mg, 23 mmol) was washed with hexane and the hexane decanted. This procedure was repeated and the remaining hexane removed in vacuo. This pre-washed sodium hydride was suspended in DMF (10 mL) and a solution of Intermediate 12 (2.0 g, 2.3 mmol) in DMF (13 mL) was added. Methyl iodide (1.45 mL, 23 mmol) was added and the reaction mixture heat at 65°C under nitrogen for 90 minutes. After cooling to room temperature the reaction was partitioned between ethyl acetate and saturated aqueous bicarbonate. The organic phase was washed with brine, dried (MgSO₄) filtered and concentrated in vacuo. Column chromatography (Biotage 40+M column, 5-100% EA/Hex) yielded the product (1.95 g)

The product from Step 1 was subjected to sodium/ammonia reduction by a procedure analogous to that described for the preparation of Intermediate 14. The product thus obtained was lyophilized from MeOH/benzene to give Intermediate 16 (1.4 g) as an off-white solid.

¹H NMR (CD₃OD, 500 MHz, ppm) δ 0.76 (s, 3H), 0.77 (d, 3H, partially obscured), 0.82 (s, 3H), 0.84 (d, J=6.8 Hz, 3H), 0.91 (d, J=6.7 Hz, 3H), 1.06 (s, 3H), 1.07 (s, 9H), 1.16 - 1.3 (m), 1.15 (s, 3H), 1.22 (s, 3H), 1.3 - 1.5 (m), 1.6 (m), 1.64 - 1.84 (m), 1.96 - 2.1 (m, 2H), 2.31 (m, 1H), 2.57 (m, 1H, partially obscured), 2.57 (s, 3H), 2.74 (s, 1H), 2.93 (d, J=8.5 Hz, 1H), 3.35 (m, 1H, partially obscured), 3.36 (s, 3H), 3.41 (br s, 2H), 3.62 (d, J=11.6 Hz, 1H), 3.8 (AB, 2H), 4.23 (m, 1H), 5.53 (m, 1H).

m/z = 630.59 (M + H)

### INTERMEDIATE 26

### Benzyl (1S,4aR,6aS,7R,8R,10aR,10bR,12aR,14R,15R)-8-[(1R)-1,2-dimethylpropyl]-14-methoxy-15-[[tetrahydro-4-[[(4-methylphenyl)sulfonyl]amino]-2H-pyran-4-yl]methoxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylate

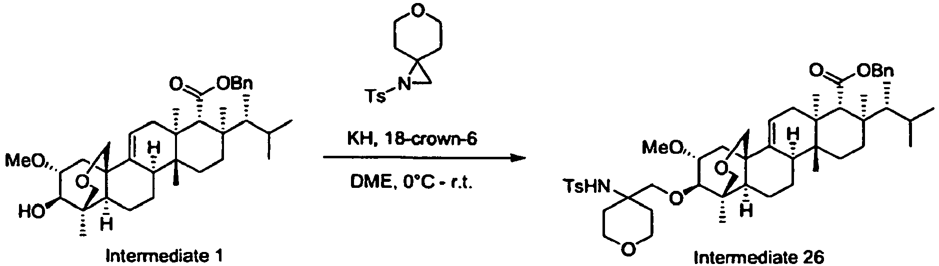

Intermediate 1 (0.38 g, 0.641 mmol), 18-crown-6 (0.508 g, 1.92 mmol), and 1-[(4-methylphenyl)sulfonyl]-6-oxa-1-azaspiro[2.5]octane (0.257 g, 0.961 mmol) were dissolved in toluene and concentrated then placed under high vacuum for 1 hour. The resulting mixture was dissolved in dimethoxyethane (10 mL) placed under nitrogen atmosphere and cooled to 0°C. Potassium hydride (30% dispersion in mineral oil, 0.171 g, 1.28 mmol) was added and the reaction evacuated and charged with nitrogen (repeat three times). After an additional hour the reaction mixture was treated with aqueous ammonium chloride carefully, and the mixture was extracted with dichloromethane (2x 20mL). The combined organic phase was dried over MgSO₄, filtered then concentrated. The crude product was purified by flash chromatography (10-50% ethyl acetate/hexane) to yield Intermediate 26 (0.5 g) as a white foam.

¹H NMR (CDCl₃, 500 MHz, ppm) δ 0.74 (s, 3H), 0.75 (s, 3H) 0.80 (d, J=6.9 Hz, 3H), 0.84 (d, J=6.7 Hz, 3H), 1.14 - 1.3 (m), 1.19 (s, 3H), 1.26 (s, 3H), 1.32 - 1.8 (m), 1.84 - 1.98 (m), 2 - 2.18 (m), 2.45 (s, 3H), 2.47 (m, 1H), 2.89 (d, J=9.4 Hz, 1H), 2.90 (s, 1H), 3.29 (d, J=9.3 Hz, 1H), 3.35 - 3.45 (m, 5H = C2 MeO+2H?), 3.54 (m, 2H), 3.57 (m, 2H), 3.64 - 3.72 (m, 2H), 3.79 (d, J=10.1 Hz, 1H), 4.20 (m, 1H), 5.0 (d, J=12.1 Hz, 1H), 5.14 (d, J=12.1 Hz, 1H), 5.44 (m, 1H), 5.85 (s, 1H), 7.30 (d, J=9.2 Hz, 2H), 7.32 - 7.4 (m, 5H), 7.81 (d, J=8.2 Hz, 2H).

### INTERMEDIATE 28

### (1S,4aR,6aS,7R,8R,10aR,10bR,12aR,14R,15R)-8-[(1R)-1,2-dimethylpropyl]-14-methoxy-15-[[tetrahydro-4-(methylamino)-2H-pyran-4-yl]methoxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

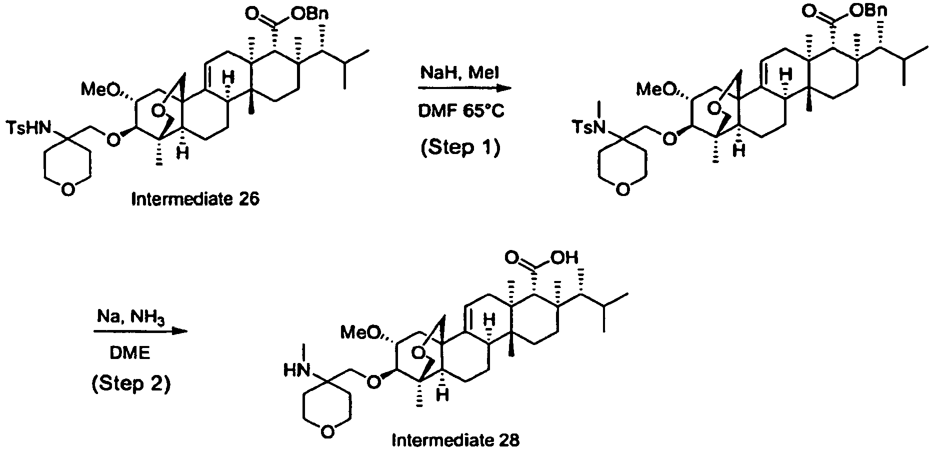

### Step 1

Sodium hydride (60% dispersion in mineral oil, 84 mg, 2.1 mmol) was washed with hexane and the hexane decanted. This procedure was repeated and the remaining hexane removed in vacuo. This pre-washed sodium hydride was suspended in DMF (1 mL) and a solution of Intermediate 26 (0.18 g, 0.21 mmol) in DMF (1 mL) was added. Methyl iodide (0.131 mL, 2.1 mmol) was added and the reaction mixture heat at 60°C under nitrogen for 60 minutes. After cooling to room temperature the reaction was partitioned between ethyl acetate and saturated aqueous bicarbonate. The organic phase was washed with brine, dried (MgSO₄) filtered and concentrated in vacuo to give the product (0.18 g, 98%)

### Step 2

The product was subjected to the sodium/ammonia reduction by analogy to the preparation of intermediate 27 then lyophilized from MeOHBenzene to give intermediate 28 (0.16 g) as an off-white foam.

¹H NMR (CD₃OD, 500 MHz, ppm) δ 0.78 (s, 3H), 0.79 (d, 3H, partially obscured), 0.80 (s, 3H), 0.86 (d, J=6.6 Hz, 3H), 0.91 (d, J=6.6 Hz, 3H), 1.21 (s, 3H), 1.18 - 1.42 (m), 1.50 (m, 1H), 1.56 - 1.96 (m), 1.98 (m, 1H), 2.02 (m, 1H), 2.08 (m, 1H), 2.10 (m, 1H), 2.26 (m, 1H), 2.55 (s, 3H), 2.57 (m, 1H, partially obscured), 2.80 (s, 1H), 2.99 (d, J=8.7 Hz, 1H), 3.31 (s, 3H), 3.43 (s, 3H), 3.58 (m, 2H), 3.68 (d, J=11.7 Hz, 1H), 3.82 (d, J=10.5 Hz, 1H), 3.84 - 3.9(m, 2H), 4.02 (d, J=10.7 Hz, 1H), 4.23 (m, 1H), 5.55 (m, 1H).

### INTERMEDIATE 32

### (1S,4aR,6aS,7R,8R,10aR,10bR,12aR,14R,15R)-15-[[(2R)-2-amino-2,3-dimethylbutyl]oxy]-8-[(1R)-1,2-dimethylpropyl]-14-hydrazino-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

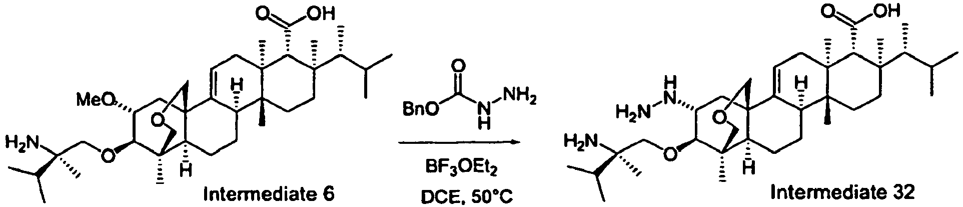

Benzyl carbazate (208.9 mg, 1.257 mmol) and BF₃O(CH₂CH₃)₂ (0.53 ml, 4.18 mmol) were added to a stirred hazy solution of Intermediate 6 (251.7 mg, 0.418 mmol) in 1,2-dichloroethane (4.2 ml). The reaction mixture was a hazy solution that was heated to 50°C. After 2.5 hours, LCMS and ¹H NMR showed complete consumption of Intermediate 6. The reaction mixture was cooled to room temperature and partitioned between ethyl acetate (75 ml) and water (40 ml). The aqueous layer was extracted with ethyl acetate (2 x 40 ml). The organic layers were combined, dried over sodium sulfate, and filtered. The solvent was evaporated under reduced pressure to give a light yellow residue. The residue was dissolved in methanol and purified using four HPLC runs (∼63 mg / run) on a 30 x 150 mm Sunfire Prep C18 OBD 10 µm column by eluting with acetonitrile/water + 0.1% TFA. The total flow rate was 20 ml/min and the HPLC method employed a 17 minute 20%-100% acetonitrile/water gradient followed by a 2 minute acetonitrile flush. The product fractions were combined, the solvent was evaporated under reduced pressure, and the residue was lyophilized from ethanol and benzene to give Intermediate 32 (146.4 mg) as a white solid (TFA salt).

¹H NMR (CD₃OD, 500 MHz, ppm) δ 0.78 (s, 3H, Me), 0.79 (d, 3H, Me), 0.85 (s, 3H, Me), 0.88 (d, 3H, Me), 0.92 (d, 3H, Me), 1.01 (d, 3H, Me), 1.04 (d, 3H, Me), 1.20 (s, 6H, 2Me), 1.23 (s, 3H, Me), 1.24-1.36 (m), 1.41-1.55 (m), 1.59-1.68 (m), 1.74-1.91 (m), 1.96-2.03 (m), 2.09-2.15 (m), 2.18-2.28 (m), 2.55 (dd, 1H, H 13), 2.88 (s, 1H, H7), 3.17 (d, 1H), 3.42 (d, 1H), 3.49 (abq, 2H), 3.78 (d, 1H), 3.80 (d, 1H), 3.81 (dd, 1H), 4.17-4.25 (m, 1H, H 14), 5.58 (dd, 1H, H5).

Mass Spectrum: (ESI) *m*/*z* = 602.73 (M+H).

### INTERMEDIATE 33

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-hydrazino-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

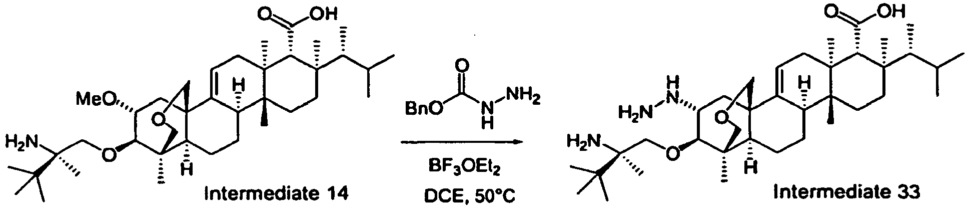

A suspension of Intermediate 14 (2.98 g, 0.048 mol), benzyl carbazate (2.55 g, 0.015 mol) and boron trifluoride etherate (6.15 mL, 0.048 mol) in 1,2-dichloroethane (50 mL) was blanketed with nitrogen and heated in a 50°C oil bath for 4.5 hours. The mixture was cooled to room temperature, and partitioned between ethyl acetate (400 mL) and water (200 mL). The aqueous layer was extracted with more ethyl acetate (4 x 50 mL) and the combined ethyl acetate layers were dried with sodium sulfate, filtered and evaporated to a solid (6.0 g). The solid was purified by reverse phase HPLC using a 19 x 150 mm Sunfire Preparative C18 OBD column. Fractions containing the product were combined, evaporated and freeze-dried from a mixture of ethanol and benzene to give the title compound as a TFA salt (2.0 g).

### INTERMEDIATE 33

### (ALTERNATIVE PROCEDURE)

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-hydrazino-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

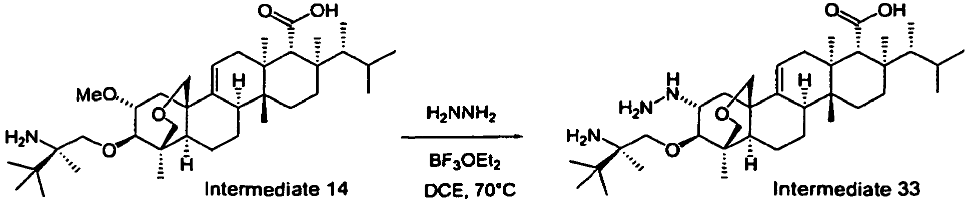

A suspension of Intermediate 14 (3.0 g, 0.048 mol), anhydrous hydrazine (0.762 mL, 0.024 mol) and boron trifluoride etherate (12.36 mL, 0.098 mol) in 1,2-dichloroethane (24.4 mL) was blanketed with nitrogen and heated in a 70°C oil bath for 2 hours. The mixture was cooled to room temperature, methanol (30 mL) and water (20 mL) were added and the mixture was evaporated to approximately 20 mL. Ethyl acetate (125 mL) and saturated aqueous sodium bicarbonate (180 mL) were added, the aqueous layer was re-extracted with ethyl acetate (3 x 40 mL) and the combined ethyl acetate layers were dried with magnesium sulfate, filtered and evaporated to a solid (3.0 g).

¹H NMR (CD₃OD, 600MHz, ppm) δ 0.76 (s, 3H, Me), 0.77 (d, 3H, Me), 0.81 (s, 3H, Me), 0.85 (d, 3H, Me), 0.90 (d, 3H, Me), 1.05 (s, 9H, Me), 1.17 (s, 3H, Me), 1.20 (s, 3H, Me), 1.25 (s, 3H, Me), 1.23-1.34 (m), 1.41-1.45 (m), 1.48-1.65 (m), 1.70-1.90 (m), 1.90-2.00 (m), 2.06-2.11 (m), 2.16-2.24 (m), 2.49 (dd, 1H, H 13), 2.85 (s, 1H, H7), 2.96 (d, 1H), 3.38 (d, 1H), 3.40-3.60 (m), 3.49 (br d, 1H), 3.82 (d, 1H), 3.89 (br m, 1H, H14), 4.07 (d, 1H), 5.56 (dd, 1H, H5).

Mass spectrum: (ESI) *m*/*z* = 616.40 (M+H)

### INTERMEDIATE 34

### (1S,4aR,6aS,7R,8R,10aR,10bR,12aR,14R,15R)-8-[(1R)-1,2-dimethylpropyl]-14-hydrazino-15-[[(2R)-2,3,3-trimethyl-2-(methylamino)butyl]oxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

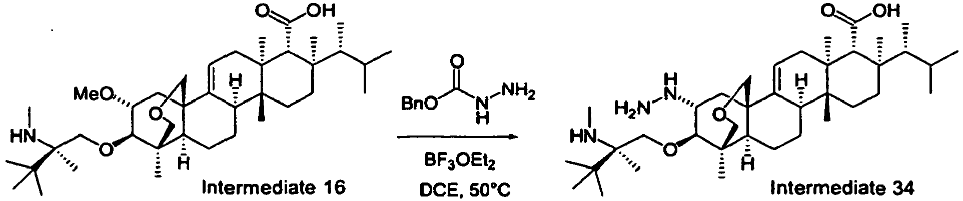

Intermediate 16 (700 mg, 1.11 mmol) and benzyl carbazate (554 mg, 3.33 mmol) were combined then diluted with dichloroethane (11 mL), then treated with BF₃OEt₂ (1.4 mL, 11 mmol) and this mixture was heated to 50°C under nitrogen. After 1.5 hours more BF₃OEt₂ (1.4 mL, 11 mmol) was added. After 4.5 hours the reaction was cooled to room temperature then concentrated *in vacuo* to give a brown slurry. The crude reaction mixture was suspended in methanol (8 mL) then filtered. The filtrate was purified by multiple runs on a preparative LCMS (30x100 mm Waters Sunfire column, 5µm, Electrospray positive detection, 0-100% MeCN/water with 0.05% TFA over 12 minutes, using Masslynx software). The product fractions from were combined and partially concentrated by rotovap then frozen and lyophilized to give Intermediate 34 (310 mg) as a white amorphous solid (TFA salt).

¹H NMR (CD₃OD, 500 MHz, ppm) δ 0.77 (s, 3H), 0.78 (d, 3H, partially obscured), 0.85 (s, 3H), 0.86 (d, 3H, partially obscured), 0.91 (d, J=6.8 Hz, 3H), 1.14 (s, 9H), 1.18 (s, 3H), 1.21 (s, 3H0, 1.23 (s, 3H), 1.23 - 1.67 (m), 1.73 - 1.90 (m), 1.98 (m, 1 H), 2.10 (m, 1H), 2.19 (m, 1H), 2.61 (dd, J=13.4 Hz, 6.5 Hz, 1H), 2.80 (s, 3H), 2.86 (s, 1H), 2.90 (m, 1H), 3.44 (d, J=11.9 Hz, 1H), 3.48 (m, 2H), 3.71 (d, J=11.9 Hz, 1H), 3.83 (d, J=11.4 Hz, 1H), 3.94 (d, J=11.4 Hz, 1H), 4.04 (m, 1H), 5.57 (m, 1H).

Mass Spectrum: (ESI) *m*/*z* = 630.35 (M+H).

### INTERMEDIATE 36

### (1S,4aR,6aS,7R,8R,10aR,10bR,12aR,14R,15R)-8-[(1R)-1,2-dimethylpropyl]-14-hydrazino-15-[[tetrahydro-4-(methylamino)-2H-pyran-4-yl]methoxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

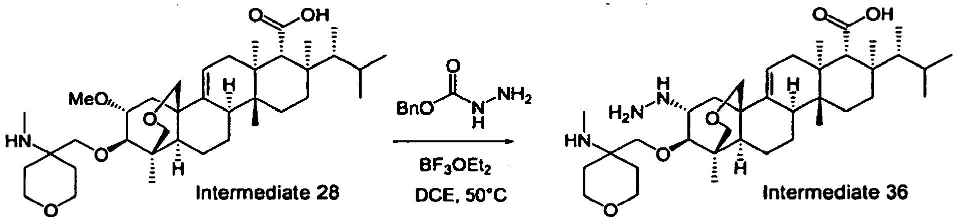

A solution of Intermediate 28 (1.58 g, 2.57 mmol) in dichloroethane (25.8 mL) was treated with benzyl carbazate (1.28 g, 7.7 mmol) then BF₃OEt₂ (3.25 mL, 25.7 mmol) and this mixture was heated to 55°C under nitrogen. After 2 hours the reaction was cooled to room temperature then concentrated *in vacuo.* The crude product mixture was suspended in methanol (5 mL) and filtered through a sintered glass funnel. The filtrate was purified by preparative HPLC (19x100 mm Waters Sunfire column, 5µm, UV-detection, 30-100% MeCN/water with 0.05% TFA over 20 minutes). The product fractions were combined and partially concentrated by rotovap then frozen and lyophilized to give Intermediate 36 (800 mg) as a white amorphous solid (TFA salt).

¹H NMR (CD₃OD, 500 MHz, ppm) δ 0.79 (s, 3H), 0.80 (d, 3H, partially obscured), 0.83 (s, 3H), 0.88 (d, J=6.6 Hz, 3H), 0.92 (d, J=6.8 Hz, 3H), 1.20 (s, 3H), 1.23 (s, 3H), 1.24 -2.16 (m), 2.21 (m, 1H), 2.62 (m, 1H), 2.74 (s, 3H), 2.88 (s, 1H), 3.02 (m, 1H), 3.42 (d, J=11.7 Hz, 1H), 3.44 - 3.67( m, 4H), 3.76 -3.85 (m, 2H), 3.76 - 3.85 (m, 2H), 3.86 - 3.96 (m, 2H), 4.00 (m, 1H),4.04 - 4.14 (m, 2H), 5.60 (m, 1H).

Mass Spectrum: (ESI) *m*/*z* = 630.54 (M+H).

### INTERMEDIATE 37

### (1S,4aR,6aS,7R,8R,10aR,10bR,12aR,14R,15R)-8-[(1R)-1,2-dimethylpropyl]-14-hydrazino-15-hydroxy-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

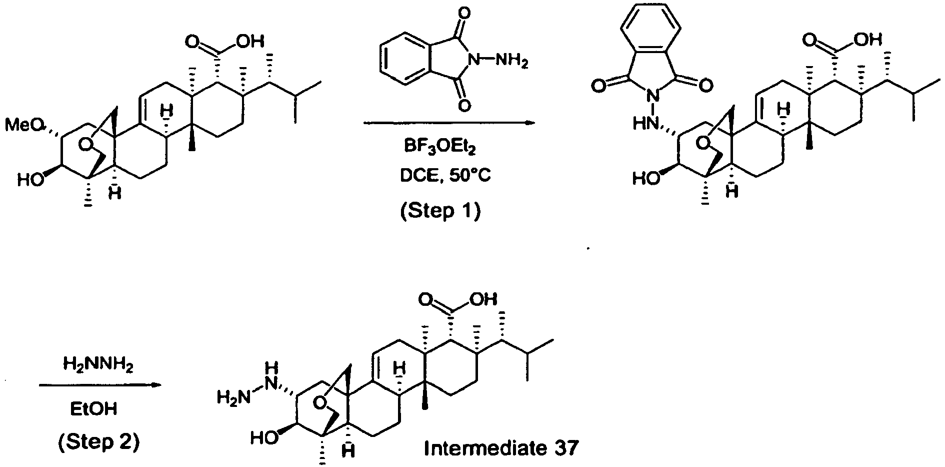

### Step 1:

Boron trifluoride diethyl etherate (12.3 mL, 97.9 mmol) was added slowly to a solution of (1*S*,4a*R*,6a*S*,7*R*,8*R*,10a*R*,10b*R*,12a*R*,14*R*,15*R*)-8-[(1*R*)-1,2-dimethylpropyl]-15-hydroxy-14-methoxy-1,6,6a,7,8,9,10,10a,10b,11,12,12a-dodecahydro-1,6a,8,10a-tetramethyl-4*H*-1,4a-propano-1*H-*phenanthro[1,2-c]pyran-7-carboxylic acid (product compound from Step 2 in the synthesis of Intermediate 1; 5 g, 9.96 mmol) and N-aminophthalimide (1.94 g, 1.20 mmol) in 1, 2-dichloroethane (112 mL) at room temperature. The reaction mixture was heated overnight at 50°C and allowed to cool to room temperature. The reaction mixture was partitioned between EtOAc and water, and the layers were separated. The aqueous layer was extracted with EtOAc. The combined organic layers were dried (Na₂SO₄) and concentrated under reduced pressure. The residue was purified by flash chromatography, elution with 0- 50% EtOAc in heptane, to give the product (4.16 g, 66%) as a pale yellow solid.

¹H NMR (400 MHz, CD₃OD) δ 0.71 - 0.82 (m, 9 H) 0.86 (d, *J*=6.69 Hz, 3 H) 0.91 (d, *J*=6.78 Hz, 3 H) 1.20 (d, *J*=15.72 Hz, 20 H) 1.94 (d, *J*=17.67 Hz, 1 H) 2.10 (d, *J*=12.89 Hz, 1 H) 2.20 (dt, *J*=13.61, 6.84 Hz, 1 H) 2.29 (dd, *J*=13.57, 6.49 Hz, 1 H) 2.85 (s, 1 H) 3.26 (d, *J*=9.52 Hz, 1 H) 3.35 (s, 2 H) 3.42 (s, 2 H) 3.69 (d, *J*=11.67 Hz, 1 H) 4.05 (ddd, *J*=11.69, 9.49, 6.49 Hz, 1H) 5.47 (d, *J*=5.86 Hz, 1 H) 7.58 - 8.08 (m, 4 H).

### Step 2:

Hydrazine monohydrate (13 mL, 267 mmol) was added to a solution of the product from Step 1 (4.16 g, 6.58 mmol) in EtOH (140 mL) at room temperature, and the reaction mixture was stirred overnight. The reaction mixture was poured into a mixture water (ca. 200 mL) and EtOAc (250 mL), and saturated aqueous NaCl (100 mL) was added to generate a white precipitate. The solid was collected by suction filtration, washed with water, and dried on the funnel. The remaining water was removed by evaporation with methanol under reduced pressure. Excess solvent was removed *in vacuo* to provide Intermediate 37 (3.24 g, 100%) as a white solid.

¹H NMR (400 MHz, CD₃OD) δ 0.70 - 0.81 (m, 12 H) 0.84 (d, *J*=6.64 Hz, 3 H) 0.91 (d, *J*=6.78 Hz, 3 H) 1.24 (d, *J*=13.76 Hz, 19 H) 1.98 - 2.13 (m, 2 H) 2.28 - 2.35 (m, 1 H) 2.38 (dd, *J*=13.40, 6.37 Hz, 1H) 2.73 (s, 1 H) 3.14 (d, *J*=9.47 Hz, 1H) 3.35 (d, *J*=11.67 Hz, 1H) 3.46 (s, 2 H) 3.62 (ddd, *J*=11.62, 9.54, 6.32 Hz, 1 H) 3.70 (d, *J*=11.71 Hz, 1 H) 5.55 (d, *J*=5.71 Hz, 1H).

### INTERMEDIATE 38

### (1S,4aR,6aS,7R,8R,10aR,10bR,12aR,14R,15R)-8-[(1R)-1,2-dimethylpropyl]-15-hydroxy-14-[5-(4-pyridinyl)-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid, (4-methoxyphenyl)methyl ester

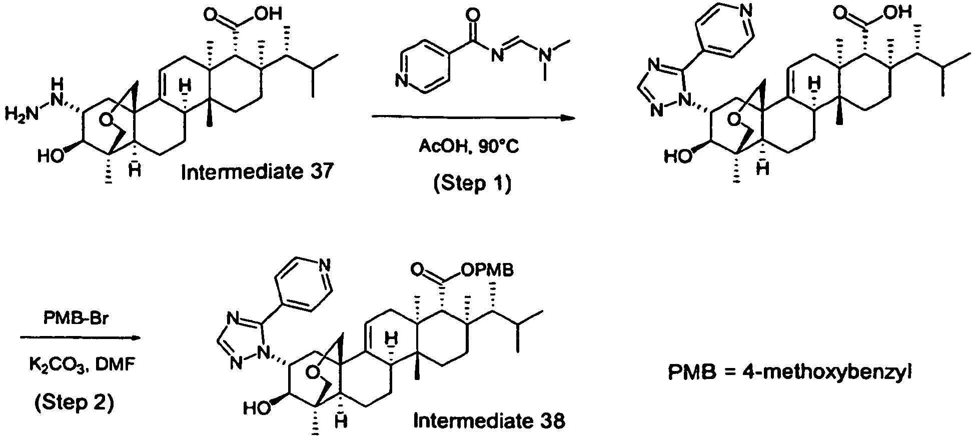

### Step 1:

A solution of Intermediate 37 (3.24 g, 6.45 mmol) and N-[(1*E*)-(dimethylamino)methylidene]pyridine-4-carboxamide (1.26 g, 7.10 mmol) in HOAc (100 mL) was evacuated and flushed with nitrogen. The solution was heated under nitrogen at 90 °C for 1 h. The solution was allowed to cool to room temperature, and the acetic acid was removed under reduced pressure. The residue was partitioned between EtOAc and saturated aqueous NaHCO₃, and the layers were separated. The aqueous layer was extracted with EtOAc. The combined organic layers were washed with saturated aqueous NaCl, dried (Na₂SO₄), and concentrated under reduced pressure. The residue was purified by flash chromatography, elution with 0% - 10% MeOH in CH₂Cl₂, to give the product (2.71 g, 68%) as a white solid.

¹H NMR (400 MHz, CD₃OD) δ ppm 0.74 - 0.83 (m, 9 H) 0.87 (d, *J*=6.69 Hz, 3 H) 0.91 (d, *J*=6.78 Hz, 3 H) 1.08 - 1.72 (m, 15 H) 1.71 - 2.00 (m, 5 H) 2.06 - 2.26 (m, 3 H) 2.26 - 2.42 (m, 1 H) 2.85 (s, 1 H) 3.41 (d, *J*=11.81 Hz, 1 H) 3.45 - 3.57 (m, 2 H) 3.72 - 3.93 (m, 2 H) 5.48 (d, *J*=5.66 Hz, 1 H) 5.62 (ddd, *J*=11.68, 9.82, 6.47 Hz, 1 H) 7.90 - 7.93 (m, 2 H) 8.11 (s, 1 H) 8.74 (dd, *J*=4.61, 1.49 Hz, 2 H).

### Step 2:

To a solution of the product from Step 1 (2.71 g, 4.40 mmol) in DMF (30 mL) was added potassium carbonate (12.0 g, 88.0 mmol) and 4-methoxybenzyl bromide (0.64 mL, 4.40 mmol). The mixture was stirred at room temperature overnight. The reaction mixture was poured into water (ca. 300 mL) and extracted with EtOAc. The combined organic layers were washed with saturated aqueous NaCl, dried (Na₂SO₄), and concentrated under reduced pressure. The residue was purified by flash chromatography, elution with 0 - 10% MeOH in CH₂Cl₂, to give Intermediate 38 (2.60 g, 80%) as a white solid.

¹H NMR (400 MHz, CDCl₃) δ 0.71 (d, *J*=7.17 Hz, 6 H) 0.78 (d, *J*=6.78 Hz, 3 H) 0.81 (d, *J*=6.69 Hz, 3 H) 0.87 (s, 3 H) 1.04 - 1.57 (m, 11 H) 1.68 - 1.92 (m, 4 H) 1.97 - 2.28 (m, 3 H) 2.59 (d, *J*=5.27 Hz, 1 H) 2.81 (s, 1 H) 3.41 (d, *J*=12.15 Hz, 1H) 3.44 (s, 2 H) 3.79 (s, 3 H) 3.89 (d, *J*=12.01 Hz, 1H) 4.04 (dd, *J*=9.37, 5.27 Hz, 1 H) 4.86 (d, *J*=11.96 Hz, 1 H) 5.03 (d, *J*=11.96 Hz, 1 H) 5.28 (d, *J*=5.66 Hz, 1H) 5.73 (td, *J*=10.30, 7.17 Hz, 1 H) 6.85 (q, *J*=4.82 Hz, 2 H) 7.25 (d, *J*=8.74 Hz, 2 H) 7.75 (dd, *J*=4.54, 1.46 Hz, 2 H) 7.98 (s, 1H) 8.74 (d, *J*=5.86 Hz, 2 H).

### EXAMPLES

Unless otherwise indicated, compounds described in the following examples which contain a basic amine group were isolated as trifluoroacetic acid salts. Thus, where applicable, reference to a reaction "to give the title compound " or "to provide the title compound", "to give" a particular Example Number, "title compound was prepared", and similar language, refers the title compound as a TFA salt. Conversion to the parent free amines may be accomplished by standard methods known in the art (e.g. neutralization with an appropriate inorganic base such as NaHCO₃). Other desired amine salts may be prepared in a conventional manner by reacting the free base with a suitable organic or inorganic acid. Alternatively, a desired amine salt may be prepared directly from the trifluoroacetic acid salt by employing an appropriate ion exchange resin.

### EXAMPLE 6 (PROCEDURES)

### (1S,4aR,6aS,7R,8R,10aR,10bR,12aR,14R,15R)-15-[[(2R)-2-amino-2,3-dimethylbutyl]oxy]-14-(3-bromo-4H-1,2,4-triazol-4-yl)-8-[(1R)-1,2-dimethylpropyl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid (EXAMPLE 6A)

### and

### (1S,4aR,6aS,7R,8R,10aR,10bR,12aR,14R,15R)-15-[[(2R)-2-amino-2,3-dimethylbutyl]oxy]-14-(5-bromo-1H-1,2,4-triazol-1-yl)-8-[(1R)-1,2-dimethylpropyl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid (EXAMPLE 6B)

### and

### (1S,4aR,6aS,7R,8R,10aR,10bR,12aR,14R,15R)-15-[[(2R)-2-amino-2,3-dimethylbutyl]oxy]-14-(3-bromo-1H-1,2,4-triazol-1-yl)-8-[(1R)-1,2-dimethylpropyl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid (EXAMPLE 6C)

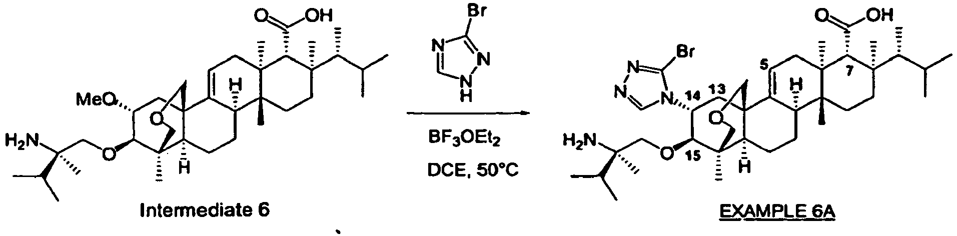

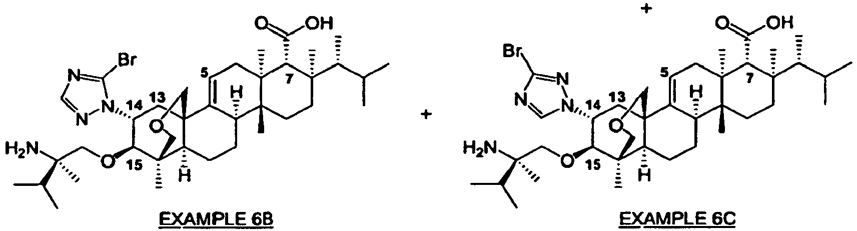

3-bromo-1H-1,2,4-triazole (32.1 mg, 0.217 mmol) and BF₃OEt₂ (54 µl, 0.426 mmol) were added to a stirred solution of Intermediate 6 (26.0 mg, 0.043 mmol) in 1,2-dichloroethane (0.43 ml). The reaction mixture was a light yellow solution that was heated to 50°C. After 1.75 hr, LCMS and ¹H NMR showed complete consumption of Intermediate 6. The reaction mixture was cooled to room temperature, the solvent was evaporated, and the resulting residue was placed under high vacuum. The residue was dissolved in methanol and separated using a single HPLC run on a 19 x 150 mm Sunfire Prep C18 OBD 10 µm column by eluting with acetonitrile/water + 0.1% TFA. The HPLC fractions containing the fastest eluting regioisomer were combined, the solvent was evaporated under reduced pressure, and the residue was lyophilized from ethanol and benzene to give EXAMPLE 6A (3.6 mg) as a white solid. The HPLC fractions containing the second eluting regioisomer were combined, the solvent was evaporated under reduced pressure, and the residue was lyophilized from ethanol and benzene to give EXAMPLE 6B (13.1 mg) as a white solid. The HPLC fractions containing the slowest eluting regioisomer were combined, the solvent was evaporated under reduced pressure, and the residue was lyophilized from ethanol and benzene to give EXAMPLE 6C (5.8 mg) as a white solid.

### EXAMPLE 6A:

¹H NMR (CD₃OD, 600 MHz, ppm) δ 0.76 (s, 3H, Me), 0.76 (d, 3H, Me), 0.81 (d, 3H, Me), 0.84 (d, 3H, Me), 0.85 (d, 3H, Me), 0.88 (s, 3H, Me), 0.89 (d, 3H, Me), 0.94 (s, 3H, Me), 1.16 (s, 3H, Me), 1.20 (s, 3H, Me), 1.22-1.35 (m), 1.40-1.44 (m), 1.46-1.65 (m), 1.73-1.96 (m), 2.11-2.22 (m), 2.43 (broad dd, 1H, H13), 2.79 (broad d, 1H), 2.84 (s, 1H, H7), 3.49 (d, 1H), 3.53 (d, 2H), 3.60 (d, 1H), 3.73 (broad d, 1H), 3.94 (d, 1H), 5.50 (dd, 1H, H5), 5.72-5.80 (broad m, 1H, H14), 9.29 (broad s, 1H, triazole).

Mass Spectrum: (ESI) *m*/*z* = 717.32 (719.32) (M+H).

### EXAMPLE 6B:

¹H NMR (CD₃OD, 600 MHz, ppm) δ 0.75 (s, 3H, Me), 0.76 (d, 3H, Me), 0.79 (d, 3H, Me), 0.83 (d, 3H, Me), 0.84 (d, 3H, Me), 0.88 (s, 3H, Me), 0.89 (d, 3H, Me), 0.91 (s, 3H, Me), 1.13 (s, 3H, Me), 1.19 (s, 3H, Me), 1.22-1.34 (m), 1.39-1.43 (m), 1.46-1.56 (m), 1.58-1.64 (m), 1.72-1.95 (m), 2.09-2.21 (m), 2.30 (dd, 1H, H13), 2.83 (s, 1H, H7), 2.85 (d, 1H), 3.48 (d, 1H), 3.48 (d, 1H), 3.54 (dd, 1H), 3.60 (d, 1H), 3.88 (d, 1H), 3.95 (d, 1H), 5.47 (dd, 1H, H5), 5.71-5.77 (m, 1H, H 14), 8.10 (s, 1H, triazole).

Mass Spectrum: (ESI) *m*/*z* = 717.32 (719.32) (M+H).

### EXAMPLE 6C:

¹H NMR (CD₃OD, 600 MHz, ppm) δ 0.76 (s, 3H, Me), 0.77 (d, 3H, Me), 0.82 (d, 3H, Me), 0.85 (d, 3H, Me), 0.86 (s, 3H, Me), 0.86 (d, 3H, Me), 0.89 (d, 3H, Me), 0.92 (s, 3H, Me), 1.16 (s, 3H, Me), 1.20 (s, 3H, Me), 1.22-1.34 (m), 1.39-1.44 (m), 1.48-1.65 (m), 1.76-1.96 (m), 2.11-2.22 (m), 2.41 (dd, 1H, H13), 2.72 (d, 1H), 2.84 (s, 1H, H7), 3.47 (d, 1H), 3.50 (d, 1H), 3.52 (dd, 1H), 3.58 (d, 1H), 3.72 (d, 1H), 3.91 (d, 1H), 5.48 (dd, 1H, H5), 5.51-5.57 (m, 1H, H14), 8.52 (s, 1H, triazole).

Mass Spectrum: (ESI) *m*/*z* = 717.32 (719.32) (M+H).

### EXAMPLE 11 (INTERMEDIATE)

### (1S,4aR,6aS,7R,8R,10aR,10bR,12aR,14R,15R,15-[[(2R)-2-amino-2,3,3-trimethylbutyl]oxy]-14-(5-bromo-1H-1,2,4-triazol-1-yl)-8-[(1R)-1,2-dimethylpropyl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

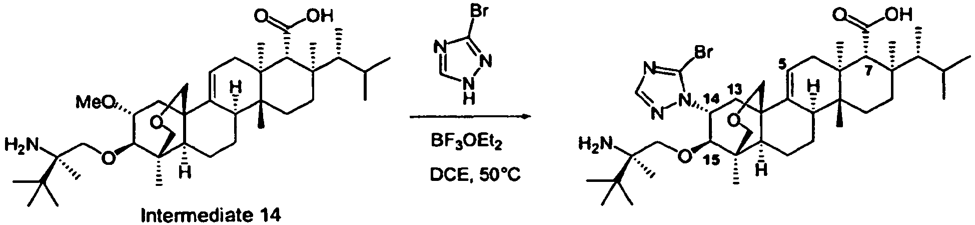

By a procedure analogous to that described in Example 6, but starting with Intermediate 14, the title compound was prepared and isolated as a white solid.

¹H NMR (CD₃OD, 500 MHz, ppm) δ 0.77 (d, 3H, partially obscured), 0.77 (s, 3H), 0.86 (d, 3H, partially obscured), 0.86 (s, 9H), 0.90 (d, 3H, partially obscured), 0.90 (s, 3H), 0.93 (s, 3H), 1.15 (s, 3H), 1.21 (s, 3H), 1.22 - 1.67 (m), 1.78 - 1.98 (m), 2.14 (m, 1H), 2.19 (m, 1H), 2.32 (dd, J=13.5 Hz, 6.4 Hz, 1H), 2.84 (s, 1H), 2.91 (d, J=10.1 Hz, 1H), 3.51 (d, J=11.8 Hz, 1 H), 3.56 (dd, J=11.7 Hz, 1.9 Hz, 1H), 3.62 (d, J=11.6 Hz, 1H), 3.65 (d, J=9.8 Hz, 1H), 3.93 (d, J=9.8 Hz, 1H), 3.99 (d, J=11.9 Hz, 1H), 5.49 (m, 1H), 5.78 (m, 1H), 8.08 (s, 1H).

Mass spectrum: (ESI) *m*/*z* = 733.58, 735.57 (M+H).

### EXAMPLE 37

### (1S,4aR,6aS,7R,8R,10aR,10bR,12aR,14R,15R)-15-[[(2R)-2-amino-2,3-dimethylbutyl]oxy]-8-[(1R)-1,2-dimethylpropyl]-14-[5-[[(1-methylethyl)amino]carbonyl]-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid (EXAMPLE 37C)

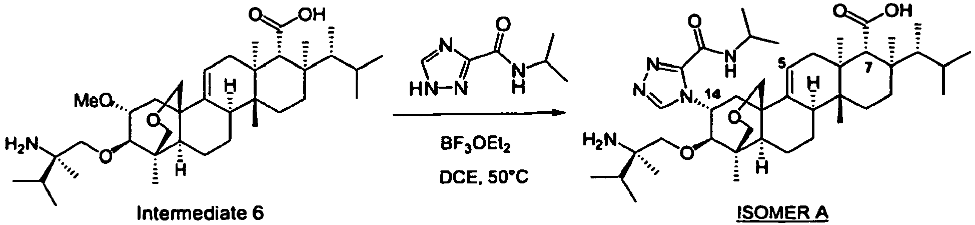

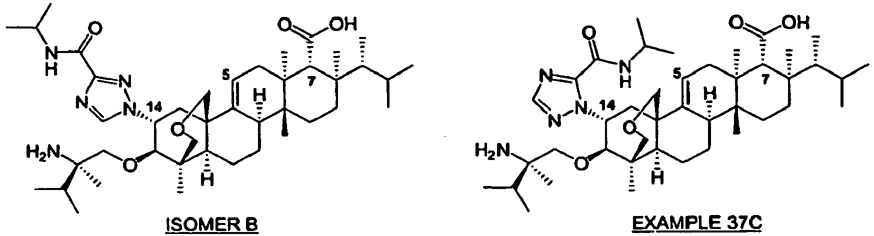

N-(1-methylethyl)-1H-1,2,4-triazole-3-carboxamide (42.0 mg, 0.272 mmol) and BF₃OEt₂ (68 µl, 0.537 mmol) were added to a stirred solution of Intermediate 6 (32.6 mg, 0.054 mmol) in 1,2-dichloroethane (1.0 ml). The reaction mixture was a pale yellow solution that was heated to 50°C. After 4.5 hr, LCMS and ¹H NMR showed about 75% conversion of Intermediate 6 to a mixture of the three triazole regioisomers at C14. After 4.75 hours, additional BF₃OEt₂ (30 µl, 0.237 mmol) was added to the reaction mixture. After 6 hr, the reaction mixture was cooled to room temperature, the solvent was evaporated, and the resulting residue was placed under high vacuum. The residue was dissolved in methanol and separated using a single HPLC run on a 19 x 150 mm Sunfire Prep C18 OBD 10 µm column by eluting with acetonitrile/water + 0.1% TFA. The total flow rate was 20 ml/min and the HPLC method employed a 12 minute 20%-100% acetonitrile/water gradient followed by a 6 minute acetonitrile flush. The HPLC fractions of the fastest eluting regioisomer were combined, the solvent was evaporated under reduced pressure, and the residue was lyophilized from ethanol and benzene to give Isomer A (4.3 mg) as a white solid. The HPLC fractions of the second eluting regioisomer were combined, the solvent was evaporated under reduced pressure, and the residue was lyophilized from ethanol and benzene to give Isomer B (2.5 mg) as a white solid. The HPLC fractions of the slowest eluting regioisomer were combined, the solvent was evaporated under reduced pressure, and the residue was lyophilized from ethanol and benzene to give EXAMPLE 37C (10.5 mg) as a white solid.

### EXAMPLE 37C:

¹H NMR (CD₃OD, 500 MHz, ppm) δ 0.78 (s, 3H, Me), 0.79 (d, 3H, Me), 0.81 (d, 3H, Me), 0.82 (s, 3H, Me), 0.86 (d, 3H, Me), 0.88 (d, 3H, Me), 0.89 (s, 3H, Me), 0.92 (d, 3H, Me), 1.16 (s, 3H, Me), 1.22 (s, 3H, Me), 1.24-1.37 (m), 1.25 (d, 3H, Me), 1.28 (d, 3H, Me), 1.41-1.47 (m), 1.49-1.58 (m), 1.60-1.68 (m), 1.76-1.98 (m), 2.12-2.25 (m), 2.41 (dd, 1H, H13), 2.76 (d, 1H), 2.86 (s, 1H, H7), 3.48 (d, 1H), 3.52 (d, 1H), 3.54 (dd, 1H), 3.63 (d, 1H), 3.93 (d, 1H), 3.96 (d, 1H), 4.15-4.23 (m, 1H, CONCH), 5.48 (dd, 1H, H5), 6.61-6.69 (m, 1H, H 14), 8.04 (s, 1H, triazole), 8.56 (d, 1H, CONH).

Mass Spectrum: (ESI) *m*/*z* = 724.35 (M+H).

### EXAMPLE 38

### (1S,4aR,6aS,7R,8R,10aR,10bR,12aR,14R,14R)-15-[[(2R)-2,3-dimethyl-2-(methylamino)butyl]oxy]-8-[(1R)-1,2-dimethylpropyl]-14-[5-[[(1-methylethyl)amino]carbonyl]-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

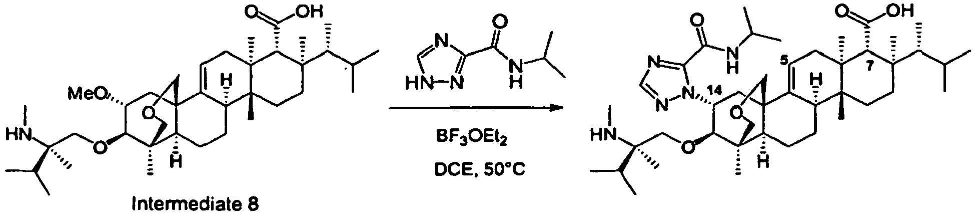

N-(1-methylethyl)-1*H*-1,2,4-triazole-3-carboxamide (753.1 mg, 4.88 mmol) and BF₃OEt₂ (1.3 ml, 10.26 mmol) were added to a stirred solution of Intermediate 8 (602.2 mg, 0.978 mmol) in 1,2-dichloroethane (14.0 ml). The reaction mixture was a white suspension that was heated to 50°C. After 6 hr, LCMS and ¹H NMR showed complete consumption of. Intermediate 8. The reaction mixture was cooled to room temperature and partitioned between ethyl acetate (100 ml) and saturated NaHCO₃ (50 ml). The aqueous layer was extracted with ethyl acetate (1 x 50 ml). The organic layers were combined, dried over magnesium sulfate, and filtered. The solvent was evaporated under reduced pressure to give a light yellow residue. The residue was dissolved in methanol and purified using 15 HPLC runs (∼40 mg / run) on a 19 x 150 mm Sunfire Prep C 18 OBD 10 µm column by eluting with acetonitrile/water + 0.1 % TFA. The total flow rate was 15 ml/min and the HPLC method employed a 12 minute 20%-100% acetonitrile/water gradient followed by a 7 minute acetonitrile flush. The HPLC fractions containing the desired product were combined and the solvent was evaporated under reduced pressure to give the title compound (450 mg) as a colorless residue.

¹H NMR (CD₃OD, 600 MHz, ppm) δ 0.74 (s, 3H, Me), 0.76 (s, 3H, Me), 0.77 (d, 3H, Me), 0.81 (d, 3H, Me), 0.85 (d, 3H, Me), 0.86 (d, 3H, Me), 0.89 (s, 3H, Me), 0.89 (d, 3H, Me), 1.13 (s, 3H, Me), 1.20 (s, 3H, Me), 1.22-1.37 (m), 1.24 (d, 3H, Me), 1.26 (d, 3H, Me), 1.39-1.44 (m), 1.47-1.54 (m), 1.58-1.65 (m), 1.78-1.95 (m), 1.98-2.03 (m), 2.09-2.21 (m), 2.38 (dd, 1H, H13), 2.40 (s, 3H, NMe), 2.83 (s, 1H, H7), 2.92 (d, 1H), 3.47 (d, 1H), 3.52 (dd, 1H), 3.61 (d, 1H), 3.63 (d, 1H), 3.89 (d, 1H), 4.02 (d, 1H), 4.14-4.20 (m, 1H, CONCH), 5.45 (dd, 1H, H5), 6.58-6.65 (m, 1H, H14), 8.02 (s, 1H, triazole), 8.56 (d, 1H, CONCH).

Mass Spectrum: (ESI) *m*/*z* = 738.78 (M+H).

### EXAMPLE 39

### (15,4aR,6aS,7R,8R,10aR,10bR,12aR,14R,15R)-15-[[(2R)-2,3-dimethyl-2-(methylamino)butyl]oxy]-8-[(1R)-1,2-dimethylpropyl]-14-[5-[[(1-methylethyl)amino)carbonyl]-1H-1,2,4-triazol-1-yl]-1,6,6a,7,8,9,10,10a,10b,11,12,12a-dodecahydro-1,6a,8,10a-tetramethyl-6-oxo-4H-1,4a-propano-2H-phenanthro[1,2-c]pyran-7-carboxylic acid

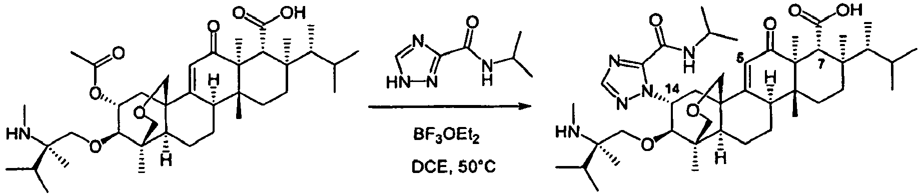

N-(1-methylethyl)-1*H*-1,2,4-triazole-3-carboxamide (82.3 mg, 0.534 mmol) and BF₃OEt₂ (200 µl, 1.578 mmol) were added to a stirred solution of (1*S*,4a*R*,6a*S*,7*R*,8*R*,10a*R*,10b*R*,12a*R*,14*R*,15*R*)-14-(acetyloxy)-15-[[(2*R*)-2,3-dimethyl-2-(methylamino)butyl]oxy]-8-[(1*R*)-1,2-dimethylpropyl]-1,6,6a,7,8,9,10,10a,10b,11,12,12a-dodecahydro-1,6a,8,10a-tetramethyl-6-oxo-4*H*-1,4a-propano-2*H*-phenanthro[1,2-c]pyran-7-carboxylic acid (free base form of Example 152 in International Patent Publication No. WO 2007/127012, 70.2 mg, 0.107 mmol) in 1,2-dichloroethane (1.5 ml). The reaction mixture was a light amber solution that was heated to 50 °C. After 50 hours, the reaction mixture was cooled to room temperature, the solvent was evaporated under reduced pressure, and the resulting residue was placed under high vacuum.

The residue was dissolved in methanol and purified using two HPLC runs (∼35 mg / run) on a 19 x 150 mm Sunfire Prep C18 OBD 10 µm column by eluting with acetonitrile/water + 0.1% TFA. The total flow rate was 15 ml/min and the HPLC method employed a 12 minute 20%-100% acetonitrile/water gradient followed by a 7 minute acetonitrile flush. The product HPLC fractions were combined, the solvent was evaporated under reduced pressure, and the residue was lyophilized from ethanol and benzene to give the title compound (24.2 mg, 0.028 mmol) as a white solid.

¹H NMR (CD₃OD, 600 MHz, ppm) δ 0.75 (s, 3H, Me), 0.76 (d, 3H, Me), 0.80 (s, 3H, Me), 0.82 (d, 3H, Me), 0.86 (d, 3H, Me), 0.87 (d, 3H, Me), 0.92 (s, 3H, Me), 0.94 (d, 3H, Me), 1.08 (s, 3H, Me), 1.24 (d, 3H, Me), 1.26 (d, 3H, Me), 1.28-1.33 (m), 1.36-1.48 (m), 1.52-1.56 (m), 1.66-1.72 (m), 1.67 (s, 3H, Me), 1.74-1.81 (m), 1.87-2.04 (m), 2.18-2.25 (m), 2.41 (s, 3H, NMe), 2.43 (dd, 1H, H13), 2.68-2.73 (m), 2.92 (d, 1H), 3.09 (s, 1H, H7), 3.50 (d, 1H), 3.60 (dd, 1H), 3.64 (d, 1H), 3.67 (d, 1H), 3.94 (d, 1H), 4.06 (d, 1H), 4.14-4.22 (m, 1H, CONCH), 5.74 (d, 1H, H5), 6.64-6.72 (m, 1H, H 14), 8.04 (s, 1H, triazole), 8.58 (d, 1 H, CONH).

Mass Spectrum: (ESI) *mlz* = 752.73 (M+H).

### EXAMPLE 40

### (1S,4aR,6aS,7R,8R,10aR,10bR,12aR,14R,15R)-15-[[(2R)-2,3-dimethyl-2-(methylamino)butyl]oxy]-8-[(1R)-1,2-dimethylpropyl]-14-[5-[(methylamino)carbonyl]-1H-1,2,4-triazol-1-yl]-1,6,6a,7,8,9,10,10a,10b,11,12,12a-dodecahydro-1,6a,8,10a-tetramethyl-6-oxo-4H-1,4a-propano-2H-phenanthro[1,2-c]pyran-7-carboxylic acid

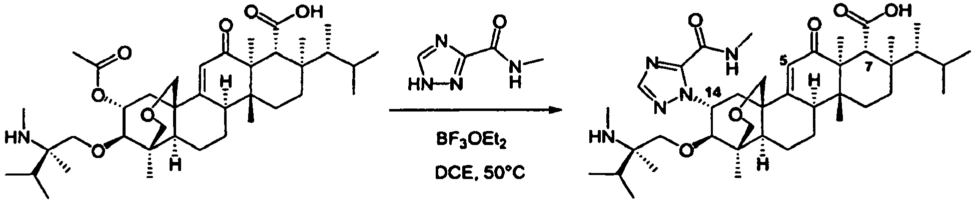

By a procedure analogous to that described for Example 39, but employing N-methyl-1*H-*1,2,4-triazole-3-carboxamide, the title compound was prepared and isolated as a white solid.

¹H NMR (CD₃OD, 600MHz, ppm) δ 0.73 (s, 3H, Me), 0.76 (d, 3H, Me), 0.80 (s, 3H, Me), 0.81 (d, 3H, Me), 0.85 (d, 3H, Me), 0.87 (d, 3H, Me), 0.93 (s, 3H, Me), 0.94 (d, 3H, Me), 1.08 (s, 3H, Me), 1.30 (m), 1.40 (m), 1.46 (m), 1.52-1.56 (m), 1.67 (s, 3H, Me), 1.68-1.80 (m), 1.84-2.03 (m), 2.22 (m), 2.40 (s, 3H, NMe), 2.43 (dd, 1H, H 13), 2.54 (d, 1H), 2.70 (m), 2.90 (s, 3H, CONMe), 2.95 (d, 1H), 3.08 (s, 1H, H7), 3.49 (d, 1H), 3.59 (dd, 1H), 3.64 (d, 1H), 3.68 (d, 1H), 3.94 (d, 1H), 4.08 (d, 1H), 5.74 (d, 1H, H5), 6.74 (m, 1H, H14), 8.02 (d, 1H, triazole H).

Mass spectrum: (ESI) *m*/*z* = 724.70 (M+H)

### EXAMPLE 41

### (1S,4aR,6aS,7R,8R,10aR,10bR,12aR,14R,15R)-14-[5-(aminocarbonyl)-1H-1,2,4-triazol-1-yl]-15-[[(2R)-2-amino-2,3-dimethylbutyl]oxy]-8-[(1R)-1,2-dimethylpropyl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

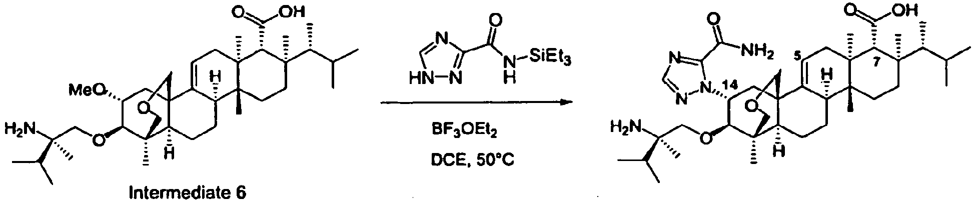

N-(triethylsilyl)-1*H*-1,2,4-triazole-3-carboxamide (78.6 mg, 0.347 mmol) and BF₃OEt₂ (130 µl, 1.026 mmol) were added to a stirred solution of Intermediate 6 (41.6 mg, 0.069 mmol) in 1,2-dichloroethane (1.15 ml). The reaction mixture was a tan suspension that was heated to 50°C. After 2 hr, LCMS and ¹H NMR showed complete consumption of Intermediate 6. The reaction mixture was cooled to room temperature, the solvent was evaporated, and the resulting residue was placed under high vacuum. The residue was dissolved in methanol and purified using a single HPLC run on a 19 x 150 mm Sunfire Prep C18 OBD 10 µm column by eluting with acetonitrile/water + 0.1% TFA. The total flow rate was 15 ml/min and the HPLC method employed a 12 minute 20%-100% acetonitrile/water gradient followed by a 7 minute acetonitrile flush. The HPLC fractions containing the desired product were combined, the solvent was evaporated under reduced pressure, and the residue was lyophilized from ethanol and benzene to give the title compound (9.0 mg) as a white solid.

¹H NMR (CD₃OD, 600 MHz, ppm) δ 0.76 (s, 3H, Me), 0.77 (d, 3H, Me), 0.80 (d, 3H, Me), 0.80 (s, 3H, Me), 0.84 (d, 3H, Me), 0.85 (d, 3H, Me), 0.87 (s, 3H, Me), 0.89 (d, 3H, Me), 1.14 (s, 3H, Me), 1.20 (s, 3H, Me), 1.22-1.35 (m), 1.39-1.44 (m), 1.47-1.55 (m), 1.58-1.65 (m), 1.73-1.95 (m), 2.09-2.21 (m), 2.39 (dd, 1H, H13), 2.80 (d, 1H), 2.84 (s, 1H, H7), 3.45 (d, 1H), 3.51 (d, 1H), 3.51 (dd, 1H), 3.60 (d, 1H), 3.93 (d, 1H), 3.95 (d, 1H), 5.46 (dd, 1H, H5), 6.64-6.71 (m, 1H, H14), 8.02 (s, 1H, triazole).

Mass Spectrum: (ESI) *m*/*z* = 682.71 (M+H).

### EXAMPLE 42

### (1S,4aR,6aS,7R,8R,10aR,10bR,12aR,14R,15R)-14-[5-(aminocarbonyl)-1H-1,2,4-triazol-1-yl]-15-[[(2R)-2-(dimethylamino)-2,3-dimethylbutyl]oxy]-8-[(1R)-1,2-dimethylpropyl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

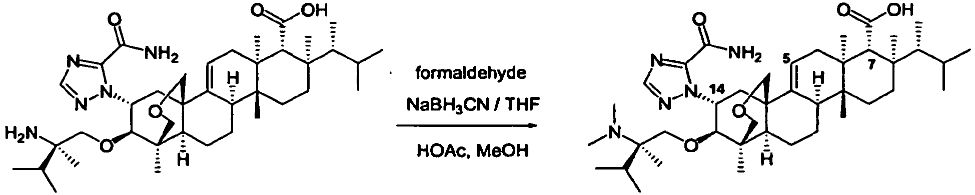

Acetic acid (2 µl, 0.035 mmol), formaldehyde 37% in water (5.4 µl, 0.073 mmol), and sodium cyanoborohydride 1.0 M in THF (67 µl, 0.067 mmol) were added to a stirred hazy solution of (1*S*,4a*R*,6a*S*,7*R*,8*R*,10a*R*,10b*R*,12a*R*,14*R*,15*R*)-14-[5-(aminocarbonyl)-1*H*-1,2,4-triazol-1-yl]-15-[[(2*R*)-2-amino-2,3-dimethylbutyl]oxy]-8-[(1*R*)-1,2-dimethylpropyl]-1,6,6a,7,8,9,10,10a,10b,11,12,12a-dodecahydro-1,6a,8,10a-tetramethyl-4*H*-1,4a-propano-2*H-*phenanthro[1,2-c]pyran-7-carboxylic acid (Example 41, 13.3 mg, 0.017 mmol) in methanol (0.33 ml). The reaction mixture was a hazy solution. After 6 hr, LCMS showed complete consumption of starting material. The reaction mixture was diluted with methanol, the solvent was evaporated under reduced pressure, and the resulting residue was placed under high vacuum. The residue was dissolved in methanol and purified using a single HPLC run on a 19 x 150 mm Sunfire Prep C18 OBD 10 µm column by eluting with acetonitrile/water + 0.1% TFA. The total flow rate was 15 ml/min and the HPLC method employed a 12 minute 20%-100% acetonitrile/water gradient followed by a 7 minute acetonitrile flush. The product HPLC fractions were combined and the solvent was evaporated under reduced pressure to give the title compound as a colorless residue.

¹H NMR (CD₃OD, 600 MHz, ppm) δ 0.75 (s, 3H, Me), 0.76 (d, 3H, Me), 0.85 (d, , 3H, Me), 0.86 (s, 3H, Me), 0.89 (d, 3H, Me), 0.92 (s, 3H, Me), 0.93 (d, 3H, Me), 0.94 (d, 3H, Me), 1.12 (s, 3H, Me), 1.19 (s, 3H, Me), 1.21-1.34 (m), 1.39-1.43 (m), 1.46-1.54 (m), 1.58-1.64 (m), 1.71-1.94 (m), 2.08-2.13 (m), 2.14-2.21 (m), 2.24-2.31 (m), 2.37 (dd, 1H, H13), 2.64 (s, 3H, NMe), 2.71 (s, 3H, NMe), 2.83 (s, 1H, H7), 3.14 (d, 1H), 3.49 (d, 1H), 3.50 (dd, 1H), 3.60 (d, 1H), 3.69 (d, 1H), 3.75 (d, 1H), 4.06 (d, 1H), 5.43 (dd, 1H, H5), 6.63-6.70 (m, 1H, H 14), 8.05 (s, 1H, triazole).

Mass Spectrum: (ESI) *m*/*z* = 710.66 (M+H).

### EXAMPLE 43

### (1S,4aR,6aS,7R,8R,10aR,10bR,12aR,14R,15R)-14-[5-(aminocarbonyl)-1H-1,2,4-triazol-1-yl]-8-[(1R)-1,2-dimethylpropyl]-15-[[(2R)-2-(ethylamino)-2,3-dimethylbutyl]oxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

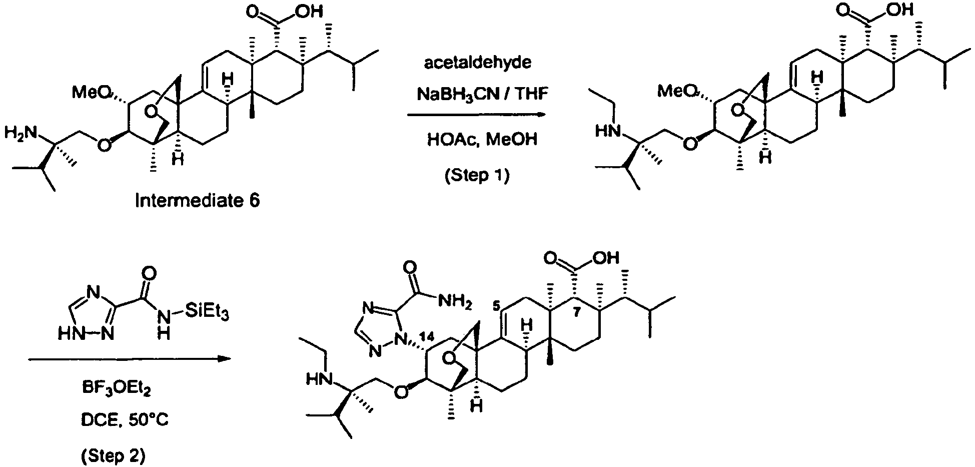

### Step 1:

Acetic acid (65 µl, 1.135 mmol), acetaldehyde (0.41 ml, 7.26 mmol), and sodium cyanoborohydride 1.0 M in THF (2.3 ml, 2.30 mmol) were added to a stirred solution of Intermediate 6 (338.0 mg, 0.562 mmol) in methanol (5.6 ml). The reaction mixture was a colorless solution. After 4 hours, LCMS showed complete consumption of Intermediate 6. The reaction mixture was partitioned between ethyl acetate (100 ml) and water (100 ml). The aqueous layer was extracted with ethyl acetate (1 x 50 ml). The organic layers were combined, dried over magnesium sulfate, and filtered. The solvent was evaporated under reduced pressure and the residue was lyophilized from ethanol and benzene to give the product (337.5 mg) as a white solid.

¹H NMR (CD₃OD, 500 MHz, ppm) δ 0.78 (s, 3H, Me), 0.79 (d, 3H, Me), 0.82 (s, 3H, Me), 0.88 (d, 3H, Me), 0.92 (d, 3H, Me), 1.02 (d, 3H, Me), 1.04 (d, 3H, Me), 1.16 (s, 3H, Me), 1.19 (s, 3H, Me), 1.23 (s, 3H, Me), 1.24-1.35 (m), 1.38 (t, 3H), 1.40-1.46 (m), 1.48-1.55 (m), 1.58-1.68 (m), 1.72-1.88 (m), 1.95-2.02 (m), 2.08-2.14 (m), 2.18-2.25 (m), 2.27-2.35 (m), 2.60 (dd, 1H, H13), 2.87 (s, 1H, H7), 2.98 (d, 1H), 3.07-3.15 (m, 1H, NCH₂), 3.28-3.35 (obscured m, 1H, NCH₂), 3.37 (d, 1H), 3.38 (s, 3H, OMe), 3.44 (s, 2H), 3.70 (d, 1H), 3.72 (d, 1 H), 3.90 (d, 1H), 4.22-4.29 (m, 1H, H14), 5.57 (dd, 1H, H14).

Mass Spectrum: (ESI) *m*/*z* = 630.58 (M+H).

### Step 2:

N-(triethylsilyl)-*1H-*1,2,4-triazole-3-carboxamide (220.0 mg, 0.972 mmol) and BF₃OEt₂ (0.37 ml, 2.92 mmol) were added to a stirred solution of the product compound from Step 1 (122.8 mg, 0.195 mmol) in 1,2-dichloroethane (3.2 ml). The reaction mixture was a light tan suspension that was heated to 50°C. After 2.5 hr, LCMS and ¹H NMR showed complete consumption of starting material. The reaction mixture was cooled to room temperature, the solvent was evaporated, and the resulting residue was placed under high vacuum. The residue was taken up in methanol and the resulting white suspension was filtered (0.45 µm syringe filter) before being purified using three HPLC runs (∼41 mg / run) on a 19 x 150 mm Sunfire Prep C18 OBD 10 µm column by eluting with acetonitrile/water + 0.1% TFA. The total flow rate was 15 ml/min and the HPLC method employed a 12 minute 20%-100% acetonitrile/water gradient followed by a 7 minute acetonitrile flush. The product HPLC fractions were combined, the solvent was evaporated under reduced pressure, and the residue was lyophilized from ethanol and benzene to give the title compound (21.6 mg) as a white solid.

¹H NMR (CD₃OD, 600 MHz, ppm) δ 0.73 (s, 3H, Me), 0.75 (s, 3H, Me), 0.76 (d, 3H, Me), 0.79 (d, 3H, Me), 0.85 (d, 3H, Me), 0.88 (d, 3H, Me), 0.88 (s, 3H, Me), 0.89 (d, 3H, Me), 1.12 (s, 3H, Me), 1.20 (s, 3H, Me), 1.21-1.35 (m), 1.26 (t, 3H), 1.39-1.44 (m), 1.46-1.54 (m), 1.58-1.65 (m), 1.74-1.95 (m), 2.01-2.07 (m), 2.08-2.13 (m), 2.14-2.22 (m), 2.38 (dd, 1H, H 13), 2.66-2.73 (m, 1H, NCH₂), 2.80-2.87 (m, 1H, NCH₂), 2.83 (s, 1H, H7), 2.96 (d, 1H), 3.48 (d, 1H), 3.51 (dd, 1H), 3.60 (d, 1H), 3.65 (d, 1H), 3.87 (d, 1H), 4.06 (d, 1H), 5.44 (dd, 1H, H5), 6.64-6.72 (m, 1H, H14), 8.03 (s, 1H, triazole).

Mass Spectrum: (ESI) *m*/*z* = 710.66 (M+H).

### EXAMPLE 44

### (1S,4aR,6aS,7R,8R,10aR,10bR,12aR,14R,15R)-14-[5-(aminocarbonyl)-1H-1,2,4-triazol-l-yl]-15-[[(2R)-2-amino-2,3,3-trimethylbutyl]oxy]-8-[(1R)-1,2-dimethylpropyl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

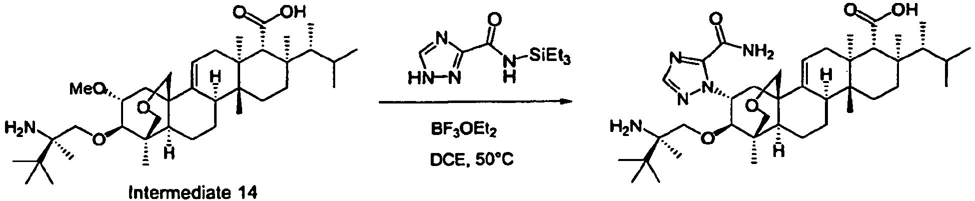

A solution of Intermediate 14 (520 mg, 0.844 mmol) in dichloroethane (5.7 mL) was treated with N-(triethylsilyl)-*1H-*1,2,4-triazole-3-carboxamide (669 mg, 2.95 mmol) then BF₃OEt₂ (1.07 mL, 8.44 mmol) and this mixture was heated to 50°C under nitrogen. After 2 hours the reaction was cooled to room temperature and quenched with sat. NaHCO₃. The mixture was partitioned between ethyl acetate and sat. NaHCO₃ and the organic phase was washed with water (a small amount of methanol was added to help dissolve a precipitate). The organic phase was dried (MgSO₄) and concentrated *in vacuo* to give a white solid. Purification was accomplished by preparative HPLC (30 x 100 mm Waters Sunfire column, 5 µm, UV-detection, 10-100% MeCN/water with 0.05% TFA over 12 minutes, 5 runs). The product fractions (the desired product is the third regioisomer to elute under these conditions) were combined and partially concentrated by rotovap then frozen and lyophilized to give the title compound (255 mg) as a white amorphous solid (trifluoroacetate salt).

¹H NMR (CD₃OD, 500 MHz, ppm) δ 0.77 (s, 3H), 0.78 (d, 3H, partially obscured), 0.81 (s, 3H), 0.85 (s, 9H), 0.86 (d, 3H, partially obscured), 0.89 (s, 3H), 0.90 (d, 3H, partially obscured), 1.14 (s, 3H), 1.21 (s, 3H), 1.22 -1.67 (m), 1.78 - 1.97 (m), 2.14 (m, 1 H), 2.19 (m, 1H), 2.41 (dd, J=13.3 Hz, 6.4 Hz, 1H), 2.84 (d, J=9.8 Hz, 1H), 2.84 (s, 1H), 3.46 (d, J=11.8 Hz, 1H), 3.52 (d, J=11.4 Hz, 1.8Hz, 1H), 3.62 (d, J=11.6 Hz, 1H), 3.70 (d, J=9.8 Hz, 1H), 3.94 - 4.0 (m, 2H), 5.47 (m, 1H), 6.73 (m, 1H), 8.02 (s, 1H).

Mass Spectrum: m/z = 696.50 (M + H).

### CONVERSION OF EXAMPLE 44 TO HYDROCHLORIDE SALT

Example 44 TFA salt (255 mg, 0.315 mmol) was dissolved in methanol (1.5 mL) and the solution was diluted with 3 mL of 1:1 MeCN/H₂O. The solution was loaded onto a column of Dowex® 1X8 chloride form ion exchange resin (10 g, ∼1.8 meq/g) and the column was eluted with 1:1 MeCN/H₂O (∼1.5 column volumes). The eluant was concentrated *in vacuo* to remove most of the acetonitrile and then frozen and lyophilized to give 226 mg of the hydrochloride salt as a white solid.

### EXAMPLE 45

### (1S,4aR,6aS,7R,8R,10aR,10bR,12aR,14R,15R)-8-[(1R)-1,2-dimethylpropyl]-14-[5-[(methylamino)carbonyl]-1H-1,2,4-triazol-1-yl]-15-[[(2R)-2,3,3-trimethyl-2-(methylamino)butyl]oxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

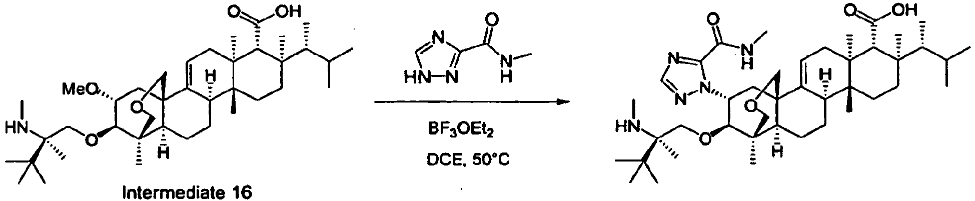

A solution of Intermediate 16 (75 mg, 0.11 mmol) in dichloroethane (1.1 mL) was treated with N-methyl-*1H*-1,2,4-triazole-3-carboxamide (75 mg, 0.65 mmol) then BF₃OEt₂ (0.15 mL, 1.1 mmol) and this mixture was heated to 50°C under nitrogen. After 1.5 hours the reaction was cooled to room temperature then concentrated *in vacuo.* The crude product mixture was suspended in methanol (3 mL) and filtered through a sintered glass funnel. The filtrate was purified by preparative HPLC (19x100 mm Waters Sunfire column, 5µm, UV-detection, 30-100% MeCN/water with 0.05% TFA over 20 minutes). The product fractions (the desired product is the third regioisomer to elute under these conditions) were combined and partially concentrated by rotovap then frozen and lyophilized to give the title compound (38 mg) as a white amorphous solid.

¹H NMR (CD₃OD, 500 MHz, ppm) δ 0.76 (s, 3H), 0.77 (d, 3H, partially obscured), 0.80 (s, 3H), 0.85 (d, J=6.8 Hz, 3H), 0.90 (d, J=7.0 Hz, 3H), 0.91 (s, 9H), 0.93 (s, 3H), 1.13 (s, 3H), 1.20 (s, 3H), 1.22 - 1.56 (m), 1.62 (m, 1H), 1.75 - 1.96 (m), 2.12 (m, 1H), 2.19 (m, 1H), 2.39 (dd, J=13.5 Hz, 6.5 Hz, 1H), 2.53 (s, 3H), 2.84 (s, 1H), 2.92 (d, J=4.3 Hz, 3H), 3.12 (d, J=11.2 Hz, 1H), 3.51 (d, J=11.8 Hz, 1H), 3.52 (dd, J=11.4 Hz, 2.1 Hz, 1H), 3.62 (d, 11.4 Hz, 1H), 3.78 (d, J=11.8 Hz, 1H), 3.84 (d, J=11.2 Hz, 1H), 4.03 (d, J=9.9 Hz, 1Hz, 5.45 (m, 1H), 6.65 (m, 1H), 8.02 (s, 1H), 8.79 (m, 1H).

Mass Spectrum: m/z = 724.79 (M + H).

### EXAMPLE 46

### (1S,4aR,6aS,7R,8R,10aR,10bR,12aR,14R,15R)-14-[5-(aminocarbonyl)-1H-1,2,4-triazol-1-yl]-8-[(1R)-1,2-dimethylpropyl]-15-[[tetrahydro-4-(methylamino)-2H-pyran-4-yl]methoxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

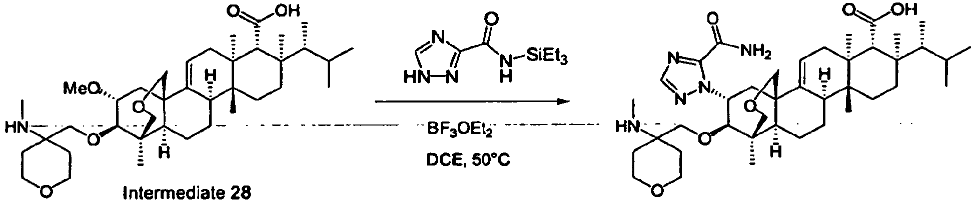

A solution of Intermediate 28 (180 mg, 0.286 mmol) in dichloroethane (1.3 mL) was treated with N-(triethylsilyl)-*1H-*1,2,4-triazole-3-carboxamide (323 mg, 1.43 mmol) then BF₃OEt₂ (0.36 mL, 2.86 mmol) and this mixture was heated to 55° C under nitrogen. After 1 hour the reaction was cooled to room temperature then concentrated *in vacuo.* The crude product mixture was suspended in methanol (3 mL) and filtered through a sintered glass funnel. The filtrate was purified by preparative HPLC (19x100 mm Waters Sunfire column, 5µm, UV-detection, 30-100% MeCN/water with 0.05% TFA over 20 minutes). The fractions containing the desired product (the desired product is the third regioisomer to elute under these conditions) were combined and partially concentrated by rotovap then frozen and lyophilized to give the title compound (80 mg) as a white amorphous solid.

¹H NMR (CD₃OD, 500 MHz, ppm) δ 0.77 (s, 3H), 0.78 (d, 3H, partially obscured), 0.87 (d, J=6.6 Hz, 3H), 0.91 (d, 3H, partially obscured), 0.92 (s, 3H), 1.14 (s, 3H), 1.22 (s, 3H), 1.23 -1.58 (m), 1.64 (m, 2H), 1.74 - 1.98 (m), 2.13 (m, 1H), 2.20 (m, 1 H), 2.38 (dd, partially obscured, 1H), 2.40 (s, 3H), 2.85 (s, 1H), 2.94 (m, 1H), 3.35 (t, 2H, partially obscured), 3.49 (d, 1H, partially obscured), 3.52 (dd, 1H, partially obscured), 3.61 (d, 1H, partially obscured),3.62 (m, 1H, partially obscured), 3.80 (m, 1H, partially obscured), 3.85(m, 2H, partially obscured), 4.15 (d, J=9.9 Hz, 1H), 5.46 (m, 1H), 6.65 (m, 1H), 8.08 (s, 1H).

Mass Spectrum m/z = 710.67 (M + H).

### EXAMPLES 47-62

The following compounds were prepared using methods analogous to those described in the preceding examples:

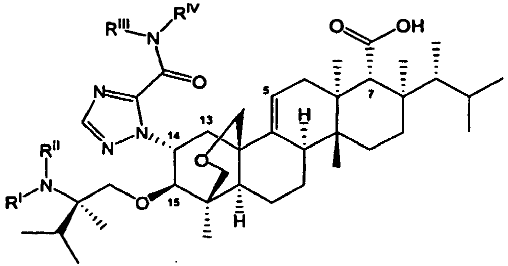

| | | |
|---|---|---|
| 47 | R^{I} = Me | (1*S*,4a*R*,6a*S*,7*R*,8*R*,10a*R*,10b*R*,12a*R*,14*R*,15*R*)-14-[5-(aminocarbonyl)-*1H*-1,2,4-triazol-1-yl]-8-[(1*R*)-1,2-dimethylpropyl]-15-[[(2*R*)-2,3-dimethyl-2-(methylamino)butyl]oxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = H | |
| | R^{IV} = H | |
| ¹H NMR (CD₃OD, 600MHz, ppm) δ 0.74 (s, 3H, Me), 0.76 (s, 3H, Me), 0.76 (d, 3H, Me), 0.82 (d, 3H, Me), 0.85 (d, 3H, Me), 0.86 (d, , 3H, Me), 0.90 (d, 3H, Me), 0.90 (s, 3H, Me), 1.12 (s, , 3H, Me), 1.20 (s, 3H, Me), 1.22-1.35 (m), 1.39-1.44 (m), 1.47-1.55 (m), 1.58-1.65 (m), 1.75-1.95 (m), 1.98-2.05 (m), 2.09-2.21 (m), 2.39 (dd, 1H, H 13), 2.40 (s, 3H, NMe), 2.83 (s, 1H, H7), 2.96 (d, 1H), 3.47 (d, 1H), 3.51 (dd, 1H), 3.60 (d, 1H), 3.63 (d, 1H), 3.88 (d, 1H), 4.05 (d, 1H), 5.45 (dd, 1H, H5), 6.63-6.70 (m, 1H, H 14), 8.03 (s, 1H, triazole). | | |
| Mass spectrum: (ESI) *m*/*z* = 696.64 (M+H). | | |
| 48 | R^{I} = n-Pr | (1*S*,4a*R*,6a*S*,7*R*,8*R*,10a*R*,10b*R*,12a*R*,14*R*,15*R*)-14-[5-(aminocarbonyl)-*1H*-1,2,4-triazol-1-yl]-8-[(1*R*)-1,2-dimethylpropyl]-15-[[(2*R*)-2,3-dimethyl-2-(propylamino)butyl]oxy]-1,6,6a,7,8,9,10,10,10b,1,12,12a-dodecahydro-1,6a,8,10a-tetramethyl-*4H*-1,4a-propano-2*H-*phenanthro[1,2-c]pyran-7-carboxylic acid |
| | R^{II} = H | |
| | R^{III} = H | |
| | R^{IV} = H | |
| ¹H NMR (CD₃OD, 500MHz, ppm) δ 0.74 (s, 3H, Me), 0.78 (s, 3H, Me), 0.79 (d, 6H, 2Me), 0.87 (d, 3H, Me), 0.90 (d, 3H, Me), 0.91 (s, 3H, Me), 0.92 (d, 3H, Me), 1.02 (t, 3H), 1.15 (s, 3H, Me), 1.22 (s, 3H, Me), 1.24-1.37 (m), 1.41-1.46 (m), 1.49-1.57 (m) 1.60-1.73 (m), 1.78-1.98 (m), 2.00-2.08 (m), 2.11-2.24 (m), 2.39 (dd, 1H, H13), 2.70-2.81 (m, 2H, NCH₂), 2.86 (s, 1H, H7), 3.00 (d, 1H), 3.50 (d, 1H), 3.54 (dd, 1H), 3.63 (d, 1H), 3.69 (d, 1H), 3.88 (d, 1H), 4.08 (d, 1H), 5.47 (dd, 1H, H5), 6.66-6.74 (m, 1H, H 14), 8.06 (s, 1H, triazole). | | |
| Mass spectrum: (ESI) *m*/*z* = 724.61 (M+H). | | |
| | | |
| 49 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1,6a,8,10a-tetramethyl-*4H*-1,4a-propano-2*H*-phenanthro[1,2-c]pyran-7-carboxylic acid |
| | R^{II} = H | |
| | R^{III} = Me | |
| | R^{IV} = H | |
| ¹H NMR (CD₃OD, 600MHz, ppm) δ 0.76 (d, 3H, Me), 0.77 (s, 3H, Me), 0.79 (d, 3H, Me), 0.84 (d, 3H, Me), 0.85 (d, 3H, Me), 0.87 (s, 3H, Me), 0.89 (d, 3H, Me), 1.13 (s, 3H, Me), 1.20 (s, 3H, Me), 1.22-1.34 (m), 1.40-1.44 (m), 1.48-1.55 (m), 1.58-1.65 (m), 1.74-1.95 (m), 2.10-2.21 (m), 2.38 (dd, 1H, H13), 2.80 (d, 1H), 2.83 (s, 1H, H7), 2.90 (s, 3H, NMe), 3.46 (d, 1H), 3.51 (d, 1H), 3.52 (dd, 1H), 3.61 (d, 1H), 3.93 (d, 1H), 3.94 (d, 1H), 5.45 (dd, 1H, H5), 6.68 (m, 1H, H14), 7.99 (s, 1H, triazole). | | |
| Mass spectrum: (ESI) *m*/*z* = 696.33 (M+H). | | |
| | | |
| 50 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-14-[5-[(dimethylamino)carbonyl]-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-2*H-*phenanthro[1,2-c]pyran-7-carboxylic acid |
| | R^{II} = H | |
| | R^{III} = Me | |
| | R^{IV} = Me | |
| ¹H NMR (CD₃OD, 600MHz, ppm) 0.76 (s, 3H, Me), 0.77 (d, 3H, Me), 0.79 (s, 3H, Me), 0.85 (d, 3H, Me), 0.87 (d, 3H, Me), 0.88 (s, 3H, Me), 0.89 (d, 3H, Me), 0.92 (d, 3H, Me), 1.14 (s, 3H, Me), 1.20 (s, 3H, Me), 1.22-1.32 (m), 1.40-1.44 (m), 1.48-1.56 (m), 1.58-1.64 (m), 1.78-2.04 (m), 2.10-2.21 (m), 2.33 (dd, 1H, H13), 2.73 (d, 1H), 2.84 (s, 1H, H7), 2.98 (s, 3H, NMe), 3.16 (s, 3H, NMe), 3.46 (d, 1H), 3.51 (dd, 1H), 3.57 (d, 1H), 3.61 (d, 1H), 3.86 (d, 1H), 3.87 (d, 1H), 5.47 (dd, 1H, H5), 5.61 (m, 1H, H14), 8.10 (s, 1H, triazole). | | |
| Mass spectrum: (ESI) *m*/*z* = 710.48 (M+H). | | |
| | | |
| 51 | R^{I} = Me | (1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = Me | |
| | R^{IV} = H | |
| ¹H NMR (CD₃OD, 500MHz, ppm) δ 0.74 (s, 3H, Me), 0.78 (s, 3H, Me), 0.79 d, 3H, Me), 0.84 (d, 3H, Me), 0.87 (d, 3H, Me), 0.88 (d, 3H, Me), 0.92 (d, 3H, Me), 0.92 (s, 3H, Me), 1.14 (s, 3H, Me), 1.22 (s, 3H, Me), 1.23-1.38 (m), 1.41-1.47 (m), 1.49-1.57 (m), 1.60-1.68 (m), 1.77-1.98 (m), 1.99-2.07 (m), 2.11-2.24 (m), 2.40 (dd, 1H, H13), 2.41 (s, 3H, NMe), 2.86 (s, 1H, H7), 2.93 (d, 3H, CONMe), 2.98 (d, 1H), 3.50 (d, 1H), 3.54 (dd, 1H), 3.64 (d, 1H), 3.66 (d, 1H), 3.91 (d, 1H), 4.07 (d, 1H), 5.47 (dd, 1H, H5), 6.66-6.73 (m, 1H, H14), 8.04 (s, 1H, triazole), 8.80 (q, 1H, CONH). | | |
| Mass spectrum: (ESI) *m*/*z* = 710.32 (M+H). | | |
| | | |
| 52 | R^{I} = Me | (1*S*,4a*R*,6a*S*,7*R*,8*R*,10a*R*,10b*R*,12a*R*,14R,15*R*)-15-[[(2*R*)-2-(dimethylamino)-2,3-dimethylbutyl]oxy]-8-[(1*R*)-1,2-dimethylpropyl]-14-[5-[(methylamino)carbonyl]-*1H-*1,2,4-triazol-l-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = Me | |
| | R^{III} = Me | |
| | R^{IV} = H | |
| ¹H NMR (CD₃OD, 600MHz, ppm) δ 0.75 (s, 3H, Me), 0.76 (d, 3H, Me), 0.85 (s, 3H, Me), 0.85 (d, 3H, Me), 0.89 (d, 3H, Me), 0.92 (d, 3H, Me), 0.92 (s, 3H, Me), 0.94 (d, 3H, Me), 1.11 (s, 3H, Me), 1.19 (s, 3H, Me), 1.21-1.36 (m), 1.39-1.43 (m), 1.46-1.54 (m), 1.58-1.65 (m), 1.70-1.77 (m), 1.77-1.94 (m), 2.08-2.13 (m), 2.14-2.21 (m), 2.23-2.30 (m), 2.37 (dd, 1H, H13), 2.64 (s, 3H, NMe), 2.70 (s, 3H, NMe), 2.83 (s, 1H, H7), 2.92 (d, 3H, CONMe), 3.13 (d, 1H), 3.50 (d, 1H), 3.50 (dd, 1H), 3.61 (d, 1H), 3.69 (d, 1H), 3.76 (d, 1H), 4.06 (d, 1H), 5.43 (dd, 1H, H5), 6.64-6.71 (m, 1H, H14), 8.03 (s, 1H, triazole), 8.77 (q, 1H, CONH). | | |
| Mass spectrum: (ESI) *m*/*z* = 724.47 (M+H). | | |
| | | |
| 53 | R^{I} = n-Pr | (1*S*,4a*R*,6a*S*,7*R*,8*R*,10a*R*,10b*R*,12a*R*,14*R*,15*R*)-8-[(1*R*)-1,2-dimethylpropyl]-15-[[(2*R*)-2,3-dimethyl-2-(propylamino)butyl]oxy]-14-[5-[(methylamino)carbonyl]-1*H*-1,2,4-triazol-1-yl]-1,6,6a,7,8,9,10,10a,10b,11,12,12a-dodecahydro-1,6a,8,10a-tetramethyl-*4H-*1,4a-propano-2*H-*phenanthro[1,2-c]pyran-7-carboxylic acid |
| | R^{II} = H | |
| | R^{III} = Me | |
| | R^{IV} = H | |
| ¹H NMR (CD₃OD, 600MHz, ppm) δ 0.70 (s, 3H, Me), 0.76 (d, 3H, Me), 0.76 (s, 3H, Me), 0.76 (d, 3H, Me), 0.85 (d, 3H, Me), 0.87 (d, 3H, Me), 0.88 (s, 3H, Me), 0.89 (d, 3H, Me), 0.99 (t, 3H), 1.12 (s, 3H, Me), 1.20 (s, 3H, Me), 1.21-1.35 (m), 1.39-1.44 (m), 1.47-1.54 (m), 1.58-1.69 (m), 1.75-1.95 (m), 1.98-2.05 (m), 2.08-2.03 (m), 2.14-2.22 (m), 2.36 (dd, 1H, H13), 2.67-2.77 (m, 2H, NCH₂), 2.83 (s, 1H, H7), 2.91 (d, 3H, CONMe), 2.96 (d, 1H), 3.48 (d, 1H), 3.51 (dd, 1H), 3.61 (d, 1H), 3.66 (d, 1H), 3.87 (d, 1H), 4.04 (d, 1H), 5.44 (dd, 1H, H5), 6.66-6.73 (m, 1H, H 14), 8.01 (s, 1H, triazole), 8.79 (q, 1H, CONH). | | |
| Mass spectrum: (ESI) *m*/*z* = 738.50 (M+H). | | |
| | | |
| 54 | R^{I} = Me | (1*S*,4a*R*,6a*S*,7*R*,8*R*,10a*R*,10b*R*,12a*R*,14*R*,15*R*)-14-[5-[(dimethylamino)carbonyl]-*1H-*1,2,4-triazol-1-yl]-15-[[(2*R*)-2,3-dimethyl-2-(methylamino)butyl]oxy]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = Me | |
| | R^{IV} = Me | |
| ¹H NMR (CD₃OD, 600MHz, ppm) δ 0.68 (s, 3H, Me), 0.75 (s, 3H, Me), 0.76 (d, 3H, Me), 0.85 (d, 3H, Me), 0.89 (d, 3H, Me), 0.90 (d, 3H, Me), 0.90 (d, 3H, Me), 0.92 (s, 3H, Me), 1.14 (s, 3H, Me), 1.20 (s, 3H, Me), 1.22-1.35 (m), 1.39-1.44 (m), 1.47-1.65 (m), 1.58-1.65 (m), 1.79-1.96 (m), 2.03-2.10 (m), 2.09-2.14 (m), 2.15-2.21 (m), 2.31 (dd, 1H, H13), 2.33 (s, 3H, NMe), 2.83 (s, 1H, H7), 3.11 (s, 3H, CONMe), 3.16 (s, 3H, CONMe), 3.17 (d, 1H), 3.48 (d, 1H), 3.50 (dd, 1H), 3.56 (d, 1H), 3.77 (d, 1H), 3.82 (d, 1H), 3.96 (d, 1H), 5.46 (dd, 1H, H5), 5.64-5.70 (m, 1H, H14), 8.10 (s, 1H, triazole). | | |
| Mass spectrum: (ESI) *m*/*z* = 724.55 (M+H). | | |
| | | |
| 55 | R^{I} = Et | (1*S*,4a*R*,6a*S*,7*R*,8*R*,10a*R*,10b*R*,12a*R*,14*R*,15*R*)-14-[5-[(dimethylamino)carbonyl]-*1H*-1,2,4-triazol-1-yl]-8-[(1*R*)-1,2-dimethylpropyl]-15-[[(2*R*)-2-(ethylamino)-2,3-dimethylbutyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = H | |
| | R^{III} = Me | |
| | R^{IV} = Me | |
| ¹H NMR (CD₃OD, 600MHz, ppm) δ 0.54 (s, 3H, Me), 0.76 (s, 3H, Me), 0.77 (d, 3H, Me), 0.81 (d, 3H, Me), 0.85 (d, 3H, Me), 0.88 (d, 3H, Me), 0.89 (d, 3H, Me), 0.91 (s, 3H, Me), 1.14 (s, 3H, Me), 1.20 (s, 3H, Me), 1.22-1.36 (m), 1.34 (t, 3H), 1.39-1.44 (m), 1.47-1.56 (m), 1.58-1.65 (m), 1.79-2.05 (m), 2.10-2.22 (m), 2.30 (dd, 1H, H 13), 2.82-2.89 (m, 1H, NCH), 2.83 (s, 1H, H7), 2.91-2.97 (m, 1H, NCH), 3.12 (s, 3H, CONMe), 3.16 (s, 3H, CONMe), 3.20 (d, 1H), 3.50 (d, 1H), 3.51 (d, 1H), 3.56 (d, 1H), 3.79 (d, 1H), 3.84 (d, 1H), 3.96 (d, 1H), 5.47 (dd, 1H, H5), 5.69-5.75 (m, 1H, H14), 8.09 (s, 1H, triazole). | | |
| Mass spectrum: (ESI) *m*/*z* = 738.65 (M+H). | | |
| | | |
| 56 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-[(ethylamino)carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = Et | |
| | R^{IV} = H | |
| ¹H NMR (CD₃OD, 600MHz, ppm) δ 0.76 (s, 3H, Me), 0.77 (d, 3H, Me), 0.78 (d, 3H, Me), 0.79 (s, 3H, Me), 0.84 (d, 3H, Me), 0.85 (d, 3H, Me), 0.88 (d, 3H, Me), 0.89 (s, 3H, Me), 1.14 (s, 3H, Me), 1.20 (s, 3H, Me), 1.21 (t, 3H), 1.22-1.34 (m), 1.40-1.44 (m), 1.48-1.55 (m), 1.58-1.65 (m), 1.74-1.95 (m), 2.10-2.21 (m), 2.39 (dd, 1H, H13.), 2.77 (d, 1H), 2.84 (s, 1H, H7), 3.34-3.45 (m), 3.46 (d, 1H), 3.50 (d, 1H), 3.52 (dd, 1H), 3.61 (d, 1H), 3.92 (d, 1H), 3.93 (d, 1H), 5.46 (dd, 1H, H5), 6.67 (m, 1H, H 14), 8.00 (s, 1H, triazole). | | |
| Mass spectrum: (ESI) *m*/*z* = 710.48 (M+H). | | |
| | | |
| 57 | R^{I} = Me | (1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-[(ethylamino)carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-2*H-*phenanthro[1,2-c]pyran-7-carboxylic acid |
| | R^{II} = H | |
| | R^{III} = Et | |
| | R^{IV} = H | |
| ¹H NMR (CD₃OD, 600MHz, ppm) δ 0.73 (s, 3H, Me), 0.76 (s, 3H, Me), 0.76 (d, 3H, Me), 0.81 (d, 3H, Me), 0.85 (d, 6H, 2Me), 0.89 (d, 3H, Me), 0.89 (s, 3H, Me), 1.12 (s, 3H, Me), 1.20 (s, 3H, Me), 1.21 (t, 3H), 1.21-1.35 (m), 1.39-1.44 (m), 1.47-1.54 (m), 1.58-1.65 (m), 1.77-1.95 (m), 1.97-2.04 (m), 2.09-2.14 (m), 2.15-2.22 (m), 2.38 (dd, 1H, H13), 2.40 (s, 3H, NMe), 2.83 (s, 1H, H7), 2.92 (d, 1H), 3.35-3.44 (m, 2H, CONCH₂), 3.47 (d, 1H), 3.51 (dd, 1H), 3.61 (d, 1H), 3.63 (d, 1H), 3.89 (d, 1 H), 4.03 (d, 1H), 5.44 (dd, 1H, H5), 6.63-6.70 (m, 1H, H 14), 8.02 (s, 1H, triazole), 8.83 (t, 1H, CONH). | | |
| Mass spectrum: (ESI) *mlz* = 724.84 (M+H). | | |
| | | |
| 58 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-14-[5-[(diethylamino)carbonyl]-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = Et | |
| | R^{IV} = Et | |
| ¹H NMR (CD₃OD, 500MHz, ppm) δ 0.78 (s, 3H, Me), 0.79 (d, 3H, Me), 0.82 (s, 3H, Me), 0.87 (d, 3H, Me), 0.90 (s, 3H, Me), 0.91 (d, 3H, Me), 0.92 (d, 3H, Me), 0.95 (d, 3H, Me), 1.17 (s, 3H, Me), 1.22-1.37 (m), 1.23 (s, 3H, Me), 1.25 (t, 3H), 1.29 (t, 3H), 1.41-1.47 (m), 1.49-1.68 (m), 1.80-1.99 (m), 2.01-2.10 (m), 2.12-2.24 (m), 2.27 (dd, 1H, H13), 2.74 (d, 1H), 2.86 (s, 1H, H7), 3.20-3.28 (m, 1H, CONCH₂), 3.42-3.59 (m, 2H, CONCH₂), 3.48 (d, 1H), 3.54 (s, 2H), 3.66 (d, 1H), 3.69-3.77 (m, 1H, CONCH₂), 3.85 (d, 1H), 3.87 (d, 1H), 5.45 (dd, 1H, H5), 5.55-5.63 (m, 1H, H14), 8.11 (s, 1H, triazole). | | |
| Mass spectrum: (ESI) *m*/*z* = 738.79 (M+H). | | |
| | | |
| 59 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-[(propylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = n-Pr | |
| | R^{IV} = H | |
| ¹H NMR (CD₃OD, 600MHz, ppm) δ 0.76 (s, 3H, Me), 0.77 (d, 3H, Me), 0.78 (d, 3H, Me), 0.79 (s, 3H, Me), 0.83 (d, 3H, Me), 0.85 (d, 3H, Me), 0.87 (s, 3H, Me), 0.89 (d, 3H, Me), 0.96 (t, 3H), 1.13 (s, 3H, Me), 1.20 (s, 3H, Me), 1.22-1.34 (m), 1.40-1.44 (m), 1.48-1.55 (m), 1.58-1.65 (m), 1.74-1.95 (m), 2.10-2.21 (m), 2.38 (dd, 1H, H13), 2.78 (d, 1H), 2.83 (s, 1H, H7), 3.26-3.37 (m), 3.45 (d, 1H), 3.51 (d, 1H), 3.52 (dd, 1H), 3.60 (d, 1H), 3.92 (d, 1H), 3.93 (d, 1H), 5.45 (dd, 1H, H5), 6.66 (m, 1H, H14), 8.00 (s, 1H, triazole), 8.79 (t, 1H, NH). | | |
| Mass spectrum: (ESI) *m*/*z* = 724.48 (M+H). | | |
| | | |
| 60 | R^{I} = Me | (1*S*,4a*R*,6a*S*,7*R*,8*R*,10a*R*,10b*R*,12a*R*,14*R*,15*R*)-15-[[(2*R*)-2-(dimethylamino)-2,3-dimethylbutyl]oxy]-8-[(1*R*)-1,2-dimethylpropyl]-14-[5-[[(1-methylethyl)amino]carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = Me | |
| | R^{III} = i-Pr | |
| | R^{IV} = H | |
| ¹H NMR (CD₃OD, 500MHz, ppm) δ 0.77 (s, 3H, Me), 0.78 (d, 3H, Me), 0.87 (d, 3H, Me), 0.89 (s, 3H, Me), 0.91 (d, 3H, Me), 0.94 (s, 3H, Me), 0.94 (d, 3H, Me), 0.96 (d, 3H, Me), 1.14 (s, 3H, Me), 1.22 (s, 3H, Me), 1.23-1.37 (m), 1.27 (d, 3H), 1.29 (d, 3H), 1.40-1.46 (m), 1.48-1.56 (m), 1.59-1.67 (m), 1.75-1.97 (m), 2.10-2.15 (m), 2.16-2.24 (m), 2.25-2.33 (m), 2.39 (dd, 1H, H13), 2.67 (s, 3H, NMe), 2.74 (s, 3H, NMe), 2.85 (s, 1H, H7), 3.13 (d, 1H), 3.52 (d, 1H), 3.53 (dd, 1H), 3.62 (d, 1H), 3.71 (d, 1H), 3.78 (d, 1H), 4.06 (d, 1H), 4.15-4.24 (m, 1H, CONCH), 5.45 (dd, 1H, H5), 6.59-6.67 (m, 1H, H14), 8.07 (s, 1H, triazole), 8.58 (d, 1H, CONH). | | |
| Mass spectrum: (ESI) *m*/*z* = 752.66 (M+H). | | |
| | | |
| 61 | R^{I} = Et | (1*S*,4a*R*,6a*S*,7*R*,8*R*,10a*R*,10b*R*,12a*R*,14*R*,15*R*)-8-[(1*R*)-1,2-dimethylpropyl]-15-[[(2*R*)-2-(ethylamino)-2,3-dimethylbutyl]oxy]-14-[5-[[(1-methylethyl)amino]carbonyl]-*1H*-1,2,4-triazol-1-yl]-1,6,6a,7,8,9,10,10a,10b,11,12,12a-dodecahydro-1,6a, 8,10a-tetramethyl-*4H-*1,4a-propano-*2H-*phenanthro[1,2-c]pyran-7-carboxylic acid |
| | R^{II} = H | |
| | R^{III} = i-Pr | |
| | R^{IV} = H | |
| ¹H NMR (CD₃OD, 600MHz, ppm) δ 0.73 (s, 3H, Me), 0.76 (s, 3H, Me), 0.76 (d, 3H, Me), 0.77 (d, 3H, Me), 0.85 (d, 3H, Me), 0.87 (d, 3H, Me), 0.88 (s, 3H, Me), 0.89 (d, 3H, Me), 1.12 (s, 3H, Me), 1.20 (s, 3H, Me), 1.22-1.37 (m), 1.24 (d, 3H, Me), 1.25 (t, 3H), 1.26 (d, 3H, Me), 1.39-1.44 (m), 1.47-1.55 (m), 1.58-1.65 (m), 1.76-1.95 (m), 1.99-2.06 (m), 2.08-2.13 (m), 2.14-2.22 (m), 2.38 (dd, 1H, H13), 2.65-2.72 (m, 1H, NCH₂), 2.80-2.87 (m, 1H, NCH₂), 2.83 (s, 1H, H7), 2.95 (d, 1H), 3.48 (d, 1H), 3.52 (dd, 1H), 3.61 (d, 1H), 3.66 (d, 1H), 3.88 (d, 1H), 4.04 (d, 1H), 4.14-4.21 (m, 1H, CONCH), 5.44 (dd, 1H, H5), 6.62-6.69 (m, 1H, H 14), 8.02 (s, 1H, triazole), 8.55 (d, 1H, CONH). | | |
| Mass spectrum: (ESI) *m*/*z* = 752.66 (M+H). | | |
| | | |
| 62 | R^{I} = n-Pr | (1*S*,4a*R*,6a*S*,7*R*,8*R*,10a*R*,10b*R*,12a*R,*14*R*,15*R*)-8-[(1*R*)-1,2-dimethylpropyl]-15-[[(2*R*)-2,3-dimethyl-2-(propylamino)butyl]oxy]-14-[5-[[(1-methylethyl)amino]carbonyl]-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = H | |
| | R^{III} = i-Pr | |
| | R^{IV} = H | |
| ¹H NMR (CD₃OD, 600MHz, ppm) δ 0.72 (s, 3H, Me), 0.75 (d, 3H, Me), 0.76 (s, 3H, Me), 0.76 (d, 3H, Me), 0.85 (d, 3H, Me), 0.86 (d, 3H, Me), 0.88 (s, 3H, Me), 0.89 (d, 3H, Me), 0.99 (t, 3H), 1.12 (s, 3H, Me), 1.20 (s, 3H, Me), 1.22-1.37 (m), 1.24 (d, 3H, Me), 1.26 (d, 3H, Me), 1.39-1.44 (m), 1.47-1.55 (m), 1.58-1.70 (m), 1.78-1.95 (m), 1.96-2.03 (m), 2.09-2.14 (m), 2.15-2.22 (m), 2.36 (dd, 1H, H13), 2.68-2.78 (m, 2H, NCH₂), 2.83 (s, 1H, H7), 2.94 (d, 1H), 3.48 (d, 1H), 3.52 (dd, 1H), 3.60 (d, 1H), 3.68 (d, 1H), 3.87 (d, 1H), 4.02 (d, 1H), 4.14-4.22 (m, 1H, CONCH), 5.44 (dd, 1H, H5), 6.61-6.68 (m, 1H, H14), 8.02 (s, 1H, triazole), 8.56 (d, 1H, CONH). | | |
| Mass spectrum: (ESI) *m*/*z* = 766.56 (M+H). | | |

### EXAMPLES 88-94

The following compounds were prepared using methods analogous to those described in the preceding examples:

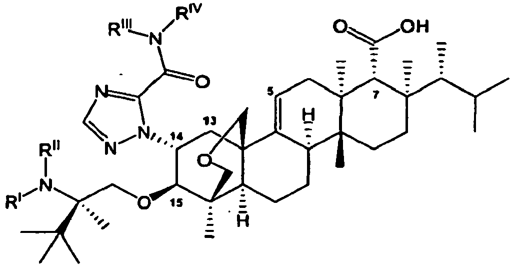

| | | |
|---|---|---|
| 88 | R^{I}=Me | (1*S*,4a*R*,6a*S*,7*R*,8*R*,10a*R*,10b*R*,12a*R*,14*R*,15*R*)-14-[5-(aminocarbonyl)-*1H*-1,2,4-triazol-1-yl]-8-[(1*R*)-1,2-dimethylpropyl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | R^{II} = H | |
| | R^{III} = H | |
| | R^{IV} = H | |
| ¹H NMR (CD₃OD, 500 MHz, ppm) δ 0.76 (s, 3H), 0.77 (d, J=7.1 Hz, 3H), 0.83 (s, 3H), 0.85 (d, J=6.6 Hz, 3H), 0.90 (d, J=6.9 Hz, 3H), 0.92 (s, 9H), 0.93 (s, 3H), 1.13 (s, 3H), 1.20 (s, 3H), 1.22 - 1.56 (m), 1.63 (m, 1H), 1.74 - 1.96 (m), 2.12 (m, 1H), 2.19 (m, 1H), 2.39 (dd, J=13.2 Hz, 6.4 Hz, 1H), 2.53 (s, 3H), 2.84 (s, 1H), 3.12 (d, J=11.2 Hz, 1H), 3.51 (d, J=11.7 Hz, 1H), 3.52 (dd, J=11.6 Hz, 1.9 Hz, 1H), 3.61 (d, J=11.4 Hz, 1H), 3.77 (d, J=11.9 Hz, 1H), 3.85 (d, J=11.0 Hz, 1H), 4.04 (d, J=10.1 Hz, 1h), 5.45 (m, 1H), 6.64 (m, 1H), 8.04 (s, 1H). | | |
| Mass spectrum: (ESI) *m*/*z* = 710.77 (M + H). | | |
| | | |
| 89 | R^{I} = H | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-[(methylamino)carbonyl]-*1H*-1,2,4-triazol-1-yl]-1,6,6a,7,8,9,10,10a,1b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = Me | |
| | R^{IV} = H | |
| ¹H NMR (CD₃OD, 500 MHz, ppm) δ 0.77 (s, 3H), 0.78 (s, 3H), 0.78 (d, 3H, partially obscured), 0.85 (s, 9H), 0.86 (d, 3H, partially obscured), 0.90 (s, 3H), 0.91 (d, 3H, partially obscured), 1.14 (s, 3H), 1.21 (s, 3H), 1.22 - 1.67 (m), 1.77 - 1.97 (m), 2.13 (m, 1H), 2.19 (m, 1H), 2.40 (dd, J=13.3 Hz, 6.4 Hz, 1H), 2.83 (d, 1H, partially obscured), 2.84 (s, 1H), 2.91 (d, J=4.3 Hz, 3H), 3.47 (d, J=11.9 Hz, 1H), 3.53 (dd, J=11.7 Hz, 1.8 Hz, 1H), 3.62 (d, J=11.7 Hz, 1H), 3.69 (d, J=9.8 Hz, 1H), 3.94 - 3.97 (m, 2H), 5.46 (m, 1H), 6.75 (m, 1H), 8.00 (s, 1H), 8.77 (m, 1H). | | |
| Mass spectrum: (ESI) *m*/*z* = 710.62 (M+H). | | |
| | | |
| 90 | R^{I} = H | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-14-[5-[(dimethylamino)carbonyl]-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | R^{II} = H | |
| | R^{III} = Me | |
| | R^{IV} = Me | |
| ¹H NMR (CD₃OD, 500 MHz, ppm) δ 0.71 (s, 3H), 0.77 (s, 3H), 0.78 (d, 3H, partially obscured), 0.86 (d, J=6.8 Hz, 3H), 0.90 (d, J=6.8 Hz, 3H), 0.92 (s, 9H), 0.92 (s, 3H), 1.15 (s, 3H), 1.21 (s, 3H), 1.20 - 1.57 (m), 1.63 (m, 1H), 1.80 - 1.97 (m), 2.13 (m, 1H), 2.19 (m, 1H), 2.37 (dd, J=13.4 Hz, 6.5 Hz, 1H), 2.84 (s, 1H), 2.98 (d, J=9.9 Hz, 1H), 3.14 (s, 3H), 3.16 (s, 3H), 3.48 (d, J=12.4 Hz, 1H), 3.52 (dd, J=11.8 Hz, 2.0 Hz, 1H), 3.58 (d, J=11.7 Hz, 1H), 3.85 (d, J=10.1 Hz, 1H), 3.90 (d, J=11.9 Hz, 1H), 3.98 (d, J=10.1 Hz, 1H), 5.48 (m, 1H), 5.79 (m, 1H), 8.09 (s, 1H). | | |
| Mass spectrum: (ESI) *m*/*z* = 724.69 (M+H). | | |
| | | |
| 91 | R^{I} = H | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-[(ethylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = Et | |
| | R^{IV} = H | |
| Mass spectrum: (ESI) *m*/*z* = 724.48 (M+H). | | |
| | | |
| 93 | R^{I} = H | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-[[(1-methylethyl)amino]carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | R^{III} = i-Pr | |
| | R^{IV} = H | |
| ¹H NMR (CD₃OD, 500 MHz, ppm) δ 0.77 (s, 3H) 0.77 (d, 3H, partially obscured), 0.80 (s, 3H), 0.85 (s, 9H), 0.86 (d, 3H, partially obscured), 0.89 (s, 3H), 0.90 (d, 3H, partially obscured), 1.14 (s, 3H), 1.21 (s, 3H), 1.24 (d, J=6.7 Hz, 3H), 1.27 (d, J=6.7 Hz, 3H), 1.23 - 1.63 (m), 1.78 -1.97 (m), 2.13 (m, 1H), 2.19 (m, 1H), 2.40 (dd, J=13.2 Hz, 6.4 Hz, 1H), 2.79 (d, J=9.8 Hz, 1H), 2.84 (s, 1H), 3.47 (d, J=11.9 Hz, 1H), 3.53 (dd, J=11.4 Hz, 1.8 Hz, 1H), 3.62 (d, J=11.5 Hz, 1H), 3.68 (d, J=9.9 Hz, 1H), 3.94 - 3.97 (m, 2H), 4.18 (m, 1H), 5.47 (m, 1H), 6.72 (m, 1H), 8.01 (s, 1H), 8.52 (d, 9.3 Hz, 1H) | | |
| Mass spectrum: (ESI) *m*/*z* = 738.67 (M+H). | | |
| 94 | R^{I} = Me | (1*S*,4a*R*,6a*S*,7*R*,8*R*,10a*R*,10b*R*,12a*R*,14*R*,15*R*)-8-[(1*R*)-1,2-dimethylpropyl]-14-[5-[[(1-methylethyl)amino]carbonyl]-*1H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = H | |
| | R^{III} = i-Pr | |
| | R^{IV} = H | |
| ¹H NMR (CD₃OD, 500 MHz, ppm) δ 0.76 (s, 3H), 0.77 (d, 3H, partially obscured), 0.83 (s, 3H), 0.86 (d, J=6.6 Hz, 3H), 0.90 (d, J=6.8 Hz, 3H), 0.92 (s, 9H), 0.93 (s, 3H), 1.13 (s, 3H), 1.20 (s, 3H), 1.22 - 1.56 (m), 1.25 (d, J=6.7 Hz, 3H), 1.27 (d, J=6.5 Hz, 3H), 1.63 (m, 1H), 1.77 - 1.96 (m), 2.12 (m, 1H), 2.19 (m, 1H), 2.39 (dd, J=13.3 Hz, 6.7 Hz, 1H), 2.51 (s, 3H), 2.84 (s, 1H), 3.08 (d, J=11.2 Hz, 1H), 3.50 (d, J=11.9 Hz, 1H), 3.52 (dd, J=11.4 Hz, 2.0 Hz, 1H), 3.61 (d, J=11.5 Hz, 1H), 3.78 (d, J=11.8 Hz, 1H), 3.84 (d, J=11.2 Hz, 1H), 4.00 (d, J=9.9 Hz, 1H), 4.18 (m, 1H), 5.45 (m, 1H), 6.57 (m, 1H), 8.03 (s, 1H), 8.58 (d, J=8.2 Hz, 1H). | | |
| Mass spectrum: (ESI) *m*/*z* = 752.82 (M+H). | | |

### EXAMPLES 100-102

The following compounds were prepared using methods analogous to those described in the preceding examples:

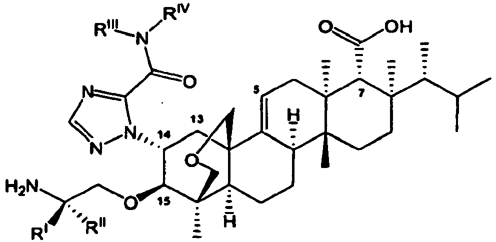

| | | |
|---|---|---|
| 100 | R^{I} = Et | (1*S*,4a*R*,6a*S*,7*R*,8*R*,10a*R*,10b*R*,12a*R*,14*R*,15*R*)-14-[5-(aminocarbonyl)-*1H-*1,2,4-triazol-1-yl]-15-(2-amino-2-ethylbutoxy)-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid |
| | R^{II} = Et | |
| | R^{III} = H | |
| | R^{IV} = H | |
| ¹H NMR (CD₃OD, 500 MHz, ppm) δ 0.64 (t, J=7.5 Hz, 3H), 0.76 (s, 3H), 0.77 (d, 3H, partially obscured), (t, J=7.8 Hz, 3H), 0.86 (d, J=6.8 Hz, 3H), 0.88 (s, 3H), 0.90 (d, J=6.6 Hz, 3H), 1.14 (s, 3H), 1.21 (s, 3H), 1.22 - 1.57 (m), 1.63 (m, 1H), 1.77 - 1.96 (m), 2.12 (m, 1H), 2.19 (m, 1H), 2.38 (dd, J=13.3 Hz, 6.6 Hz, 1H), 2.83 (d, 1H, partially obscured), 2.84 (s, 1H), 3.45 (d, 1H, partially obscured), 3.46 (d, 1H, partially obscured), 3.51 (dd, J=11.4 Hz, 1.8 Hz, 1H), 3.60 (d, J=11.7 Hz, 1H), 3.91 (d, J=11.9 Hz, 1H), 3.94 (d, J=9.9 Hz, 1H), 5.45 (m, 1H), 6.65 (m, 1H), 8.02 (s, 1H). | | |
| Mass spectrum: (ESI) *m*/*z* = 682.59 (M+H). | | |
| | | |
| 101 | R^{I} = Et | (1*S*,4a*R*,6a*S*,7*R*,8*R*,10a*R*,10b*R*,12a*R*,14*R*,15*R*)-15-(2-amino-2-ethylbutoxy)-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = Et | |
| | R^{III} = Me | |
| | R^{IV} = H | |
| ¹H NMR (CD₃OD, 500 MHz, ppm) δ 0.62 (t, J=7.5 Hz, 3H), 0.76 (s, 3H), 0.77 (d, 3H, partially obscured), 0.78 (t, 3H, partially obscured), 0.86 (d, J=6.9 Hz, 3H), 0.88 (s, 3H), 0.90 (d, 3H), 1.14 (s, 3H), 1.19 -1.56 (m), 1.14 (s, 3H), 1.20 (s, 3H), 1.63 (m, 1H), 1.77 - 1.96 (m), 2.13 (m, 1H), 2.19 (m), 2.37 (dd, J=13.2 Hz, 6.6 Hz, 1H), 2.82 (d, J=9.9 Hz, 1H), 2.84 (s, 1H), 2.92 (d, J=4.3 Hz, 3H), 3.44 - 3.47 (m, 2H), 3.52 (dd, J=11.5 Hz, 1.7 Hz, 1H), 3.61 (d, J=11.5 Hz, 1H), 3.90 - 3.96 (m, 2H), 5.45 (m, 1H), 6.65 (m, 1H), 8.00 (s, 1H), 8.78 (m, 1H) | | |
| Mass spectrum: (ESI) *m*/*z* = 696.53 (M+H). | | |
| | | |
| 102 | R^{I} = Et | (1*S*,4a*R*,6a*S*,7*R*,8*R*,10a*R*,10b*R*,12a*R*,14*R*,15*R*)-15-(2-amino-2-ethylbutoxy)-8-[(1*R*)-1,2-dimethylpropyl]-14-[5-[[(1-methylethyl)amino]carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = Et | |
| | R^{III} = i-Pr | |
| | R^{IV} = H | |
| ¹H NMR (CD₃OD, 500 MHz, ppm) δ 0.64 (t, J=7.5 Hz, 3H), 0.77 (s, 3H), 0.78 (d, 3H, partially obscured), 0.78 (t, J=7.5 Hz, 3H), 0.86 (d, J=6.6 Hz, 3H), 0.88 (s, 3H), 0.90 (d, J=6.9 Hz, 3H), 1.14 (s, 3H), 1.19 - 1.46 (m), 1.21 (s, 3H), 1.25 (d, J=7.1 Hz, 3H), 1.27 (d, J=7.1 Hz, 3H), 1.63 (m, 1H), 1.76 - 1.97 (m), 2.13 (m, 1H), 2.19 (m, 1H), 2.37 (dd, J=13.3, 6.7 Hz, 1H), 2.79 (d, J=9.8 Hz, 1H), 2.84 (s, 1H), 3.43 - 3.48 (m), 3.52 (dd, J=11.5 Hz, 2.0 Hz, 1H), 3.60 (d, J=11.4 Hz, 1H), 3.89 - 3.95 (m, 2H), 4.18 (m, 1H), 5.45 (m, 1H), 6.59 (m, 1H), 8.02 (s, 1H), 8.56 (d, J=8.2 Hz, 1H). | | |
| Mass spectrum: (ESI) *m*/*z* = 724.58 (M+H). | | |

### EXAMPLES 111-115

The following compounds were prepared using methods analogous to those described in the preceding examples:

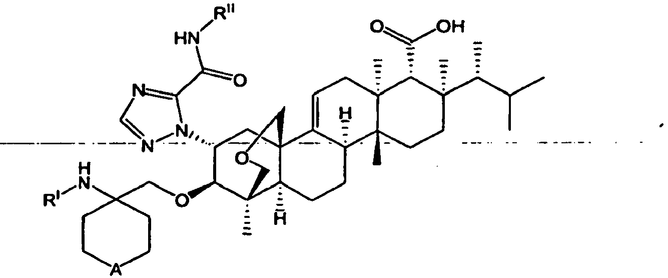

| | | |
|---|---|---|
| 111 | R^{I} = H | (1*S*,4a*R*,6a*S*,7*R*,8*R*,10a*R*,10b*R*,12a*R*,14*R*,15*R*)-14-[5-(aminocarbonyl)-*1H-*1,2,4-triazol-1-yl]-15-[(4-aminotetrahydro-2*H-*pyran-4-yl)methoxy]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = H | |
| | A=O | |
| ¹H NMR (CD₃OD, 500 MHz, ppm) δ 0.77 (s, 3H), 0.78 (d, 3H, partially obscured), 0.86 (d, J=6.6 Hz, 3H), 0.89 (s, 3H), 0.91 (d, 3H, partially obscured), 1.15 (s, 3H), 1.22 (s, 3H), 1.20-1.36 (m), 1.40 -1.57 (m), 1.58 - 1.72 (m), 1.78 -1.98 (m), 2.13 (m, 1H), 2.20 (m, 1H), 2.38 (dd, J=13.5, 6.6 Hz, 1H), 2.85 (s, 1H), 2.90 (m, 1H), 3.21 (d, J=10.0 Hz, 1H), 3.31 (partially obscured), 3.47 (d, J=12.2, 1H), 3.50-3.63 (m, 4H), 3.72 (m, 1H), 3.93 (d, J=11.9 Hz, 1H), 4.01 (d, J=10.1 Hz, 1H), 5.46 (m, 1H), 6.67 (m, 1H), 8.08 (s, 1H). | | |
| Mass spectrum: (ESI) *m*/*z* = 696.61 (M+H). | | |
| 112 | R^{I} = H | (1*S*,4a*R*,6a*S*,7*R*,8*R*,10a*R*,10b*R*,12a*R*,14*R*,15*R*)-15-[(4-aminotetrahydro-*2H*-pyran-4-yl)methoxy]-8-[(1*R*)-1,2-dimethylpropyl]-14-[5-[(methylamino)cubonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = Me | |
| | A=O | |
| ¹H NMR (CD₃OD, 500 MHz, ppm) δ 0.78 (s, 3H), 0.79 (d, 3H, partially obscured), 0.87 (d, J=6.6 Hz, 3H), 0.90 (s, 3H), 0.91 (d, 3H, partially obscured), 1.15 (s, 3H), 1.17 (s, 3H), 1.20-1.72 (m), 1.80 - 1.98 (m), 2.14 (m, 1H), 2.20 (m, 1H), 2.37 (dd, J=13.3, 6.4 Hz, 1H), 2.85 (s, 1H), 2.86 (m, partially obscured, 1H), 2.90 (s, 3H), 3.22(d, J=10.1 Hz, 1H),3.30 (partially obscured), 3.47 (d, J=12.1, 1H), 3.50-3.63 (m, 4H), 3.72 (m, 1H), 3.93 (d, J=11.9 Hz, 1H), 4.01 (d, J=10.1 Hz, 1H), 5.46 (m, 1H), 6.67 (m, 1H), 8.06 (s, 1H). | | |
| Mass spectrum: (ESI) *m*/*z* = 710.75 (M+H). | | |
| | | |
| 113 | R^{I} = Me | (1*S*,4a*R*,6a*S*,7*R*,8*R*,10a*R*,10b*R*,12a*R*,14*R*,15*R*)-8-[(1*R*)-1,2-dimethylpropyl]-14-[5-[(methylamino)carbonyl]-*1H*-1,2,4-triazol-1-yl]-15-[[tetrahydro-4-(methylamino)-*1H-*pyran-4-yl]methoxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | R^{II} = Me | |
| | A=O | |
| ¹H NMR (CD₃OD, 500 MHz, ppm) δ 0.77 (s, 3H), 0.78 (d, 3H, partially obscured), 0.87 (d, J=6.6 Hz, 3H), 0.90 (d, 3H, partially obscured), 0.92 (s, 3H), 1.14 (s, 3H), 1.22 (s, 3H), 1.23 - 1.72 (m), 1.77 - 1.98 (m), 2.13 (m, 1H), 2.20 (m, 1H), 2.38 (dd, partially obscured, 1H), 2.39 (s, 3H), 2.85 (s, 1H),2.90 (m, partially obscured, 1H), 2.93 (s, 3H), 3.32-3.39 (m, 2H, partially obscured), 3.50 (d, 1H, partially obscured), 3.53 (dd, 1H, partially obscured), 3.60 (m, 1H, partially obscured), 3.62 (d, 1H, partially obscured), 3.80 (m, 1H, partially obscured), 3.85(m, 2H, partially obscured), 4.15 (d, J=10.0 Hz, 1H), 5.46 (m, 1H), 6.65 (m, 1H), 8.07 (s, 1H). | | |
| Mass spectrum: (ESI) *m*/*z* = 724.69 (M+H). | | |
| 114 | R^{I} = H | (1*S,*4a*R*,6a*S,*7*R*,8*R*,10a*R*,10b*R*,12a*R*,14*R*,15*R*)*-*15-[(4-aminotetrahydro-2*H*-pyran-4-yl)methoxy]-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = i-Pr | |
| | A = O | |
| ¹H NMR (CD₃OD, 500 MHz, ppm) δ 0.78 (s, 3H), 0.79 (d, 3H, partially obscured), 0.87 (d, J=6.6 Hz, 3H), 0.89 (s, 3H), 0.91 (d, J=6.6, 3H, partially obscured), 1.16 (s, 3H), 1.22 (s, 3H), 1.25 (d, J=6.3 Hz, 3H), 1.27 (d, J=6.7 Hz, 3H), 1.16 - 1.70 (m), 1.80 -1.98 (m), 2.15 (m, 1H), 2.20 (m, 1H), 2.38 (dd, J = 13.4, 6.4 Hz, 1H), 2.85(s, 1H), 2.90(m, 1H), 3.18 (d, J=10.3, 1H, partially obscured), 3.22 (m, 1H, partially obscured), 3.48 (d, J = 11.7 Hz, 1H), 3.51-3.63 (m, 3H), 3.73(m, 1H), 3.93(d, J = 12.1 Hz, 1H), 3.99 (d, J=10.0 Hz, 1H), 4.18 (m, 1H), 5.46 (m, 1H), 6.62 (m, 1H), 8.07 (s, 1H). | | |
| Mass spectrum: (ESI) *m*/*z* = 738.78 (M+H). | | |
| | | |
| 115 | R^{I} = Me | (1*S*,4a*R*,6a*S*,7*R*,8*R*,10a*R*,10b*R*,12a*R*,14*R*,15*R*)-8-[(1*R*)-1,2-dimethylpropyl]-14-[5-[[(1-methylethyl)amino]carbonyl]-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = i-Pr | |
| | A=O | |
| ¹H NMR (CD₃OD, 500 MHz, ppm) 8 0.76 (s, 3H), 0.77 (d, 3H, partially obscured), 0.85 (d, J=6.8 Hz, 3H), 0.89 (d, 3H, partially obscured), 0.90 (s, 3H), 1.12 (s, 3H), 1.20 (s, 3H), 1.24 (d, J=6.6 Hz, 3H), 1.25 (d, J=6.5 Hz, 3H), 1.13 - 1.70 (m), 1.76 - 1.96 (m), 2.11 (m, 1H), 2.18 (m, 1H), 2.36 (dd, partially obscured, 1H), 2.39 (s, 3H), 2.83 (s, 1H), 2.90 (m, 1H), 3.32-3.39 (m, 2H, partially obscured), 3.48 (d, J = 11.8 Hz, 1H), 3.51 (dd, J = 11.7, 2.1 Hz 1H,), 3.59 (d, 1H, partially obscured), 3.60 (m, 1H, partially obscured), 3.78 (m, 1H, partially obscured), 3.81(d, 1H, partially obscured), 3.86(d, J = 12.0 Hz, 1H), 4.11 (d, J=9.7 Hz, 1H), 4.18 (m, 1H), 5.44 (m, 1 H), 6.58 (m, 1H), 8.05 (s, 1H). | | |
| Mass spectrum: (ESI) *m*/*z* = 752.80 (M+H). | | |

### EXAMPLE 173

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-[5-(4-pyridinyl)-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[11,2-c]pyran-7-carboxylic acid

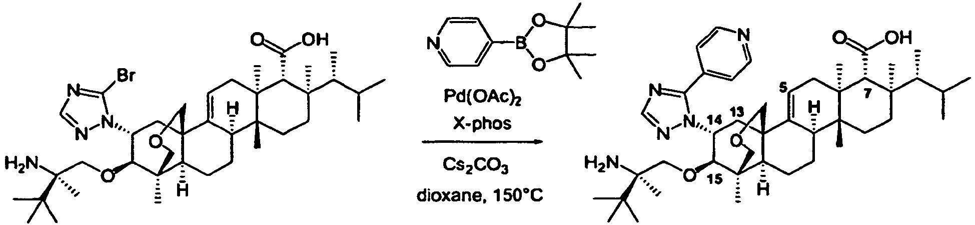

A suspension of (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-14-(5-bromo-*1H*-1,2,4-triazol-1-yl)-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid (Example 11, 37.2 mg, 0.044 mmol), cesium carbonate (255 mg, 0.78 mmol), 4-(4,4,5,5-tetramethyl-1,3,2-dioxaborolan-2-yl)pyridine (129 mg, 0.63 mmol), palladium (II) acetate (14.4 mg, 0.06 mmol) and 2-dicyclohexylphosphino-2',4',6'-triisopropylbiphenyl (24 mg, 0.05 mmol) in dioxane (1.2 mL) was blanketed with nitrogen and heated in a microwave at 150°C for 45 minutes. The mixture was cooled to room temperature, filtered and purified by reverse phase HPLC using a 19 x 150 mm Sunfire Preparative C18 OBD column eluting with acetonitrile/water + 0.1 % TFA. Product fractions were evaporated and freeze-dried from a mixture of ethanol and benzene to give the title compound (5.9 mg) as a white solid (trifluoroacetic acid salt).

¹H NMR (CD₃OD, 600MHz, ppm) 8 0.74 (s, 3H, Me), 0.76 (d, 3H, Me), 0.77 (s, 3H, Me), 0.85 (s, 9H, Me), 0.86 (d, 3H, Me), 0.87 (s, 3H, Me), 0.90 (d, 3H, Me), 1.17 (s, 3H, Me), 1.21 (s, 3H, Me), 1.23-1.34 (m), 1.41-1.45 (m), 1.48-1.65 (m), 1.82-1.98 (m), 2.06-2.11 (m), 2.13-2.21 (m), 2.62 (dd, 1H, H13), 2.85 (s, 1H, H7), 2.87 (d, 1 H), 3.46 (d, 1H), 3.56 (dd, 1H), 3.65 (d, 1H), 3.68 (d, 1H), 3.82 (d, 1H), 4.03 (d, 1H), 5.62 (dd, 1H, H5), 5.88 (m, 1H, H 14), 7.96 (br d, 2H, pyridyl H), 8.21 (s, 1H, triazole) and 8.90 (br d, 2H, pyridyl H).

Mass spectrum: (ESI) *m*/*z* = 730.71 (M+H).

### EXAMPLE 173

### (ALTERNATIVE SYNTHESIS)

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-[5-(4-pyridinyl)-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

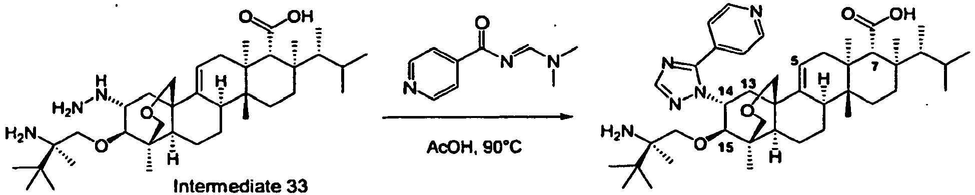

A suspension of Intermediate 33 (2.83 g, 0.046 mol) and N-[(1*E*)-(dimethylamino)methylidene]pyridine-4-carboxamide (0.93 g, 0.052 mol) in acetic acid (44.4 mL) was blanketed with nitrogen and heated in a 90°C oil bath for 1 hour. The mixture was cooled to room temperature, evaporated and purified by reverse phase HPLC using a 19 x 150 mm Sunfire Preparative C18 OBD column eluting with acetonitrile/water + 0.1% TFA. The product fractions were combined, concentrated under vacuum and freeze-dried to give the title compound (2.21 g) as a white solid (trifluoroacetic acid salt).

### CONVERSION OF EXAMPLE 173 TO HYDROCHLORIDE SALT

A portion of the trifluoroacetic acid salt from above (0.90 g, 1.07 mmol) was dissolved in methanol (9 mL) and the solution was diluted with 9 mL of 1:1 MeCN/H₂O. The solution was loaded onto a column of Dowex® 1X8 chloride form ion exchange resin (48 mL, ∼34 g, -1.8 meq/g) and the column was eluted with 1:1 MeCN/H₂O (120 mL). The eluant was concentrated *in vacuo* to remove most of the acetonitrile and then frozen and lyophilized to give 0.72 g of the hydrochloride salt as a white solid.

### CONVERSION OF EXAMPLE 173 TO FREE BASE

A portion of the trifluoroacetic acid salt from above (35.2 mg, 0.042 mmol) was dissolved in ethyl acetate (10 mL) and the solution was washed with sat. NaHCO₃ (3 mL) followed by brine (3 mL). The separated organic layer was dried over Na₂SO₄ and evaporated *in vacuo* to give a glassy solid. The solid was dissolved in benzene and a small amount of methanol and the solution was frozen and lyophilized to give 24.2 mg of the free base as a white solid.

¹H NMR (CD₃OD, 500MHz, ppm) δ 0.73 (s, 3H, Me), 0.86 (d, 3H, Me), 0.79 (s, 3H, Me), 0.81 (s, 9H, Me), 0.87 (d, 3H, Me), 0.89 (s, 3H, Me), 0.92 (d, 3H, Me), 1.23 (s, 3H, Me), 1.25 (s, 3H, Me), 1.29-1.38 (m), 1.38-1.45 (m), 1.53 (t, 1H), 1.59-1.67 (m), 1.81-1.94 (m), 2.01 (d, 1H), 2.11 (d, 1H), 2.13-2.21 (m), 2.23-2.31 (m, 1H), 2.58 (dd, 1H, H 13), 2.78 (s, 1H, H7), 2.79 (d, 1H), 3.44-3.49 (m, 2 H), 3.56 (d, 1 H), 3.67 (d, 1 H), 3.80 (d, 1 H), 3.94 (d, 1 H), 5.63 (d, 1 H, H5), 5.83-5.92 (m, 1 H, H14), 7.81 (d, 2 H, pyridyl H), 8.18 (s, 1 H, triazole), 8.79 (d, 2 H, pyridyl H).

Mass spectrum: (ESI) *m*/*z* = 730.70 (M+H).

### EXAMPLE 174

### (1S,4aR,6aS,7R,8R,10aR,10bR,12aR,14R,15R)-8-[(1R)-1,2-dimethylpropyl]-14-[5-(4-pyridinyl)-1H-1,2,4-triazol-1-yl]-15-[[(2R)-2,3,3-trimethyl-2-(methylamino)butyl]oxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

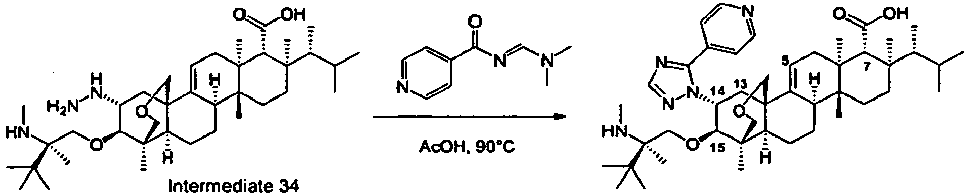

Intermediate 34 (166 mg, 0.22 mmol) was dissolved in acetic acid (7.5 mL) under nitrogen. To this solution was added N-[(1*E*)-(dimethylamino)methylidene]pyridine-4-carboxamide (47 mg, 0.27 mmol) and the resulting solution heated at 90°C. After two hours the reaction was allowed to cool to room temperature then concentrated *in vacuo.* The crude reaction mixture was dissolved in methanol then purified by HPLC (30x100 mm Waters Sunfire column, 5µm, UV detection, 30-100% MeCN/water with 0.05% TFA over 20 minutes). The product fractions were partially concentrated by rotovap then frozen and lyophilized overnight to provide the title compound (128 mg) as an amorphous white solid.

¹H NMR (CD₃OD, 500 MHz, ppm) δ 0.73 (s, 3H), 0.77 (d, J=6.9 Hz, 3H), 0.78 (s, 3H), 0.86 (d, J=6.6 Hz, 3H), 0.89 (s, 9H), 0.89 (d, 3H, partially obscured), 0.91 (s, 3H), 1.17 (s, 3H), 1.22 (s, 3H), 1.24 - 1.68 (m), 1.83 - 2.08 (m), 2.15 - 2.24 (m, 2H), 2.56 (s, 3H), 2.57 (dd, 1H, partially obscured), 2.85 (s, 1H), 3.15 (d, J=11.0 Hz, 1H), 3.50 (d, J=12.1 Hz, 1H), 3.56 (dd, J=11.7 Hz, 2.0 Hz, 1H), 3.64 (d, J=8.7 Hz, 1H), 3.67 (d, J=8.5 Hz, 1H), 3.84 (d, J=11.2 Hz, 1H), 4.07 (d, J=9.9 Hz, 1H), 5.61 (m, 1 H), 5.87 (m, 1H), 7.81 (m, 2H), 8.20 (s, 1H), 8.82 (m, 2H).

Mass Spectrum: (ESI) *m*/*z* = 744.32 (M+H).

### EXAMPLE 176

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-[5-(5-pyrimidinyl)-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

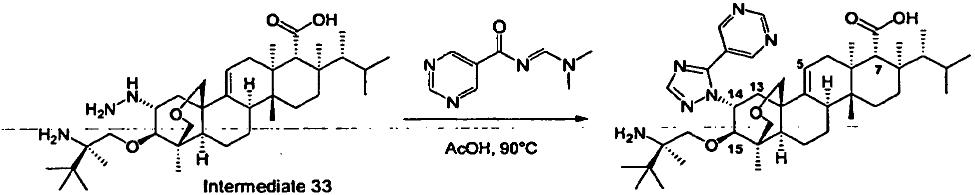

N-[(1*E*)-(dimethylamino)methylidene]pyrimidine-5-carboxamide (13.8 mg, 0.077 mmol) was added to a stirred solution of Intermediate 33 (50.7 mg, 0.069 mmol) in acetic acid (1.0 ml, 17.47 mmol). The reaction mixture was a light yellow solution that was degassed (2x) and placed under nitrogen before being heated to 90°C. After 30 minutes, LCMS and ¹H NMR showed complete conversion to product. The reaction mixture was cooled to room temperature, diluted with methanol, and evaporated under reduced pressure to give a light yellow residue. The residue was dissolved in methanol and purified using two HPLC runs (∼25 mg / run) on a 30 x 150 mm Sunfire Prep C18 OBD 10 µm column by eluting with acetonitrile/water + 0.1% TFA. The total flow rate was 20 ml/min and the HPLC method employed a 17 minute 20%-100% acetonitrile/water gradient followed by a 2 minute acetonitrile flush. The product fractions were combined, the solvent was evaporated under reduced pressure, and the residue was lyophilized from ethanol and benzene to give the title compound (29.0 mg) as a white solid.

¹H NMR (CD₃OD, 500 MHz, ppm) δ 0.78 (s, 3H, Me), 0.79 (d, 3H, Me), 0.80 (s, 3H, Me), 0.88 (d, 3H, Me), 0.88 (s, 9H, t-bu), 0.91 (s, 3H, Me), 0.92 (d, 3H, Me), 1.18 (s, 3H, Me), 1.22 (s, 3H, Me), 1.24-1.38 (m), 1.42-1.47 (m), 1.50-1.68 (m), 1.83-2.01 (m), 2.07-2.14 (m), 2.15-2.25 (m), 2.64 (dd, 1H, H 13), 2.87 (s, 1H, H7), 2.94 (d, 1H), 3.48 (d, 1H), 3.58 (dd, 1H), 3.66 (d, 1H), 3.70 (d, 1H), 3.85 (d, 1H), 4.05 (d, 1H), 5.62 (dd, 1H, H5), 5.79-5.86 (m, 1H, H 14), 8.24 (s, 1H, triazole), 9.21 (s, 2H, ArH), 9.36 (s, 1H, ArH).

Mass Spectrum: (ESI) *m*/*z* = 731.71 (M+H).

### EXAMPLE 181

### (1S,4aR,6aS,7R,8R,10aR,10bR,12aR,14R,15R)-8-[(1R)-1,2-dimethylpropyl]-14-[5-(4-pyridinyl)-1H-1,2,4-triazol-1-yl]-15-[[tetrahydro-4-(methylamino)-2H-pyran-4-yl]methoxy]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

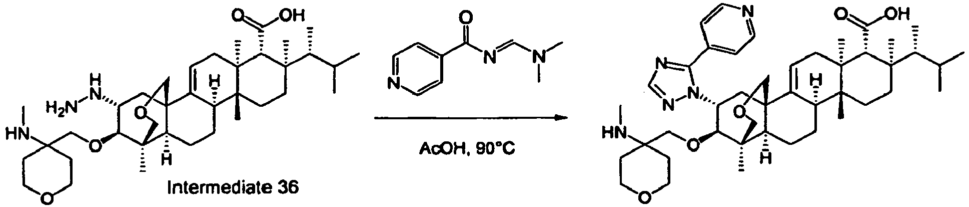

A solution of Intermediate 36 (710 mg, 0.95 mmol) in acetic acid (29 mL) was treated with N-[(1*E*)-(dimethylamino)methylidene]pyridinc4-carboxamide (186 mg, 1.05 mmol) and this mixture was heated to 90°C under nitrogen. After 0.5 hours the reaction was cooled to room temperature then concentrated *in vacuo.* The crude product mixture was suspended in methanol (3 mL) and filtered through a sintered glass funnel. The filtrate was purified by preparative HPLC (19x100 mm Waters Sunfire column, 5µm, UV-detection, 30-100% MeCN/water with 0.05% TFA over 20 minutes). The product fractions were combined and partially concentrated by rotovap then frozen and lyophilized to give the title compound (500 mg) as a white amorphous solid.

¹H NMR (CD₃OD, 500 MHz, ppm) δ 0.78 (s, 3H), 0.79 (d, 3H, partially obscured), 0.87 (d, J=6.6 Hz, 3H), 0.91 (d, 3H, partially obscured), 0.92 (s, 3H), 1.17 (s, 3H), 1.21 (s, 3H), 1.23 -1.78 (m), 1.84 -2.05 (m), 2.12 - 2.23 (m, 2H), 2.40 (s, 3H), 2.54 (dd, J=13.8, 6.2 Hz, 1H), 2.85 (s, 1H), 2.88 (m, 1H, partially obscured), 3.28-3.36 (m, 2H, partially obscured), 3.43 (d, J=11.2 Hz, 1H), 3.50 (d, J=12.1 Hz, 1H), 3.56 (dd, J=11.6, 1.6 Hz, 1H), 3.63 (m, 1H, partially obscured), 3.67 (d, 1H, partially obscured), 3.76 - 3.86 (m, 3H), 4.20 (d, J=9.6 Hz, 1H), 5.58 (m, 1H), 5.93 (m, 1H), 8.00 (br, 2H), 8.27(s, 1H), 8.92 (br, 2H).

Mass Spectrum: (ESI) *m*/*z* = 744.66 (M+H).

### EXAMPLES 182-200

The following compounds were prepared using methods analogous to those described in the preceding examples:

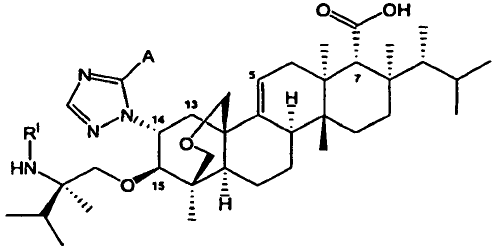

| | | |
|---|---|---|
| 182 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(3-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | A= | |
| | 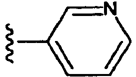 | |
| ¹H NMR (CD₃OD, 600 MHz, ppm) δ 0.77 (s, 3H, Me), 0.77 (d, 3H, Me), 0.78 (s, 3H, Me), 0.80 (d, 3H, Me), 0.83 (d, 3H, Me), 0.86 (d, 3H, Me), 0.86 (s, 3H, Me), 0.90 (d, 3H, Me), 1.17 (s, 3H, Me), 1.21 (s, 3H, Me), 1.22-1.35 (m), 1.40-1.45 (m), 1.49-1.66 (m), 1.68-1.75 (m), 1.81-1.98 (m), 2.05-2.11 (m), 2.13-2.22 (m), 2.55 (dd, 1H, H13), 2.85 (s, 1H, H7), 2.89 (d, 1H), 3.44 (d, 1H), 3.48 (d, 1H), 3.53 (dd, 1H), 3.61 (d, 1H), 3.80 (d, 1H), 4.00 (d, 1H), 5.59 (dd, 1H, H5), 5.80-5.86 (m, 1H, H14), 7.67 (dd, 1H, ArH), 8.17 (s, 1H, triazole), 8.22 (d, 1H, ArH), 8.74 (broad, 1H, ArH), 8.94 (broad, 1H, ArH). | | |
| Mass Spectrum: (ESI) *m*/*z* = 716.74 (M+H). | | |
| 183 | R^{I} = Me | (1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyyl]-14-[5-(3-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A = | |
| | 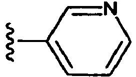 | |
| ¹H NMR (CD₃OD, 600 MHz, ppm) δ 0.74 (s, 3H, Me), 0.77 (s, 3H, Me), 0.77 (d, 3H, Me), 0.81 (d, 3H, Me), 0.83 (d, 3H, Me), 0.85 (d, 3H, Me), 0.88 (s, 3H, Me), 0.90 (d, 3H, Me), 1.17 (s, 3H, Me), 1.21 (s, 3H, Me), 1.23-1.34 (m), 1.40-1.45 (m), 1.49-1.66 (m), 1.81-1.98 (m), 2.02-2.08 (m), 2.13-2.22 (m), 2.37 (s, 3H, NMe), 2.58 (dd, 1H, H13), 2.85 (s, 1H, H7), 2.99 (d, 1H), 3.45 (d, 1H), 3.54 (dd, 1H), 3.63 (d, 1H), 3.64 (d, 1H), 3.76 (d, 1H), 4.08 (d, 1H), 5.60 (dd, 1H, H5), 5.79-5.85 (m, 1H, H14), 7.72 (broad, 1H, ArH), 8.19 (s, 1H, triazole), 8.27 (d, 1H, ArH), 8.79 (broad, 1H, ArH), 8.99 (broad, 1H, ArH). | | |
| Mass Spectrum: (ESI) *m*/*z* = 730.76 (M+H). | | |
| | | |
| 184 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | A= | |
| | 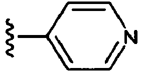 | |
| ¹H NMR (CD₃OD, 600 MHz, ppm) δ 0.76 (s, 3H, Me), 0.77 (d, 3H, Me), 0.77 (s, 3H, Me), 0.80 (d, 3H, Me), 0.82 (d, 3H, Me), 0.86 (d, 3H, Me), 0.86 (s, 3H, Me), 0.90 (d, 3H, Me), 1.17 (s, 3H, Me), 1.21 (s, 3H, Me), 1.23-1.35 (m), 1.41-1.45 (m), 1.49-1.66 (m), 1.68-1.75 (m), 1.81-1.98 (m), 2.05-2.11 (m), 2.13-2.22 (m), 2.57 (dd, 1H, H13), 2.85 (s, 1H, H7), 2.86 (d, 1H), 3.46 (d, 1H), 3.49 (d, 1H), 3.55 (dd, 1H), 3.66 (d, 1H), 3.82 (d, 1H), 4.00 (d, 1H), 5.60 (dd, 1H, H5), 5.86-5.92 (m, 1H, H 14), 7.88 (d, 2H, ArH), 8.20 (s, 1H, triazole), 8.83 (broad d, 2H, ArH). | | |
| Mass Spectrum: (ESI) *m*/*z* = 716.74 (M+H). | | |
| | | |
| 185 | R^{I} = Me | (1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 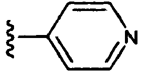 | |
| ¹H NMR (CD₃OD, 600MHz, ppm) δ 0.72 (s, 3H, Me), 0.77 (d, 3H, Me), 0.77 (s, 3H, Me), 0.81 (d, 3H, Me), 0.83 (d, 3H, Me), 0.86 (d, 3H, Me), 0.88 (s, 3H, Me), 0.90 (d, 3H, Me), 1.17 (s, 3H, Me), 1.21 (s, 3H, Me), 1.23-1.34 (m), 1.41-1.45 (m), 1.48-1.65 (m), 1.84-1.98 (m), 2.03-2.08 (m), 2.16-2.21 (m), 2.37 (s, 3H, NMe), 2.60 (dd, 1H, H13), 2.85 (s, 1H, H7), 2.95 (d, 1H), 3.47 (d, 1H), 3.56 (dd, 1H), 3.64 (d, 1H), 3.68 (d, 1H), 3.77 (d, 1H), 4.08 (d, 1H), 5.62 (dd, 1H, H5), 5.88 (m, 1H, H14), 7.90 (br d, 1H, pyridyl H), 8.21 (s, 1H, triazole) and 8.83 (br d, 1H, pyridyl H). | | |
| Mass spectrum: (ESI) *m*/*z* = 730.76 (M+H) | | |
| | | |
| 186 | R^{I} = Me | (1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-l,2-dimethylpropyl]-14-[5-(2-pyridinyl)-*1H*-1,2,4-triazol-1-yl]-1,6,6 a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 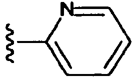 | |
| ¹H NMR (CD₃OD, 600MHz, ppm) δ 0.68 (s, 3H, Me), 0.76 (s, 3H, Me), 0.76 (d, 3H, Me), 0.85 (d, 3H, Me), 0.85 (s, 3H, Me), 0.86 (s, 3H, Me), 0.89 (s, 3H, Me), 0.90 (d, 3H, Me), 1.14 (s, 3H, Me), 1.21 (s, 3H, Me), 1.23-1.34(m), 4.41-1.45(m), 1.48-1.65 (m), 1.80-1.94 (m), 2.01 (m), 2.12-2.21 (m), 2.29 (s, 3H, NMe), 2.56 (dd, 1H, H 13), 2.83 (s, 1H, H7), 3.14 (d, 1H), 3.47 (d, 1H), 3.54 (dd, 1H), 3.60 (d, 1H), 3.65 (d, 1H), 3.82 (d, 1H), 4.16 (d, 1H), 5.44 (dd, 1H, H5), 6.72 (m, 1H, H14), 8.21 (s, 1H, triazole), 7.53 (br d, 1H, pyridyl H), 8.00(br d, 1H, pyridyl H ), 8.06 (br d, 1H, pyridyl H ), 8.12 (br d, 1H, pyridyl H) and 8.86 (br d, 1H, pyridyl H). | | |
| Mass spectrum: (ESI) *m*/*z* = 730.51 (M+H) | | |
| | | |
| 187 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-(5-pyrazinyl-*1H*-1,2,4-triazol-1-yl)-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | A= | |
| | 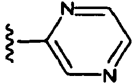 | |
| ¹H NMR (CD₃OD, 600 MHz, ppm) δ 0.67 (s, 3H, Me), 0.76 (d, 3H, Me), 0.77 (s, 3H, Me), 0.79 (d, 3H, Me), 0.80 (d, 3H, Me), 0.85 (d, 3H, Me), 0.88 (s, 3H, Me), 0.89 (d, 3H, Me), 1.14 (s, 3H, Me), 1.20 (s, 3H, Me), 1.22-1.37 (m), 1.40-1.45 (m), 1.48-1.55 (m), 1.58-1.65 (m), 1.69-1.76 (m), 1.81-1.95 (m), 2.12-2.22 (m), 2.58 (dd, 1H, H13), 2.83 (s, 1H, H7), 2.98 (d, 1H), 3.47 (d, 1H), 3.52 (d, 1H), 3.56 (dd, 1H), 3.64 (d, 1H), 3.86 (d, 1H), 4.08 (d, 1H), 5.46 (dd, 1H, H5), 6.68-6.74 (m, 1H, H14), 8.16 (s, 1H, triazole), 8.72 (d, 1H, ArH), 8.78 (dd, 1H, ArH), 9.28 (d, 1H, ArH). | | |
| Mass Spectrum: (ESI) *m*/*z* = 717.72 (M+H). | | |
| | | |
| 188 | R^{I} = H | (1*S*,4a*R*,6a*S*,7*R*,8*R*,10a*R*,10b*R*,12aR,14*R*,15*R*)-15-[[(2*R*)-2-amino-2,3-dimethylbutyl]oxy]-8-[(1*R*)-1,2-dimethylpropyl]-14-[5-(4-pyridazinyl)-1*H*-1,2,4-triazol-1-yl]-1,6,6a,7,8,9,10,10a10b,11,12,12a-dodecahydro-1,6a,8,10a-tetramethyl-*4H*-1,4a-propano-*2H*-phenanthro[1,2-c]pyran-7-carboxylic acid |
| | A= | |
| | 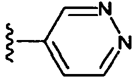 | |
| ¹H NMR (CD₃OD, 500 MHz, ppm) δ 0.74 (s, 3H, Me), 0.79 (d, 3H, Me), 0.80 (s, 3H, Me), 0.82 (d, 3H, Me), 0.85 (d, 3H, Me), 0.88 (d, 3H, Me), 0.90 (s, 3H, Me), 0.92 (d, 3H, Me), 1.19 (s, 3H, Me), 1.23 (s, 3H, Me), 1.24-1.38 (m), 1.43-1.48 (m), 1.50-1.69 (m), 1.70-1.79 (m), 1.83-2.01 (m), 2.07-2.14 (m), 2.16-2.25 (m), 2.64 (dd, 1H, H13), 2.87 (s, 1H, H7), 2.87 (d, 1H), 3.50 (d, 1H), 3.53 (d, 1H), 3.58 (dd, 1H), 3.70 (d, 1H), 3.85 (d, 1H), 4.04 (d, 1H), 5.62 (dd, 1H, H5), 5.85-5.92 (m, 1H, H 14), 8.11 (dd, 1H, ArH), 8.27 (s, 1H, triazole), 9.47 (d, 1H, ArH), 9.62 (broad d, 1 H, ArH). | | |
| Mass Spectrum: (ESI) *m*/*z* = 717.73 (M+H). | | |
| | | |
| 189 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(3-pyridazinyl)-*1H*-,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 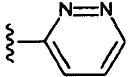 | |
| ¹H NMR (CD₃OD, 600 MHz, ppm) δ 0.68 (s, 3H, Me), 0.76 (s, 3H, Me), 0.77 (d, 3H, Me), 0.81 (d, 3H, Me), 0.83 (d, 3H, Me), 0.85 (d, 3H, Me), 0.89 (d, 3H, Me), 0.89 (s, 3H, Me), 1.14 (s, 3H, Me), 1.20 (s, 3H, Me), 1.22-1.35 (m), 1.40-1.44 (m), 1.48-1.54 (m), 1.58-1.65 (m), 1.75-1.95 (m), 2.12-2.22 (m), 2.58 (dd, 1H, H13), 2.83 (s, 1H, H7), 3.01 (d, 1H), 3.44 (d, 1H), 3.50 (dd, 1H), 3.57 (d, 1H), 3.63 (d, 1H), 3.87 (d, 1H), 4.12 (d, 1H), 5.46 (dd, 1H, H5), 6.55-6.61 (m, 1H, H14), 7.94 (dd, 1H, ArH), 8.19 (s, 1H, triazole), 8.30 (dd, 1H, ArH), 9.33 (dd, 1H, ArH). | | |
| Mass Spectrum: (ESI) *m*/*z* = 717.72 (M+H). | | |
| | | |
| 190 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyrimidinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 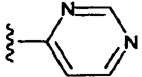 | |
| ¹H NMR (CD₃OD, 500 MHz, ppm) δ 0.67 (s, 3H, Me), 0.79 (d, 3H, Me), 0.80 (s, 3H, Me), 0.81 (d, 3H, Me), 0.82 (d, 3H, Me), 0.88 (d, 3H, Me), 0.91 (s, 3H, Me), 0.92 (d, 3H, Me), 1.17 (s, 3H, Me), 1.22 (s, 3H, Me), 1.24-1.39 (m), 1.42-1.48 (m), 1.50-1.58 (m), 1.61-1.69 (m), 1.70-1.78 (m), 1.83-1.98 (m), 2.14-2.25 (m), 2.62 (dd, 1H, H13), 2.86 (s, 1H, H7), 2.98 (d, 1H), 3.52 (d, 1H), 3.54 (d, 1H), 3.60 (dd, 1H), 3.70 (d, 1H), 3.92 (d, 1H), 4.11 (d, 1H), 5.50 (dd, 1H, H5), 6.94-7.01 (m, 1H, H 14), 8.17 (dd, 1H, ArH), 8.19 (s, 1H, triazole), 9.01 (d, 1H, ArH), 9.36 (d, 1H, ArH). | | |
| Mass Spectrum: (ESI) *m*/*z* = 717.74 (M+H). | | |
| | | |
| 191 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(5-pyrimidinyl)-1H-1,2,4-thazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 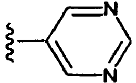 | |
| ¹H NMR (CD₃OD, 600 MHz, ppm) δ 0.77 (s, 6H, 2Me), 0.77 (d, 3H, Me), 0.81 (d, 3H, Me), 0.83 (d, 3H, Me), 0.86 (d, 3H, Me), 0.87 (s, 3H, Me), 0.90 (d, 3H, Me), 1.17 (s, 3H, Me), 1.21 (s, 3H, Me), 1.22-1.35 (m), 1.40-1.45 (m), 1.48-1.66 (m), 1.69-1.76 (m), 1.81-1.97 (m), 2.04-2.11 (m), 2.13-2.22 (m), 2.57 (dd, 1H, H13), 2.84 (s, 1H, H7), 2.88 (d, 1H), 3.46 (d, 1H), 3.50 (d, 1H), 3.54 (dd, 1H), 3.61 (d, 1H), 3.82 (d, 1H), 4.01 (d, 1H), 5.58 (dd, 1H, H5), 5.77-5.83 (m, 1H, H14), 8.21 (s, 1H, triazole), 9.19 (s, 2H, ArH), 9.34 (s, 1H, ArH). | | |
| Mass Spectrum: (ESI) *m*/*z* = 717.79 (M+H). | | |
| | | |
| 192 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(2-pyrimidinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A = | |
| | 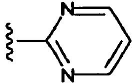 | |
| ¹H NMR (CD₃OD, 600 MHz, ppm) δ 0.68 (s, 3H, Me), 0.76 (d, 3H, Me), 0.77 (s, 3H, Me), 0.78 (d, 3H, Me), 0.81 (d, 3H, Me), 0.85 (d, 3H, Me), 0.87 (s, 3H, Me), 0.89 (d, 3H, Me), 1.15 (s, 3H, Me), 1.20 (s, 3H, Me), 1.22-1.36 (m), 1.40-1.45 (m), 1.48-1.56 (m), 1.58-1.65 (m), 1.68-1.75 (m), 1.81-1.97 (m), 2.12-2.22 (m), 2.63 (dd, 1H, H13), 2.84 (s, 1H, H-7), 2.88 (d, 1H), 3.46 (d, 1H), 3.49 (d, 1H), 3.56 (dd, 1H), 3.66 (d, 1H), 3.84 (d, 1H), 4.03 (d, 1H), 5.49 (dd, 1H, H5), 6.68-6.74 (m, 1H, H14), 7.58 (t, 1H, ArH), 8.17 (s, 1H, triazole), 9.00 (d, 2H, ArH). | | |
| Mass Spectrum: (ESI) *m*/*z* = 717.76 (M+H). | | |
| | | |
| 197 | R^{I} = H | (1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-14-[5-(3-fluoro-4-pyridinyl)-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 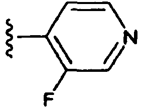 | |
| ¹H NMR (CD₃OD, 600 MHz, ppm) δ 0.77 (s, 3H, Me), 0.77 (d, 3H, Me), 0.82 (d, 3H, Me), 0.82 (s, 3H, Me), 0.85 (d, 3H, Me), 0.85 (s, 3H, Me), 0.85 (d, 3H, Me), 0.90 (d, 3H, Me), 1.17 (s, 3H, Me), 1.21 (s, 3H, Me), 1.23-1.34 (m), 1.40-1.45 (m), 1.49-1.66 (m), 1.69-1.76 (m), 1.79-1.91 (m), 1.93-1.98 (m), 2.00-2.06 (m), 2.13-2.22 (m), 2.54 (dd, 1H, H13), 2.85 (s, 1H, H7), 2.88 (d, 1H), 3.43 (d, 1H), 3.47 (d, 1H), 3.52 (dd, 1H), 3.56 (d, 1H), 3.76 (d, 1H), 3.98 (d, 1H), 5.53 (dd, 1H, H5), 5.53-5.59 (m, 1H, H14),7.72 (t, 1H, ArH), 8.24 (s, 1H, triazole), 8.65 (broad, 1H, ArH), 8.78 (broad, 1H, ArH). | | |
| Mass Spectrum: (ESI) *m*/*z* = 734.61 (M+H). | | |

### EXAMPLES 201-209

The following compounds were prepared using methods analogous to those described in the preceding examples:

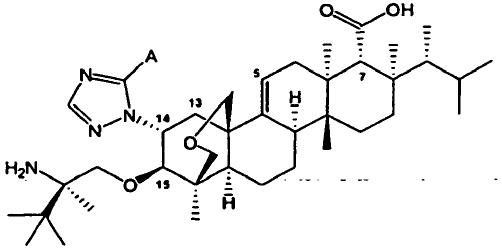

| | | |
|---|---|---|
| 201 | A= | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(3-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | 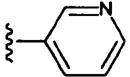 | |
| ¹H NMR (CD₃OD, 600MHz, ppm) δ 0.77 (s, 3H, Me), 0.77 (d, 3H, Me), 0.77 (s, 3H, Me), 0.86 (s, 9H, Me), 0.86 (d, 3H, Me), 0.87 (s, 3H, Me), 0.90 (d, 3H, Me), 1.17 (s, 3H, Me), 1.21 (s, 3H, Me), 1.23-1.34 (m), 1.41-1.45 (m), 1.48-1.65 (m), 1.82-1.98 (m), 2.06-2.11 (m), 2.14-2.21 (m), 2.59 (dd, 1H, H 13), 2.85 (s, 1H, H7), 2.91 (d, 1H), 3.44 (d, 1H), 3.54 (dd, 1H), 3.63 (d, 1H), 3.65 (d, 1H), 3.81 (d, 1H), 4.02 (d, 1H), 5.61 (dd, 1H, H5), 5.82 (m, 1 H, H 14), 7.74 (br d, 1H, pyridyl H), 8.18 (s, 1H, triazole), 8.28 (d, 1H, pyridyl H), 8.82 (br d, 1H, pyridyl H) and 9.02 (br d, 1H, pyridyl H). | | |
| Mass spectrum: (ESI) *m*/*z* = 730.71 (M+H) | | |
| 202 | A= | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyridazinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | 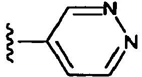 | |
| ¹H NMR (CD₃OD, 600MHz, ppm) δ 0.71 (s, 3H, Me), 0.77 (d, 3H, Me), 0.77 (s, 3H, Me), 0.85 (s, 9H, Me), 0.85 (d, 3H, Me), 0.88 (s, 3H, Me), 0.90 (d, 3H, Me), 1.17 (s, 3H, Me), 1.21 (s, 3H, Me), 1.23-1.34 (m), 1.41-1.45 (m), 1.48-1.65 (m), 1.82-1.98 (m), 2.06-2.11 (m), 2.14-2.21 (m), 2.56 (dd, 1H, H13), 2.85 (s, 1H, H7), 2.87 (d, 1H), 3.48 (d, 1H), 3.57 (dd, 1H), 3.66 (d, 1H), 3.70 (d, 1H), 3.82 (d, 1H), 4.03 (d, 1H), 5.63 (dd, 1H, H5), 5.85 (m, 1H, H 14), 8.08 (dd, 1H, pyridazinyl H), 8.24 (s, 1H, triazole), 9.46 (br d, 1H, pyridazinyl H) and 9.60 (br d, 1H, pyridazinyl H). | | |
| Mass spectrum: (ESI) *m*/*z* = 731.50 (M+H) | | |
| | | |
| 203 | A= | (1*S*,4a*R*,6a*S*,7*R*,8R,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyrimidinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | 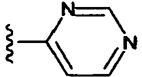 | |
| ¹H NMR (CD₃OD, 600 MHz, ppm) δ 0.64 (s, 3H, Me), 0.76 (d, 3H, Me), 0.77 (s, 3H, Me), 0.82 (s, 9H, t-bu), 0.85 (d, 3H, Me), 0.89 (d, 3H, Me), 0.90 (s, 3H, Me), 1.14 (s, 3H, Me), 1.20 (s, 3H, Me), 1.22-1.37 (m), 1.40-1.45 (m), 1.48-1.55 (m), 1.58-1.65 (m), 1.81-1.96 (m), 2.12-2.22 (m), 2.62 (dd, 1H, H 13), 2.83 (s, 1H, H7), 2.99 (d, 1H), 3.49 (d, 1H), 3.58 (dd, 1H), 3.68 (d, 1H), 3.69 (d, 1H), 3.90 (d, 1H), 4.10 (d, 1H), 5.48 (dd, 1H, H5), 6.93-6.99 (m, 1H, H 14), 8.15 (dd, 1H, ArH), 8.17 (s, 1H, triazole), 8.99 (d, 1H, ArH), 9.34 (d, 1H, ArH). | | |
| Mass Spectrum: (ESI) *m*/*z* = 731.59 (M+H). | | |
| | | |
| 208 | A= | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | 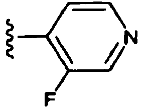 | |
| ¹H NMR (CD₃OD, 600MHz, ppm) δ 0.77 (s, 3H, Me), 0.77 (d, 3H, Me), 0.82 (s, 3H, Me), 0.86 (s, 9H, Me), 0.86 (d, 3H, Me), 0.87 (s, 3H, Me), 0.90 (d, 3H, Me), 1.18 (s, 3H, Me), 1.22 (s, 3H, Me), 1.25-1.36 (m), 1.41-1.45 (m), 1.50-1.66 (m), 1.82-2.00 (m), 2.02-2.07 (m), 2.16-2.22 (m), 2.58 (dd, 1H, H13), 2.85 (s, 1H, H7), 2.88 (d, 1H), 3.43 (d, 1H), 3.54 (dd, 1H), 3.59 (d, 1H), 3.63 (d, 1H), 3.77 (d, 1H), 4.00 (d, 1H), 5.56 (dd, 1H, H5), 5.57 (m, 1H, H 14), 7.72 (dd, 1H, pyridyl H), 8.25 (s, 1H, triazole), 8.66 (d, 1H, pyridyl H) and 8.76 (d, 1H, pyridyl H). | | |
| Mass spectrum: (ESI) *m*/*z* = 748.55 (M+H) | | |
| | | |
| 209 | A= | (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-14-[5-(3-chloro-4-pyridinyl)-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | 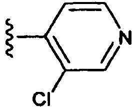 | |
| ¹H NMR (CD₃OD, 600MHz, ppm) δ 0.75 (s, 3H, Me), 0.76 (d, 3H, Me), 0.85 (d, 3H, Me), 0.89 (d, 3H, Me), 0.89 (s, 3H, Me), 0.90 (s, 9H, Me), 0.93 (s, 3H, Me), 1.15 (s, 3H, Me), 1.21 (s, 3H, Me), 1.22-1.31 (m), 1.39-1.44 (m), 1.50-1.64 (m), 1.80-1.96 (m), 2.10-2.21 (m), 2.48 (dd, 1H, H 13), 2.84 (s, 1H, H7), 3.18 (d, 1H), 3.42 (d, 1H), 3.48 (dd, 1H), 3.70 (d, 1H), 3.78 (d, 1H), 4.09 (d, 1H), 5.47 (dd, 1H, H5), 5.50 (m, 1H, H14), 7.62 (dd, 1H, pyridyl H), 8.23 (s, 1H, triazole), 8.71 (d, 1H, pyridyl H) and 8.86 (s, 1H, pyridyl H). | | |

### EXAMPLES 240-249

The following compounds were prepared using methods analogous to those described in the preceding examples:

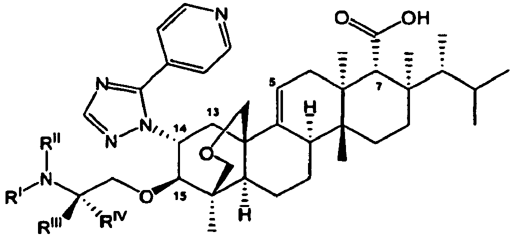

| | | |
|---|---|---|
| 240 | R^{I} = H | (1*S*,4a*R*,6a*S*,7*R*,8*R*,10a*R*,10b*R*,12a*R*,14*R*,15*R*)-15-(2-amino-2-methylpropoxy)-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = H | |
| | R^{III} = Me | |
| | R^{IV} = Me | |
| ¹H NMR (CD₃OD, 500 MHz, ppm) δ 0.79 (s, 3H, Me), 0.79 (d, 3H, Me), 0.88 (d, 3H, Me), 0.88 (s, 3H, Me), 0.92 (d, 3H, Me), 0.93 (s, 3H, Me), 1.09 (s, 3H, Me), 1.19 (s, 3H, Me), 1.23 (s, 3H, Me), 1.25-1.39 (m), 1.42-1.48 (m), 1.50-1.68 (m), 1.83-2.00 (m), 2.07-2.14 (m), 2.15-2.25 (m), 2.57 (dd, 1H, H13), 2.75 (d, 1H), 2.87 (s, 1H, H7), 3.49 (d, 1H), 3.49 (d, 1H), 3.56 (dd, 1H), 3.66 (d, 1H), 3.82 (d, 1H), 4.00 (d, 1H), 5.60 (dd, 1H, H5), 5.88-5.95 (m, 1H, H 14), 7.98 (d, 2H, ArH), 8.22 (s, 1H, triazole), 8.88 (d, 2H, ArH). | | |
| Mass Spectrum: (ESI) *m*/*z* = 688.65 (M+H). | | |
| | | |
| 241 | R^{I} = H | (1*S*,4a*R*,6a*S*,7*R*,8*R*,10a*R*,10b*R*,12a*R*,14*R*,15*R*)-15-(2-amino-2-ethylbutoxy)-8-[(1*H*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = H | |
| | R^{III} = Et | |
| | R^{IV} = Et | |
| ¹H NMR (CD₃OD, 500 MHz, ppm) δ 0.62 (t, J=7.6 Hz, 3H), 0.74 - 0.80 (m, 9H), 0.86 (d, J=6.6 Hz, 1H), 0.88 (s, 3H), 0.90 (d, J=6.8 Hz, 3H), 1.14 - 1.67 (m), 1.17 (s, 3H), 1.22 (s, 3H), 1.80 - 2.0 (m), 2.06 (m, 1H), 2.13 - 2.23 (m, 2H), 2.53 (dd, J=13.7 Hz, 6.2 Hz, 1 H), 2.85 (s, 1H), 2.91 (d, J=10.1 Hz, 1H), 3.43 - 3.49 (m, 2H), 3.54 (dd, J=11.7 Hz, 1.8 Hz, 1H), 3.64 (d, J=11.6 Hz, 1H), 3.80 (d, J=12.1 Hz, 1H), 4.01 (d, J=9.8 Hz, 1H), 5.59 (m, 1H), 5.88 (m, 1H), 7.82 (m, 2H), 8.19 (s, 1H), 8.81 (m, 1H). | | |
| Mass Spectrum: (ESI) *m*/*z* = 716.64 (M+H). | | |
| | | |
| 242 | R^{I} = Me | (1*S*,4a*R*,6a*S*,7*R*,8*R*,10a*R*,10b*R*,12a*R*,14*R*,15*R*)-8-[(1*R*)-1,2-dimethylpropyl]-15-[2-ethyl-2-(methylamino)butoxy]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | R^{II} = H | |
| | R^{III} = Et | |
| | R^{IV} = Et | |
| ¹H NMR (CD₃OD, 500 MHz, ppm) δ 0.63 (t, J=7.6 Hz, 3H), 0.76 - 0.81 (m, 9H), 0.86 (d, J=6.6 Hz, 3H), 0.90 (s, 9H), 0.90 (d, 3H, partially obscured), 1.15-1.67 (m), 1.17 (s, 3H), 1.21 (s, 3H), 1.82 - 2.06 (m), 2.13 - 2.23 (m, 2H), 2.37 (s, 3H), 2.54 (dd, J=13.7 Hz, 6.2 Hz, 1H), 2.85 (s, 1H), 3.01 (d, J=10.7 Hz, 1H), 3.48(d, J=11.9 Hz, 1H), 3.56 (dd, J=11.7 Hz, 2.1 Hz, 1H), 3.60 (d, J=11.0 Hz, 1H), 3.66 (d, J=11.7 Hz, 1H), 3.76 (d, J=12.1 Hz, 1H), 4.01 (d, J=9.6 Hz, 1H), 5.59 (m, 1H), 5.87 (m, 1H), 7.84 (m, 2H), 8.21 (s, 1H), 8.83 (m, 2H). | | |
| Mass Spectrum: (ESI) *m*/*z* = 730.71 (M+H). | | |
| | | |
| 246 | R^{I} = H | (1*S*,4a*R*,6a*S*,7*R*,8*R*,10a*R*,10b*R*,12a*R*,14*R*,15*R*)-15-[[(2*R*)-2-amino-2-(1-methylcyclopropyl)propyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = H | |
| | R^{III} = 1-Me-cPr | |
| | R^{IV} = Me | |
| ¹H NMR (MeOH-d₄, 500MHz, ppm, selected resonances) δ 0.30 (m, 2H), 0.42 (m, 1H), 0.62 (m, 1H), 3.84 (s, 1H), 5.62 (m, 1H), 5.90 (m, 1H), 7.83 (m, 2H), 8.22 (s, 1H), 8.84 (m, 2H) | | |
| Mass Spectrum: (ESI) *m*/*z* = 729 (M+H). | | |
| | | |
| 247 | R^{I} = Me | (1*S*,4a*R*,6a*S*,7*R*,8*R*,10a*R*,10b*R*,12a*R*,14*R*,15*R*)-15-[[(2*R*)-2-(dimethylamino)-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = Me | |
| | R^{III} = t-Bu | |
| | R^{IV} = Me | |
| ¹H NMR (MeOH-d₄, 500MHz, ppm, selected resonances) δ 0.80 (m, 9H), 0.90 (m, 9H), 1.00 (s, 9H), 1.20 (s, 3H), 1.22 (s, 3H), 2.77 (s, 3H), 2.84 (s, 1H), 2.92 (s, 3H), 5.62 (m, 1H), 5.90 (m, 1H), 7.82 (d, 2H), 8.22 (s, 1H), 8.82 (d, 2H) | | |
| Mass Spectrum: (ESI) *m*/*z* = 759 (M+H). | | |
| | | |
| 248 | R^{I} = Et | (1*S*,4a*R*,6a*S*,7*R*,8*R*,10a*R*,10b*R*,12a*R*,14*R*,15*R*)-8-[(1*R*)-1,2-dimethylpropyl]-15-[[(2*R*)-2-(ethylamino)-2,3,3-tnmethylbutyl]oxy]-14-[5-(4-pyridinyl)-1*H*-1,2,4-triazol-1-yl]-1,6,6a,7,8,9,10,10a,10b,11,12,12a-dodecahydro-1,6a,8,10a-tetrarnethyl-*4H*-1,4a-propano-*2H*-phenanthro[1,2-c]pyran-7-carboxylic acid |
| | R^{II} = H | |
| | R^{III} = t-Bu | |
| | R^{IV} = Me | |
| ¹H NMR (MeOH-d₄, 500MHz, ppm, selected resonances) 8 0.72 (s, 3H), 0.75 (m, 6H), 0.90 (m, 18H), 1.18 (s, 3H), 1.23 (s, 3H), 2.83 (s, 1H), 5.60 (m, 1H), 5.83 (m, 1H), 7.80 (d, 2H), 8.20 (s, 1H), 8.80 (d, 2H). | | |
| Mass Spectrum: (ESI) *m*/*z* = 759 (M+H). | | |
| | | |
| 249 | R^{I} = n-Pr | (1*S*,4a*R*,6a*S*,7*R*,8*R*,10a*R*,10b*R*,12a*R*,14*R*,15*R*)-8-((1*R*)-1,2-dimethylpropyl]-14-[5-(4-pyridinyl)-*1H*-1,2,4-triazol-1-yl]-15-[[(2*R*)-2,3,3-trimethyl-2-(propylamino)butyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | R^{II} = H | |
| | R^{III} = t-Bu | |
| | R^{IV} = Me | |
| ¹H NMR (MeOH-d₄, 500MHz, ppm, selected resonances) ¹H NMR (MeOH-d₄, 500MHz, ppm) δ 0.80 (m, 9H), 0.90 (m, 21H), 1.20(s, 3H), 1.23 (s, 3H), 2.84 (2, 1H), 5.62 (m, 1H), 5.90 (m, 1H) 7.82 (d, 2H), 8.22 (s, 1H), 8.83 (d, 2H). | | |
| Mass Spectrum: (ESI) m/z = 773 (M+H). | | |

### EXAMPLES 250-251

The following compounds were prepared using methods analogous to those described in the preceding examples:

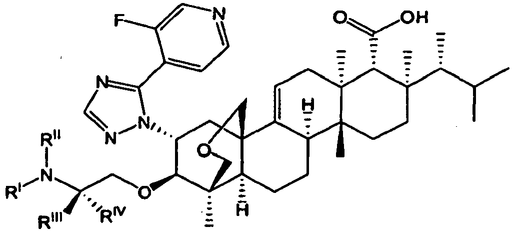

| | | |
|---|---|---|
| 250 | R^{I} = Me | (1*S*,4a*R*,6a*S*,7*R*,8*R*,10a*R*,10b*R*,12a*R*,14*R*,15*R*)-8-[(1*R*)-1,2- |
| | R^{II} = H | dimethylpropyl]-14-[5-(3-fluoro-4-pyridinyl)-*1H*-1,2,4-triazol-1-yl]- |
| | R^{III} = t-Bu | 15-[[(2*R*)-2,3,3-trimethyl-2-(methylamino)butyl]oxy]- |
| | R^{IV} = Me | 1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| ¹H NMR (CD₃OD, 500 MHz, ppm) δ 0.77 (s, 3H), 0.78 (s, 3H), 0.78 (d, 3H, partially obscured), 0.86 (d, J=6.7 Hz, 3H), 0.91 (d, 3H, partially obscured), 0.91 (s, 3H), 0.91 (s, 9H), 1.17 (s, 3H), 1.21 (s, 3H), 1.23- 1.67 (m), 1.82 - 2.0 (m), 2.13- 2.23 (m, 2H), 2.50 (dd, J=13.6 Hz, 5.8 Hz, 1H), 2.60 (s, 3H), 2.85 (s, 1H), 3.22 (d, J=11.2 Hz, 1H), 3.48 (d, J=11.9 Hz, 1H), 3.53 (AB, 2H), 3.61 (d, J=12.3 Hz, 1H), 3.87 (d, J=11.1 Hz, 1H), 4.05 (d, J=9.8 Hz, 1H), 5.51 (m, 1H), 5.59 (m, 1H), 7.72 (m, 1H), 8.25 (m, 1H), 8.66 (m, 1H), 8.79 (m, 1H). | | |
| Mass Spectrum: (ESI) *m*/*z* = 762.96 (M+H). | | |
| | | |
| 251 | R^{I}=H | (1*S*,4a*R*,6aS,7*R*,8*R*,10a*R*,10*R*,10b*R*,12a*R*,14*R*,15*R*)-15-[[(2*R*)-2-amino-2-cyclopropylpropyl]oxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid |
| | R^{II}=H | |
| | R^{III} = c-Pr | |
| | R^{IV} = Me | |
| ¹H NMR (MeOH-d₄, 500MHz, ppm, selected resonances) δ 0.28 (m, 1H), 0.50 (m, 3H), 2.83 (s, 1H), 5.58 (m,2H), 7.72 (m, 1H), 8.23 (s, 1H), 8.62 (m, 1H), 8.79 (m, 1H). | | |
| Mass Spectrum: (ESI) m/z = 732 (M+H). | | |

### EXAMPLES 252-259

The following compounds were prepared using methods analogous to those described in the preceding examples:

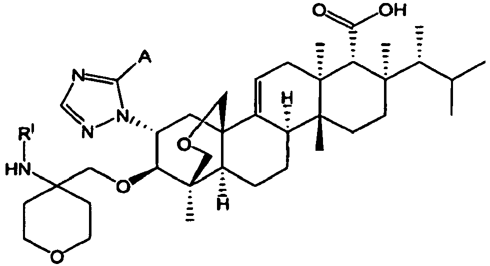

| | | |
|---|---|---|
| 252 | R^{I} = H | (1*S*,4a*R*,6a*S*,7*R*,8*R*,10a*R*,10b*R*,12a*R*,14*R*,15*R*)-15-[(4-aminotetrahydro-*2H*-pyran-4-yl)methoxy]-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-1*H-*phenanthro[1,2-*c*]pyran-7-carboxylic acid |
| | A = | |
| | 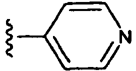 | |
| ¹H NMR (CD₃OD, 500 MHz, ppm) δ 0.79 (s, 3H), 0.80 (d, 3H, partially obscured), 0.88 (d, J=6.6 Hz, 3H), 0.90 (s, 3H), 0.91 (d, J=6.6 Hz, 3H), 1.19 (s, 3H), 1.23 (s, 3H), 1.23 -1.68 (m), 1.82 -2.00 (m), 2.07 (m, 1H), 2.14 - 2.23 (m, 2H), 2.55 (dd, J=13.8, 6.5 Hz, 1H), 2.86 (s, 1H), 2.82 (m, 1H), 3.30-3.36 (m), 3.49 (d, J=11.9 Hz, 1H), 3.53 - 3.61 (m, 3H), 3.65 (d, J=11.7 Hz, 1H), 3.72 (m, 1H), 3.83 (d, J=12.1 Hz, 1H), 4.08 (d, J=9.8 Hz, 1H), 5.59 (m, 1H), 5.90 (m, 1H), 7.83(dd, J=4.8, 1.6 Hz, 2H), 8.25 (s, 1H), 8.83 (d, J=6.1 Hz, 2H). | | |
| Mass spectrum: (ESI) *m*/*z* = 730.81 (M+H) | | |
| | | |
| 253 | R^{I} = Me | (1*S*,4a*R*,6aS,7*R*,8R,10a*R*,10b*R*,12a*R*,14*R*,15*R*)-8-[(1*R*)-1,2-dimethylropyl]-14-5-(3-pyridinyl)-*1H*-1,2,4-triazol-1-yl]-15-[[tetrahydro-4-(methylamino)-*2H-*pyran-4-yl]methoxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 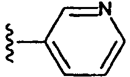 | |
| ¹H NMR (CD₃OD, 500 MHz, ppm) δ 0.78 (s, 3H), 0.79 (d, 3H, partially obscured), 0.87 (d, J=6.7 Hz, 3H), 0.91 (d, 3H, partially obscured), 0.92 (s, 3H), 1.18 (s, 3H), 1.23 (s, 3H), 1.24 - 1.78 (m), 1.82 -2.06 (m), 2.12 - 2.23 (m, 2H), 2.40 (s, 3H), 2.54 (dd, J=13.5, 6.0 Hz, 1H), 2.86 (s, 1H), 2.91 (m, 1H), 3.30-3.37 ( m, partially obscured), 3.46( d, J=11.2 Hz, 1H), 3.50 ( d, J=11.9 Hz, 1H), 3.55 (m, 1H), 3.59 - 3.67 (m, 2H), 3.76 - 3.85 (m, 3H), 4.20 (d, J=9.6 Hz, 1H), 5.58 (m, 1H), 5.85 (m, 1H), 7.83 (br, 1H), 8.26(s, 1H), 8.31 (d, J=7.8 Hz, 1H), 8.90 (br, 2H). | | |
| Mass spectrum: (ESI) *m*/*z* = 744.87 (M+H) | | |
| | | |
| 254 | R^{I} = H | (1*S*,4a*R*,6a*S*,7*R*,8*R*,10a*R*,10b*R*,12a*R*,14*R*,14*R*)-15-[(4-aminotetrahydro-*2H*-pyran-4-yl)methoxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | A = | |
| | 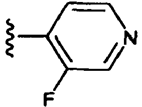 | |
| ¹H NMR (CD₃OD, 500 MHz, ppm) δ 0.78 (s, 3H), 0.79 (d, 3H, partially obscured), 0.87 (d, 3H, partially obscured), 0.88 (s, 3H), 0.91 (d, J=6.7 Hz, 3H), 1.18 (s, 3H), 1.23 (s, 3H), 1.23 -1.68 (m), 1.82 -2.04 (m), 2.12 - 2.23 (m, 2H), 2.50 (dd, J=13.7, 5.9 Hz, 1H), 2.86 (s, 1H), 3.00 (m), 3.36 - 3.42 (m, 2H), 3.45 (d, J=11.9 Hz, 1H), 3.54 (s, 1H), 3.58 (d, J=10.3 Hz, 1H), 3.63 (m, 1H), 3.70 - 3.78 ( m, 2H), 4.09 (d, J=9.8 Hz, 1H), 5.50 - 5.60 ( m, 2H), 7.70(t, J=5.7 Hz, 1H), 8.30 (s, 1H), 8.66 (d, J=4.8 Hz, 1H), 8.78 (s, 1H). | | |
| Mass spectrum: (ESI) *m*/*z* = 748.75 (M+H) | | |
| | | |
| 255 | R^{I} = Me | (1*S*,4a*R*,6a*S*,7*R*,8*R*,10a*R*,10b*R*,12a*R*,14*R*,15*R*)-8-[(1*R*)-1,2-dimethylpropyl]-14-[5-(3-fluoro-4-pyridinyl)-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-4*H-*1,4a-propano-*2H*-phenanthro[1,2-c]pyran-7-carboxylic acid |
| | A= | |
| | 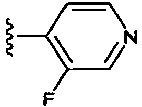 | |
| ¹H NMR (CD₃OD, 500 MHz, ppm) δ 0.78 (s, 3H), 0.79 (d, 3H, partially obscured), 0.88 (d, J=6.6 Hz, 3H), 0.92 (d, 3H, partially obscured), 0.92 (s, 3H), 1.18 (s, 3H), 1.23 (s, 3H), 1.23 - 2.00 (m), 2.12 - 2.23 (m, 2H), 2.43 (s, 3H), 2.50 (dd, J=13.7, 6.0 Hz, 1H), 2.86 (s, 1H), 2.92 (m, 1H,), 3.30 - 3.38 (m, partially obscured), 3.47 (d, J=11.9 Hz, 1H), 3.50 (d, 1H, partially obscured), , 3.54 (s, 1H, partially obscured), 3.65 - 3.74 (m, 2H), 3.77 - 3.86 (m, 2H), 4.20 (d, J=9.9 Hz, 1H), 5.53 (m, 1H), 5.58 (m, 1H), 7.72(t, J=5.5 Hz, 1H), 8.31 (s, 1H), 8.67 (d, J=4.8 Hz, 1H), 8.79 (s, 1H). | | |
| Mass spectrum: (ESI) *m*/*z* = 762.81 (M+H) | | |
| | | |
| 256 | R^{I} = H | (1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid |
| | A= | |
| | 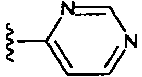 | |
| ¹H NMR (CD₃OD, 500 MHz, ppm) δ 0.79 (s, 3H), 0.79 (d, 3H, partially obscured), 0.87 (d, J=6.7 Hz, 3H), 0.89 (d, 3H, partially obscured), 0.92 (s, 3H), 1.16 (s, 3H), 1.22 (s, 3H), 1.23 -1.68 (m), 1.83 - 1.97 (m), 2.13 - 2.23 (m, 2H), 2.57 (dd, J=13.3, 6.4 Hz, 1H), 2.82 - 2.89 (m, 2H), 3.27 - 3.35 ( m), 3.38 ( d, J=10.3 Hz, 1H), 3.46 (m, 1H), 3.52 ( d, J=11.9 Hz, 1H), 3.60 (m, 1H), 3.66 - 3.74 (m, 2H), 3.91 (d, J=11.9Hz, 1H), 4.19 (d, J= 9.8 Hz, 1H), 5.50 (m, 1H), 6.95 ( m, 1H), 8.17 (d, J= 5.3 Hz, 1H), 8.23 (s, 1H), 9.01 (d, J=5.3 Hz, 1H), 9.35 (s, 1H). | | |
| Mass spectrum: (ESI) *m*/*z* = 731.82 (M+H) | | |
| | | |
| 257 | R^{I} = H | (1*S*,4a*R*,6a*S*,7*R*,8*R*,10a*R*,10b*R*,12a*R*,14*R*,15*R*)-15-[(4-aminotetrahydro-*2H*-pyran-4-yl)methoxy]-8-[(1*R*)-1,2-dimethylpropyl]-14-[5-(5-pyrimidinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid |
| | A = | |
| | 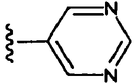 | |
| ¹H NMR (CD₃OD, 500 MHz, ppm) δ 0.78 (s, 3H), 0.79 (d, 3H, partially obscured), 0.87 (d, J=6.6 Hz, 3H), 0.91 (s, 3H), 0.92 ( d, 3H, partially obscured), 1.18 (s, 3H), 1.23 (s, 3H), 1.23 -1.68 (m), 1.83 - 1.98 (m), 2.07 (m, 1H), 2.13 - 2.23 (m, 2H), 2.55 (dd, J=13.7, 6.0 Hz, 1H), 2.86 (s, 1H), 2.96 (m, 1H), 3.32 - 3.39 ( m), 3.50 ( d, J=12.3 Hz, 1H), 3.53 - 3.63 (m, 3H), 3.73 (m, 1H), 3.85 (d, J=12.1 Hz, 1H), 4.10 (d, J= 9.8 Hz, 1H), 5.56 (m, 1H), 5.83 (m, 1H), 8.27 (s, 1H), 9.20 (s, 2H), 9.36 (s, 1H). | | |
| Mass spectrum: (ESI) *m*/*z =* 731.84 (M+H) | | |
| | | |
| 258 | R^{I} = Me | (1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyrimidinyl)-*1H*-1,2,4-triazol-1-yl]-15-[[tetrahydro-4-(methylamino)-*2H*-pyran-4-yl]methoxy]-1,6,6a,7,8,9,10,10a, 10b,11,12,12a-dodecahydro-1,6a,8,10a-tetramethyl*-*4*H-*1,4a-propano-*2H-*phenanthro[1,2-c]pyran-7-carboxylic acid |
| | A = | |
| | 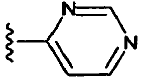 | |
| ¹H NMR (CD₃OD, 500 MHz, ppm) δ 0.78 (s, 3H), 0.78 (d, 3H, partially obscured), 0.87 (d, J=6.7 Hz, 3H), 0.91 d, J=6.8 Hz, 3H), 0.94 (s, 3H), 1.15 (s, 3H), 1.22 (s, 3H), 1.23 -1.68 (m), 1.77 -1.97 (m), 2.02 - 2.23 (m, 2H), 2.31 (s, 3H), 2.57 (dd, J=13.3, 6.4 Hz, 1H), 2.85 (s, 1H), 2.93 (m, 1H), 3.30 - 3.35 (m), 3.50 (d, J=11.0 Hz, 1H), 3.52 - 3.62 (m, 3H), 3.69 ( d, J=11.4 Hz, 1H), 3.80 (m, 1H), 3.83 - 3.89 ( m, 2H), 4.31 (d, J=9.8 Hz, 1H), 5.46 ( m, 1H), 6.92 ( m, 1H), 8.17 (dd, J=5.3, 1.4 Hz, 1H), 8.23 (s, 1H), 9.01 (d, J=5.2 Hz, 1H), 9.35 (d, J=1.2 Hz, 1H) | | |
| Mass spectrum: (ESI) *mlz* = 745.87 (M+H) | | |
| | | |
| 259 | R^{I} = Me | (1*S*,4a*R*,6a*S*,7*R*,8*R*,10a*R*,10b*R*,12a*R*,14*R*,15*R*)-8-[(1*R*)-1,2-dimethylpropyl]-14-[5-(5-pyrimidinyl)1-*1H-*1,2,4-triazol-1-yl]-15-[[tetrahydro-4-(methylamino)-*2H-*pyran-4-yl]methoxy]-1,6,6a,7,8,9,10,10a, 10b,11,12,12a-dodecahydro-1,6a,8,10a-tetrarnethyl-*4H*-1,4a-propano-*2H*-phenanthro[1,2-c]pyran-7-carboxylic acid |
| | A= | |
| | 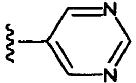 | |
| ¹H NMR (CD₃OD, 500 MHz, ppm) δ 0.78 (s, 3H), 0.79 (d, 3H, partially obscured), 0.87 (d, J=6.6 Hz, 3H), 0.91 ( d, 3H, partially obscured), 0.93 (s, 3H), 1.17 (s, 3H), 1.22 (s, 3H), 1.23 - 1.37 (m), 1.44 (m, 1H), 1.50 - 1.71- (m), 1.77 (m, 1H), 1.82 - 2.05 (m), 2.13 - 2.25 (m, 2H), 2.34 (s, 3H), 2.56 (dd, J=13.8, 7.8 Hz, 1H), 2.86 (s, 1H), 2.93 (m, 1H), 3.32 - 3.37 (m), 3.47 ( d, J=11.2 Hz, 1H), 3.51 (d, J=12.4 Hz, 1H), 3.57 (m, 1H), 3.60 - 3.67 (m, 2H), 3.76 - 3.83 (m, 2H), 3.86 (d, J=12.5 Hz, 1H), 4.22 (d, J= 9.6 Hz, 1H), 5.58 (m, 1H), 5.83 (m, 1H), 8.27 (s, 1H), 9.21 (s, 2H), 9.37 (s, 1H). | | |
| Mass spectrum: (ESI) m/z = 745.87 (M+H) | | |

### EXAMPLE 260

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-[5-(1-oxido-4-pyridinyl)-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

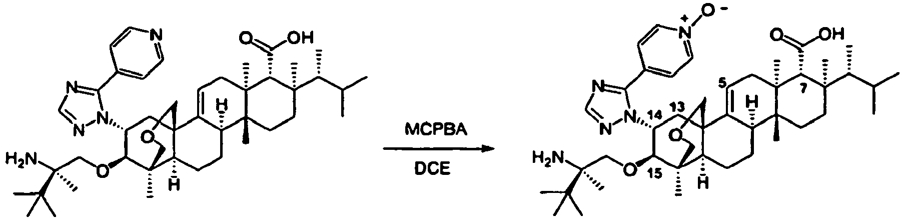

A solution of (1*S*,4a*R*,6a*S*,7*R*,8*R*,10a*R*,10b*R*,12a*R*,14*R*,15*R)*-15-[[(*2R*)-2-amino-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl*-*4*H*-1,4a-propano-*2H-*phenanthro[1,2-c]pyran-7-carboxylic acid (Example 173, 42.2 mg, 0.05 mmol) and 90% meta-chloroperbenzoic acid (42.2 mg, 0.22 mmol) in dichloroethane (2.0 mL) was blanketed with nitrogen and stirred at room temperature for 5 hours. The mixture was evaporated and purified by reverse phase HPLC using a 19 x 150 mm Sunfire Preparative C18 OBD column (elution with MeCN/water + 0.05% TFA). Product fractions were evaporated and freeze-dried from a mixture of ethanol and benzene to give the title compound as a TFA salt (21 mg).

¹H NMR (CD₃OD, 600MHz, ppm) δ 0.74 (s, 3H, Me), 0.77 (d, 3H, Me), 0.78 (s, 3H, Me), 0.85 (s, 9H, Me), 0.86 (d, 3H, Me), 0.88 (s, 3H, Me), 0.90 (d, 3H, Me), 1.17 (s, 3H, Me), 1.21 (s, 3H, Me), 1.23-1.34 (m), 1.41-1.45 (m), 1.48-1.65 (m), 1.82-1.98 (m), 2.06-2.10 (m), 2.14-2.21 (m), 2.61 (dd, 1H, H13), 2.84 (s, 1H, H7), 2.84 (d, 1H), 3.47 (d, 1H), 3.56 (dd, 1H), 3.64 (d, 1H), 3.71 (d, 1H), 3.83 (d, 1H), 4.01 (d, 1 H), 5.63 (dd, 1H, H5), 5.80 (m, 1H, H 14), 7.87 (br d, 2H, pyridyl H), 8.17 (s, 1H, triazole) and 8.49 (d, 2H, pyridyl H).

Mass spectrum: (ESI) *m*/*z* = 746.46 (M+H)

### EXAMPLE 261

### (1S,4aR,6aS,7R,8R,10aR,10bR,12aR,14R,15R)-15-[[(2R)-2-amino-2,3,3-trimethylbutyl]oxy]-8-[(1R)-1,2-dimethylpropyl]-14-[5-(1-oxido-3-pyridinyl)-1H-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

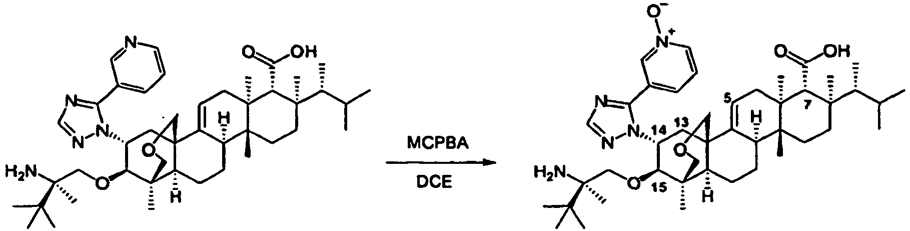

By a procedure analogous to that described for Example 260, but starting with (1*S*,4a*R*,6aS,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(3-pyridinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid (Example 201), the title compound was prepared and isolated as a white solid.

¹H NMR (CD₃OD, 600MHz, ppm) δ 0.77 (s, 3H, Me), 0.77 (d, 3H, Me), 0.77 (s, 3H, Me), 0.86 (s, 9H, Me), 0.86 (d, 3H, Me), 0.89 (s, 3H, Me), 0.90 (d, 3H, Me), 1.17 (s, 3H, Me), 1.21 (s, 3H, Me), 1.23-1.34 (m), 1.41-1.45 (m), 1.48-1.65 (m), 1.82-1.98 (m), 2.04-2.09 (m), 2.14-2.2-1 (m), 2.58 (dd, 1H, H13), 2.85 (s, 1H, H7), 2.91 (d, 1H), 3.47 (d, 1H), 3.55 (dd, 1H), 3.67 (d, 1H), 3.67 (d, 1H), 3.84 (d, 1H), 4.02 (d, 1H), 5.62 (dd, 1H, H5), 5.79 (m, 1H, H14), 7.76 (dd, 1H, pyridyl H), 7.92 (dd, 1H, pyridyl H), 8.18 (s, 1H, triazole), 8.49 (br dd, 1H, pyridyl H) and 8.66 (br dd, 1 H, pyridyl H).

Mass spectrum: (ESI) *m*/*z* = 746.46 (M+H)

### EXAMPLE 262

### (1S,4aR,6aS,7R,8R,10aR,10bR,12aR,14R,15R)-15-[[(2R)-2-amino-2,3-dimethylbutyl]oxy]-8-[(1R)-1,2-dimethylpropyl]-14-[5-(1-oxido-4-pyridinyl)-1N-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carboxylic acid

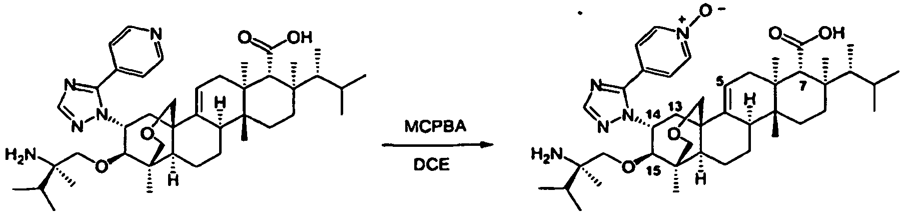

By a procedure analogous to that described for Example 260, but starting with (1*S*,4*aR*,6*aS*,7*R*,8*R*,10a*R,*10b*R,*12*aR,*14*R,*15*R)-*15*-*[[(2*R)-2-*amino*-2,3-*dimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid (Example 184), the title compound was prepared and isolated as a white solid.

¹H NMR (CD₃OD, 500 MHz, ppm) δ 0.78 (s, 3H, Me), 0.79 (d, 3H, Me), 0.80 (s, 3H, Me), 0.82 (d, 3H, Me), 0.85 (d, 3H, Me), 0.88 (d, 3H, Me), 0.89 (s, 3H, Me), 0.92 (d, 3H, Me), 1.19 (s, 3H, Me), 1.23 (s, 3H, Me), 1.25-1.39 (m), 1.43-1.48 (m), 1.51-1.69 (m), 1.70-1.78 (m), 1.83-2.01 (m), 2.05-2.12 (m), 2.15-2.25 (m), 2.59 (dd, 1H, H13), 2.86 (d, 1H), 2.87 (s, 1H, H7), 3.50 (d, 1H), 3.52 (d, 1H), 3.58 (dd, 1H), 3.71 (d, 1H), 3.86 (d, 1H), 4.01 (d, 1H), 5.62 (dd, 1H, H5), 5.81-5.88 (m, 1H, H 14), 7.90 (d, 2H, ArH), 8.19 (s, 1H, triazole), 8.51 (d, 2H, ArH).

Mass Spectrum: (ESI) *m*/*z* = 732.61 (M+H).

It will be appreciated that various of the above-discussed and other features and functions, or alternatives thereof, may be desirably combined into many other different systems or applications. Also that various presently unforeseen or unanticipated alternatives, modifications, variations or improvements therein may be subsequently made by those skilled in the art which are also intended to be encompassed by the following claims.

## Claims

1. A compound of Formula (I): 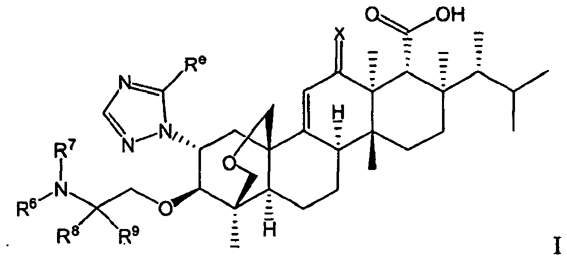 or a pharmaceutically acceptable salt thereof, wherein:
X is H, H; or O;
R^{e} is C(O)NR^{f}R^{g} or a 6-membered ring heteroaryl group containing 1 or 2 nitrogen atoms wherein the heteroaryl group is optionally mono-substituted on a ring carbon with fluoro or chloro or on a ring nitrogen with oxygen;
R^{f} R^{g}, R⁶ and R⁷ are each independently hydrogen or C₁-C₃ alkyl;
R⁸ is C₁-C₄ alkyl, C₃-C₄ cycloalkyl or C₄-C₅ cycloalkyl-alkyl;
R⁹ is methyl or ethyl; and
R⁸ and R⁹ are optionally taken together to form a 6-membered saturated ring containing 1 oxygen atom.

2. The compound of claim 1, wherein X is H, H.

3. The compound of claim 1 or claim 2, wherein R^{e} is either pyridyl or pyrimidinyl optionally mono-substituted on a ring carbon with fluoro or chloro or on a ring nitrogen with oxygen.

4. The compound of claim 3, wherein R^{e} is 4-pyridyl.

5. The compound of claim 1 or claim 2, wherein R^{e} is C(O)NH₂ or C(O)NH(C₁-C₃ alkyl).

6. The compound of claim 5, wherein R^{e} is C(O)NH₂.

7. The compound of any one of claims 1 to 6, wherein R⁸ is C₁-C₄ alkyl and R⁹ is methyl.

8. The compound of claim 7, wherein R⁸ is t-butyl.

9. The compound of any one of claims 1 to 8, wherein R⁶ and R⁷ are each independently hydrogen or methyl.

10. The compound according to claim 1, wherein the compound is selected from the group consisting of:
(1*S*,4a*R*,6a*S*,7*R*,8*R*,10a*R*,10b*R*,12a*R*,14*R*,15*R)-*15*-*[[(2*R)-*2*-*amino*-*2,3-dimethylbutyl]oxy]-8-[(1*R*)-1,2-dimethylpropyl]-14-[5-[[(1-methylethyl)amino]carbonyl]-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-[[(1-methylethyl)amino]carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6aS,7*R*,8*R*,10a*R*,10b*R*,12a*R*,14*R*,15*R*)-15-[[(2*R*)-2,3-dirnethyl-2-(methylamino)butyl]oxy]-8-[(1*R*)-1,2-dimethylpropyl]-14-[5-[[(1-methylethyl)amino]carbonyl]-1*H-*1,2,4-triazol-1-yl]-1,6,6a,7,8,9,10,10a,10b,11,12,12a-dodecahydro-1,6a,8,10a-tetramethyl-6-oxo-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-[(methylamino)carbonyl]-*1H-*1,2,4-triazol-1-yl]-1,6,6a,7,8,9,10,10a,10b,11,12,12a-dodecahydro-1,6a,8,10a-tetramethyl-6-oxo-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-14-[5-(aminocarbonyl)-1*H-*1,2,4-triazol-1-yl]-15-[[(2*R*)-2-amino-2,3-dimethylbutyl]oxy]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-14-[5-(aminocarbonyl)-*1H-*1,2,4-triazol-1-yl]-15-[[(2*R*)-2-(dimethylamino)-2,3-dimethylbutyl]oxy]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H*-phenanthro[1,2-*c*]pyran-7-carboxylic acid;
(1*S*,4a*R*,6a*S*,7*R*,8*R*,10a*R*,10b*R*,12a*R*,14*R*,15*R*)-14-[5-(aminocarbonyl)-*1H-*1,2,4-triazol-1-yl]-8-[(1*R*)-1,2-dimethylpropyl]-15-[[(2*R*)-2-(ethylamino)-2,3-dimethylbutyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-14-[5-(aminocarbonyl)-*1H-*1,2,4-triazol-1-yl]-15-[[(2*R*)-2-amino-2,3,3-trimethylbutyl]oxy]-8-[(1*R*)-1,2-dimethylpropyl]-1,6,6a,7,8,9,10,10a,10b,11,12,12a-dodecahydro-1,6a, 8,10a-tetramethyl-*4H*-1,4a-propano-2*H-*phenanthro[1,2-c]pyran-7-carboxylic acid;
(1*S*,4a*R*,6aS,7*R*,8*R*,10a*R*,10b*R*,12a*R*,14*R*,15*R*)-8-[(1*R*)-1,2-dimethylpropyl]-14-[5-[(methylamino)carbonyl]-*1H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-14-[5-(aminocarbonyl)-1*H-*1,2,4-triazol-1-yl]-8-[(1*R*)-1,2-dimethylpropyl]-15-[[tetrahydro-4-(methylamino)-*2H-*pyran-4-yl]methoxy]-1,6,6a,7,8,9,10,10a,10b,11,12,12a-dodecahydro-1,6a,8,10a-tetramethyl-4*H*-1,4a-propano-*2H-*phenanthro[1,2-c]pyran-7-carboxylic acid;
(1*S*,4a*R*,6aS,7*R*,8*R*,10a*R*,10*R*,12a*R*,14*R*,15*R*)-14-[5-(aminocarbonyl)-*1H-*1,2,4-triazol-1-yl]-8-[(1*R*)-1,2-dimethylpropyl]-15-[[(2*R*)-2,3-dimethyl-2-(methylamino)butyl]oxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R)*-14-[5-(aminocarbonyl)-*1H-*1,2,4-triazol-1-yl]-8-[(1*R*)-1,2-dimethylpropyl]-15-[[(*2R*)-2,3-dimethyl-2-(propylamino)butyl]oxy]-1,6,6a,7,8,9,10,10a,10b,11,12,12a-dodecahydro-1,6a,8,10a-tetramethyl-*4H-*1,4a-propano-2*H*-phenanthro[1,2-c]pyran-7-carboxylic acid;
(1*S*,4a*R*,6aS,7*R*,8*R*,10aR,10b*R*,12a*R*,14*R*,15*R*)-15-[[(2*R*)-2-amino-2,3-dimethylbutyl]oxy]-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-14-[5-[(dimethylamino)carbonyl]-*1H-*1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-[(methylamino)carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-(dimethylamino)-2,3-dimethylbutyl]oxy]-8-[(1*R*)-1,2-dimethylpropyl]-14-[5-[(methylamino)carbonyl]-*1H-*1,2,4-triazol-1-yl]-1,6,6a,7,8,9,10,10a,10b,11,12,12a-dodecahydro-1,6a, 8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R)*-1,2-dimethylpropyl]-15-[[(2*R*)-2,3-dimethyl-2-(propylamino)butyl]oxy]-14-[5-[(methylamino)carbonyl]-*1H-*1,2,4-triazol-1-yl]-1,6,6a,7,8,9,10,10a,10b,11,12,12a-dodecahydro-1,6a,8,10a-tetramethyl-4H-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-14-[5-[(dimethylamino)carbonyl]-*1H*-1,2,4-triazol-1-yl]-15-[[(2*R*)-2,3-dimethyl-2-(methylamino)butyl]oxy]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4aR,6aS,7R,8R,10aR,10b*R*,12a*R*,14*R*,15*R*)-14-[5-[(dimethylamino)carbonyl]-*1H-*1,2,4-triazol-1-yl]-8-[(1*R*)-1,2-dimethylpropyl]-15-[[(2*R*)-2-(ethylamino)-2,3-dimethylbutyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-[(ethylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-[(ethylamino)carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-14-[5-[(diethylamino)carbonyl]-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1S,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-[(propylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-(dimethylamino)-2,3-dimethylbutyl]oxy]-8-[(1*R*)-1,2-dimethylpropyl]-14-[5-[[(1-methylethyl)amino]carbonyl]-*1H-*1,2,4-triazol-1-yl]-1,6,6a,7,8,9,10,10a,10b,11,12,12a-dodecahydro-1,6a,8,10a-tetramethyl-*1H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-15-[[(*2R*)-2-(ethylamino)-2,3-dimethylbutyl]oxy]-14-[5-[[(1-methylethyl)amino]carbonyl]-*1H-*1,2,4-triazol-*1*-yl]-1,6,6a,7,8,9,10,10a,10b,11,12,12a-dodecahydro-1,6a,8,10a-tetramethy-*4H*-1,4a-propano-*2H*-phenanthro[1,2-c]pyran-7-carboxylic acid;
(1*S*,4a*R*,6a*S*,7*R*, 8*R*,10a*R*,10b*R*,12a*R*,14*R*,15*R*)-8-[(1*R*)-1,2-dimethylpropyl)-15-[[(2*R*)-2,3-dimethyl-2-(propylamino)butyl]oxy]-14-[5-[[(1-methylethyl)amino]carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-14-[5-(aminocarbonyl)-*1H-*1,2,4-triazol-1-yl]-8-[(1*R*)-1,2-dimethylpropyl]-15-[[(*2R*)-2,3,3-trimethyl-2-(methylamino)butyl]oxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-[(methylamino)carbonyl]-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1S,4a*R*,6a*S*,7*R*,8*R*,10a*R*,10b*R*,12a*R*,14*R*,15*R*)-15-[[(2*R*)-2-amino-2,3,3-trimethylbutyl]oxy]-14-[5-[(dimethylamino)carbonyl]-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-[(ethylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-[[(1-methylethyl)amino]carbonyl]-*1H-*1,2,4-triazol-1-yl]-1,6,6a,7,8,9,10,10a,1b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-[[(1-methylethyl)amino]carbonyl]-*1H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10bR,12a*R*,14*R*,15*R*)-14-[5-(aminocarbonyl)-*1H-*1,2,4-triazol-1-yl]-15-(2-ammo-2-ethylbutoxy)-8-[(1*R*)-1,2-dimethylpropyl]-1,6,6a,7,8,9,10,10a,10b,11,12,12a-dodecahydro-1,6a,8,10a-tetramethyl-4*H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-(2-amino-2-ethylbutoxy)-8-[(1*R*)-1,2-dimethylpropyl]-14-[5-[(methylamino)carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1S,4a*R*,6a*S*,7*R*,8*R*,10a*R*,10b*R*,12a*R*,14*R*,15*R*)-15-(2-amino-2-ethylbutoxy)-8-[(1*R*)-1,2-dimethylpropyl]-14-[5-[[(1-methylethyl)amino]carbonyl]-1*H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1S,4a*R*,6a*S*,7*R*,8*R*,10a*R*,10b*R*,12a*R*,14*R*,15*R*)-14-[5-(aminocarbonyl)-*1H-*1,2,4-triazol-1-yl]-15-[(4-aminotetrahydro-*2H-*pyran-4-yl)methoxy]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1S,4a*R*,6a*S*,7*R*,8*R*,10a*R*,10b*R*,12a*R*,14*R*,15*R*)-8-[(1*R*)-1,2-dimethylpropyl]-14-[5-[(methylamino)carbonyl]-*1H*-1,2,4-triazol-1-yl]-15-[[tetrahydro-4-(methylamino)-*2H-*pyran-4-yl]methoxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*, 10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1;6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1R)-1,2-dimethylpropyl]-14-[5-[[(1-methylethyl)amino]carbonyl]-*1H-*1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethytbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyridinyl)-*1H*-1,2,4-triazol-1-yl]-15-[[(2*R*)-2,3,3-timethyl-2-(methylamino)butyl]oxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(5-pyrimidinyl)-*1H*-1,2,4-triazol-1-yl]-1,6,6a,7,8,9,10,10a,10b,11,12,12a-dodecahydro-1,6a,8,10a-tetramethyl-4H-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyridinyl)-1H-1,2,4-triazol-1-yl]-15-[[tetrahydro-4-(methylamino)-*2H-*pyran-4-yl]methoxy]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(3-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-(3-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyridinyl)-*1H*,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-(2-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-(5-pyrazinyl-*1H-*1,2,4-triazol-1-yl)-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyridazinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(3-pyridazinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyrimidinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(5-pyrimidinyl)-1H-1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(2-pyrimidinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-14-[5-(3-fluoro-4-pyridinyl)-*1H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*-1,2-dimethylpropyl]-14-[5-(3-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyridazinyl)-*1H-1*,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyrimidinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(3-fluoro-4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-14-[5-(3-chloro-4-pyridinyl)-*1H-*1,2,4-triazol-1-yl]-8-[(1*R*)*-*1,2-dimethylpropyl]-1,6,6a,7,8,9,10,10a,10b,11,12,12a-dodecahydro-1,6a, 8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-(2-amino-2-methylpropoxy)-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-(2-amino-2-ethylbutoxy)-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-((1*R*)-1,2-dimethylpropyl]-15-[2-ethyl-2-(methylamino)butoxy]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*S*)-2-amino-2,3-dimethylbutyl]oxy]-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2-(1-methylcyclopropyl)propyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-(dimethylamino)-2,3,3-trimethylbutyl]oxy]-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
*(1S*,4a*R*,6a*S*,7*R*,8*R*,10a*R*,10b*R*,12aR,14*R*,15*R*)-8-[(1*R*)-1,2-dimethylpropyl]-15-[((2*R*)-2-(ethylamino)-2,3,3-trimethylbutyl]oxy]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1S,4a*R*,6aS,7*R*,8*R*,10a*R*,10b*R*,12a*R*,14*R*,15*R*)-8-[(1*R*)-1,2-dimethylpropyl]-14-[5-(4-pyridinyl)-1H-1,2,4-triazol-1-yl]-15-[[(2*R*)-2,3,3-trimethyl-2-(propylamino)butyl]oxy]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10aR,10b*R*,12a*R*,14*R*,15*R*)-8-[(1*R*)-1,2-dimethylpropyl]-14-[5-(3-fluoro-4-pyridinyl)-1*H*-1,2,4-triazol-1-yl]-15-[[(*2R*)-2,3,3-trimethyl-2-(methylamino)butyl]oxy]-1,6,6a,7,8,9,10,10a,10b,11,12,12a-dodecahydro-1,6a,8,10a-tetrarnethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2-cyclopropylpropyl]oxy]-8-[(1*R*)-1,2-dimethylpropyl]-14-[5-(3-fluoro-4-pyridinyl)-*1H*,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(3-pyridinyl)-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*, 7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(3-fluoro-4-pyridinyl)-*1H*-1,2)4-triazol-1-yl]-15-[[tetrahydro-4-(methylamino)-*2H-*pyran-4-yl]methoxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R,*14*R*,15*R*)-15-[(4-aminotetrahydro-*2H-*pyran-4-yl)methoxy]-8-[(1*R)*-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(5-pyrimidinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10aR,10b*R*,12a*R*,14*R*,15*R*)-8-[(1*R*)-1,2-dimethylpropyl]-14-[5-(4-pyrimidinyl)-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H-1*,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)1,2-dimethylpropyl]-14-[5-(5-pyrimidinyl)-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,68,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(1-oxido-4-pyridinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R,*12a*R*,14*R*,15*R*)-15-[[(2*R*)-2-amino-2,3,3-trimethylbutyl]oxy]-8-[(1*R*)-1,2-dimethylpropyl]-14-[*S*-(1-oxido-3-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(1-oxido-4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid; and
pharmaceutically acceptable salts thereof.

11. The compound according to claim 1, wherein the compound is selected from the group consisting of:
(1*S*,4a*R*,6a*S*,7*R*,8*R*,10aR,10b*R*,2a*R*,14*R*,15*R*)-15-[[(2*R*)-2,3-dimethyl-2-(methylamino)butyl]oxy]-8-[(1*R*)-1,2-dimethylpropyl]-14-[5-[[(1-methylethyl)amino]carbonyl]-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S,*4aR*,*6a*S,*7*R*,8*R*,10a*R*,10b*R*,12a*R*,14*R*,15*R*)-14-[5-(aminocarbonyl)-*1H-*1,2,4-triazol-1-yl]-15-[[(2*R*)-2-amino-2,3,3-trimethylbutyl]oxy]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-[(methylamino)carbonyl]-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,10a*R*,14*R*,15*R*)-15-[[(2*R*)-2-amino-2,3,3-trimethylbutyl]oxy]-8-[(1*R*)-1,2-dimethylpropyl]-14-[5-[[(1-methylethyl)amino]carbonyl]-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyridinyl)-*1H-*1,2,4-triazol-1-yl]-1-yl-[[(2*R*)-2,3,3-trimethyl-2-(methylamino)butyl]oxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,16*R*)-15-[[(2*R*)-2-amino-2,3,3-trimethylbutyl]oxy]-8-[(1*R*)-1,2-dimethylpropyl]-14-[5-(5-pyrimidinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl] - 14-[5-(4-pyridinyl)-1H-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S,*4a*R,*6a*S,*7*R,*8*R,*10a*R,*10b*R,*12a*R,*14*R,*15*R*)-15-[[(2*R*)-2-amino-2,3-dimethylbutyl]oxy]-8-[(1*R*)-1,2-dimethylpropyl]-14-[5-(3-pyridinyl)-*1H-*1,2,4-triazol-1-yl]-1,b,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-(3-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyrimidinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(5-pyrimidinyl)-1H-1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-14-[5-(3-fluoro-4-pyridinyl)-*1H-*1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropy]]-14-[5-(3-pyridinyl)-*1H*-1,2,4-triazol-1-yl]-1,6,6a,7,8,9,10,10a,1Ob,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S,*7*R*,8*R*,10a*R*,10ba*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyrimidinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-14-[5-(3-chloro-4-pyridinyl)-*1H*-1,2,4-triazol-1-yl]-8-[(1*R*)-1,2-dimethylpropyl]-1,6,6a,7,8,9,10,11a,10b,11,12,12a-dodecahydro-1,6a,8,10a-tetramethyl-*4H-*1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-(2-amino-2-methylpropoxy)-8-[(1*R*)-1,2-dimethylpropyl]-14-[5-(4-pyridinyl)-*1H*-1,2,4-triazo1-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-(2-amino-2-ethylbutoxy)-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*1,10a*R*,10b*R*,12a*R*,14*R*,15*R*)-8-[(1*R*)-1,2-dimethylpropyl]-15-[2-ethyl-2-(methylamino)butoxy]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2-(1-methylcyclopropyl)propyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-(dimethylamino)-2,3,3-trimethylbutyl]oxy]-8-[(1*R*)-1,2-dimethylpropyl]-14-[5-(4-pyridinyl)-*1H-*1,2,4-triazol-1-yl]-1,6,6a,7,8,9,10,10a,10b,11,12,12a-dodecahydro-1,6a,8,10a-tetramethyl-*4H-*1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-15-[[(2*R*)-2-(ethylamino)-2,3,3-trimethylbutyl]oxy]-14-[5-(4-pyridinyl)-1*H-*1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyridinyl)-*1H*-1,2,4-triazol-1-yl]-15-[[(2*R*)-2,3,3-trimethyl-2-(propylamino)butyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(3-fluoro-4-pyridinyl)-*1H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4aR,6a*S*,7*R*,8*R*,10a*R*,10b*R*,12a*R*,14*R*,15*R*)-15-[[(2*R*)-2-amino-2-cyclopropylpropyl]oxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-2Hphenanthro[1,2-c]pyran-7-carboxylic acid;
(1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(3-pyridinyl)-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H*-1,4a-propano-2*H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(3-fluoro-4-pyridinyl)-*1H*-1,2,4-triazol-1-yl]-15-[[tetrahydro-4-(methylamino)-*2H-*pyran-4-yl]methoxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(5-pyrimidinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[(4-aminotetrahydro-*2H-*pyran-4-yl)methoxy]-8-[(1*R*)-1,2-dimethylpropyl]-14-[5-(4-pyrimidinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyrimidinyl)-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(5-pyrimidinyl)-*1H*-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(1-oxido-4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-2*H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(1-oxido-3-pyridinyl)-1*H*-1,2,4-triazol-1-yl]-1,6,6a,7,8,9,10,10a,10b,11,12,12a-dodecahydro-1,6a,8,10a-tetramethyl-4*H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(1-oxido-4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-2*H*-phenanthro[1,2-c]pyran-7-carboxylic acid; and
pharmaceutically acceptable salts thereof.

12. The compound according to claim 1, wherein the compound is selected from the group consisting of:
(1*S*,4a*R*,6a*S*,7*R*,8*R*,10a*R*,10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-[[(1-methylethyl)amino]carbonyl]-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-14-[5-(aminocarbonyl)-*1H-*1,2,4-triazol-1-yl]-15-[[(2*R*)-2-amino-2,3,3-trimethylbutyl]oxy]-8-[(1*R*)-1,2-dimethylpropyl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyridinyl)-*1H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-8-[(1*R*)-1,2-dimethylpropyl]-14-[5-(4-pyridinyl)-1H-1,2,4-triazol-1-yl]-15-[[tetrahydro-4-(methylamino)-*2H*-pyran-4-yl]methoxy]-1,6,6a,7,8,9,10,10a,10b,11,12,12a-dodecahydro-1,6a,8,10a-tetramethyl-*4H*-1,4a-propano-2*H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(3-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2,3-dimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid;
(1*S*,4a*R*,6a*S*,7*R*,8*R*,10a*R,*10b*R*,12a*R*,14*R*,15*R*)-15-[[(2*R*)-2,3-dimethyl-2-(methylamino)butyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-,4a-propano-*2H*-henanthro[1,2-c]pyran-7-carboxylic acid;
(1*S*,4a*R*,6a*S*,7*R*,8*R,*10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(3-fluoro-4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H*-phenanthro[1,2-c]pyran-7-carboxylic acid;
(1*S*,4a*R*,6a*S*,7*R*,8*R*,10a*R*,10b*R*,12a*R*,14*R*,15*R*)-15-[[(2*R*)-2-amino-2-(1-methylcyclopropyl)propyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-2*H*-phenanthro[1,2-c]pyran-7-carboxylic acid; and
pharmaceutically acceptable salts thereof.

13. The compound according to claim 1, wherein the compound is (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid or a pharmaceutically acceptable salt thereof.

14. A pharmaceutical composition comprising the compound of any one of claims 1 to 13 and a pharmaceutically acceptable carrier, adjuvant or vehicle.

15. The composition according to claim 14, further comprising a second therapeutic agent.

16. A compound of any one of claims 1 to 13 for use in the treatment of a fungal infection in a patient in need thereof.

17. The compound for use according to claim 16, wherein said fungal infection is caused by *Cryptococcus spp., Candida spp.* or *Aspergillus spp.* fungi.

18. Use of a compound of any one of claims 1 to 13 in the manufacture of a medicament for treating a fungal infection.

19. The composition of claim 15, wherein the second therapeutic agent is an azole, a polyene, a purine or pyrimidine nucleotide inhibitor, a pneumocandin or echinocandin derivative, a protein elongation factor inhibitor, a chitin inhibitor, a mannan inhibitor, a bactericidal or permeability-inducing (BPI) protein product, or an immunomodulating agent.

20. The composition of claim 19, wherein the second therapeutic agent is itraconazole, ketoconazole, miconazole, fluconazole, voriconazole, posaconazole, amphotericin B, flucytosine, anidulafungin, micafungin, or caspofungin.

21. The compound for use of claim 16, wherein the compound of any one of claims 1 to 13, is administered in combination, either sequentially or concurrently, with a second therapeutic agent effective against fungal or bacterial infections.

22. The compound for use of claim 21, wherein the second therapeutic agent is an azole, a polyene, a purine or pyrimidine nucleotide inhibitor, a pneumocandin or echinocandin derivative, a protein elongation factor inhibitor, a chitin inhibitor, a mannan inhibitor, a bactericidal or permeability-inducing (BPI) protein product, or an immunomodulating agent.

23. The compound for use of claim 22, wherein the second therapeutic agent is itraconazole, ketoconazole, miconazole, fluconazole, voriconazole, posaconazole, amphotericin B, flucytosine, anidulafungin, micafungin, or caspofungin.

## Patentansprüche

1. Verbindung der Formel (I): 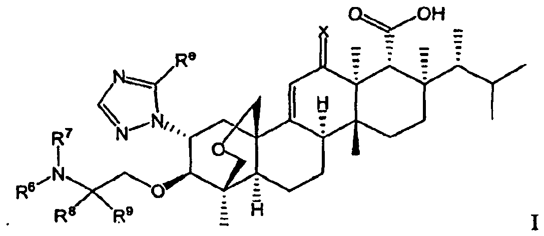 oder ein pharmazeutisch annehmbares Salz davon, worin:
X für H, H; oder O steht;
R^{e} für C(O)NR^{f}R^{g} oder eine 6-gliedrige Ring-Heteroarylgruppe steht, welche 1 oder 2 Stickstoffatome enthält, wobei die Heteroarylgruppe gegebenenfalls an einem Ring-Kohlenstoff mit Fluor oder Chlor oder an einem Ring-Stickstoff mit Sauerstoff mono-substituiert ist;
R^{f}, R^{g}, R⁶ und R⁷ jeweils unabhängig Wasserstoff oder C₁-C₃-Alkyl darstellen;
R⁸ für C₁-C₄-Alkyl, C₃-C₄-Cycloalkyl oder C₄-C₅-Cycloalkyl-alkyl steht;
R⁹ für Methyl oder Ethyl steht; und
R⁸ und R⁹ gegebenenfalls zusammengenommen sind, um einen 6-gliedrigen gesättigten Ring zu bilden, welcher 1 Sauerstoffatom enthält.

2. Verbindung nach Anspruch 1, worin X für H, H steht.

3. Verbindung nach Anspruch 1 oder Anspruch 2, worin R^{e} für entweder Pyridyl oder Pyrimidinyl steht, gegebenenfalls an einem Ring-Kohlenstoff mit Fluor oder Chlor oder an einem Ring-Stickstoff mit Sauerstoff mono-substituiert.

4. Verbindung nach Anspruch 3, worin R^{e} für 4-Pyridyl steht.

5. Verbindung nach Anspruch 1 oder Anspruch 2, worin R^{e} für C(O)NH₂ oder C(O)NH(C₁-C₃-Alkyl) steht.

6. Verbindung nach Anspruch 5, worin R^{e} für C(O)NH₂ steht.

7. Verbindung nach einem von Ansprüchen 1 bis 6, worin R⁸ für C₁-C₄-Alkyl steht und R⁹ Methyl darstellt.

8. Verbindung nach Anspruch 7, worin R⁸ für t-Butyl steht.

9. Verbindung nach einem von Ansprüchen 1 bis 8, worin R⁶ und R⁷ jeweils unabhängig Wasserstoff oder Methyl darstellen.

10. Verbindung gemäß Anspruch 1, wobei die Verbindung ausgewählt ist aus der Gruppe bestehend aus:
(1*S*, 4a*R*, 6a*S*, 7*R*, 8*R*, 10a*R*, 10b*R*, 12a*R*, 14*R*, 15*R*) -15- [[(2*R*)-2-Amino-2,3-dimethylbutyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Di-methyl-2-(methylamino)butyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-6-oxo-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-6-oxo-4*H*-1,4a-propano-2*H*-phenanthro[1,2-c]pyran-7-carbon-säure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-15-[[(2*R*)-2-amino-2,3-dimethylbutyl]oxy]-8-[(1*R*)-1,2-dimethylpropyl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-15-[[(2*R*)-2-(dimethylamino)-2,3-dimethylbutyl]oxy]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-8-[(1*R*)-1,2-dimethylpropyl]-15-[[(2*R*)-2-(ethylamino)-2,3-dimethylbutyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-15-[[(2*R*)-2-amino-2,3,3-trimethyl-butyl]oxy]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-[(methylamino)carbonyl]-1*H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-8-[(1*R*)-1,2-dimethylpropyl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-8-[(1*R*)-1,2-dimethylpropyl]-15-[[(2*R*)-2,3-dimethyl-2-(methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocar-bonyl)-1*H*-1,2,4-triazol-1-yl]-8-[(1*R*)-1,2-dimethylpropyl]-15-[[(2*R*)-2,3-dimethyl-2-(propylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-14-[5-[(dimethylamino)carbonyl]-1*H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Di-methyl-2-(methylamino)butyl]oxy)-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-(Dimethylamino)-2,3-dimethylbutyl]oxy]-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,1*R*,8*R*,10a*R*,10b*R*,12a*R*,14*R*,15*R*)-8-[(1*R*)-1,2-Dimethylpropyl]-15-[[(2*R*)-2,3-dimethyl-2-(propylamino)butyl]oxy]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-[(Dimethylamino)carbonyl]-1*H*-1,2,4-triazol-1-yl]-15-[[(2*R*)-2,3-dimethyl-2-(methylamino)butyl]oxy]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-[(Dimethylamino)carbonyl]-1*H*-1,2,4-triazol-1-yl]-8-[(1*R*)-1,2-dimethyl-propyl]-15-[[(2*R*)-2-(ethylamino)-2,3-dimethylbutyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-[(ethylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-[(ethylamino)carbonyl]-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-14-[5-[(diethylamino)carbonyl]-1*H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-[(propylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-(Dimethylamino)-2,3-dimethylbutyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-15-[[(2*R*)-2-(ethylamino)-2,3-dimethylbutyl]oxy]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-15-[[(2*R*)-2,3-dimethyl-2-(propylamino)butyl]oxy]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-8-[(1*R*)-1,2-dimethylpropyl]-15-[[(2*R*)-2,3,3-trimethyl-2-(1-methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-14-[5-[(dimethylamino)carbonyl]-1*H-*1,2,4-triazol-1-yl]-8-[(1*R*)-1,2-dimethylpropyl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-[(ethylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-[[(1-methylethyl)amino]carbonyl]-1*H*-1,2,4-triazol-1-yl]-15-[[(2*R*)-2,3,3-trimethyl-2-(methylamino)butyl]oxy]-1,6,6a,7,8,9,10,10a,10b,11,12,12a-dode-cahydro-1,6a,8,10a-tetramethyl-4*H*-1,4a-propano-2*H-*phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-15-(2-amino-2-ethylbutoxy)-8-[(1*R*)-1,2-dimethylpropyl]-1,6,6a,7,8,9,10,10a,10b,11,12,12a-dodecahydro-1,6a,8,10a-tetramethyl-4*H*-1,4a-propano-2*H-*phenanthro[1,2-c]pyran-7-carbonsäure;
(1*S*,4a*R*,6a*S*,7*R*,8*R*,10a*R*,10b*R*,12a*R*,14*R*,15*R*)-15-(2-Amino-2-ethylbutoxy)-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-(2-Amino-2-ethylbutoxy)-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-15-[(4-aminotetrahydro-2*H*-pyran-4-yl)methoxy]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminote-trahydro-2*H*-pyran-4-yl)methoxy]-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-[(methylamino)carbonyl]-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminote-trahydro-2*H*-pyran-4-yl)methoxy]-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S,*4a*R*,6a*S*,7*R*,8*R*,10a*R*,10b*R*,12a*R*,14*R*,15*R*)-8-[(1*R*)-1,2-Dimethylpropyl]-14-[5-[[(1-methylethyl)amino]carbonyl]-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(2*R*)-2-Amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(5-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(3-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-(3-pyridinyl)-1*H*-1,2,4-triazol-1-yl]-1,6,6a,7,8,9,10,10a,10b, 11,12,12a-dodecahydro-1,6a,8,10a-tetramethyl-4*H*-1,4a-propano-2*H-*phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[-1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-(2-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-(5-pyrazinyl-1*H*-1,2,4-triazol-1-yl)-1,6,6a,7,8,9,10,10a,10b,11,12,12a-dodecahydro-1,6a,8,10a-tetramethyl-4*H*-1,4a-propano-2*H-*phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(4-pyridazinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(3-pyridazinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(5-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(2-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-14-[5-(3-fluor-4-pyridinyl)-1*H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(3-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(4-pyridazinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S,*4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(3-fluor-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-14-[5-(3-chlor-4-pyridinyl)-1*H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-(2-Amino-2-methylpropoxy)-8-[(1*R*)-1,2-dimethylpropyl]-14-[5-(4-pyridinyl)-1*H-*1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-(2-Amino-2-ethylbutoxy)-8-[(1*R*)-1,2-dimethylpropyl]-14-[5-(4-pyridinyl)-1*H-*1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-15-[2-ethyl-2-(methylamino)butoxy]-14-[5-(4-pyridi-nyl)-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S,*4a*R*,6a*S*,7*R*,8*R*,10a*R*,10b*R*,12a*R*,14*R*,15*R*)-15-[[(2*S*)-2-Amino-2,3-dimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2-(1-methylcyclopropyl)propyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-(2*R*)-2-(Dimethylamino)-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-15-[[(2*R*)-2-(ethylamino)-2,3,3-trimethylbutyl]oxy]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[(2*R*)-2,3,3-trimethyl-2-(propylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(3-fluor-4-pyridinyl)-1*H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2R)-2-Amino-2-cyclopropylpropyl]oxy]-8-[(1*R*)-1,2-dimethylpropyl]-14-[5-(3-fluor-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminote-trahydro-2*H*-pyran-4-yl)methoxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S,*1*R*,8*R*,10a*R*,10b*R*,12a*R*,14*R*,15*R*)-8-[(1*R*)-1,2-Dime-thylpropyl]-14-[5-(3-pyridinyl)-1*H*-1,2,4-triazol-1-yl]-15-[[te-trahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S,*4a*R*,6a*S*,7*R*,8*R*,10a*R*,10b*R*,12a*R*,14*R*,15*R*)-15-[(4-Aminote-trahydro-2*H*-pyran-4-yl)methoxy]-8-[(1*R*)-1,2-dimethylpropyl]-14-[5-(3-fluor-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(3-fluor-4-pyridinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminote-trahydro-2*H*-pyran-4-yl)methoxyl-8-[(1*R*)-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminote-trahydro-2*H*-pyran-4-yl)methoxy]-8-[(1*R*)-1,2-dimethylpropyl]-14-[5-(5-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyrimidinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dime-thylpropyl]-14-[5-(5-pyrimidinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(1-oxido-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(1-oxido-3-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(1-oxido-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
und pharmazeutisch annehmbare Salze davon.

11. Verbindung gemäß Anspruch 1, wobei die Verbindung ausgewählt ist aus der Gruppe bestehend aus:
(1*S*,4a*R*,6a*S*,7*R*,8*R*,10a*R*,10b*R*,12a*R*,14*R*,15*R*)-15-(2*R*)-2,3-Di-methyl-2-(methylamino)butyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-15-[[(2*R*)-2-amino-2,3,3-trimethyl-butyl]oxy]-8-[(1*R*)-1,2-dimethylpropyl]-1,6,6a,7,8,9,10,10a,10b,11,12,12a-dodecaydro-1,6a,8,10a-tetrame-thyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-[(methylamino)carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(5-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(3-pyridinyl)-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-(3-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(5-pyrimidinyl)-1*H*-1,2,4-triazol-1-yl]-1,6,6a,7,8,9,10,10a,10b,11-,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-14-[5-(3-fluor-4-pyridinyl)-1*H*-1,2,4-triazol-1-yl]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(3-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(3-fluor-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-14-[5-(3-chlor-4-pyridinyl)-1*H*-1,2,4-triazol-1-yl]-8-[(1*R*)-1,2-dimethylpropyl]-1,6,6a,7,8,9,10,10a,10b,11,12,12a-dodec:ahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-(2-Amino-2-methylpropoxy)-8-[(1*R*)-1,2-dimethylpropyl]-14-[5-(4-pyridinyl)-1H-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-(2-Amino-2-ethylbutoxy)-8-[(1R)-1,2-dimethylpropyl]-14-[5-(4-pyridinyl)-1*H-*1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-15-[2-ethyl-2-(methylamino)butoxy]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2-(1-methylcyclopropyl)propyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2R)-2-(Dimethylamino)-2,3,3-trimethylbutyl]oxy]-8-[(1R)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-15-[[(2*R*)-2-(ethylamino)-2,3,3-trimethylbutyl]oxy]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[(2*R*)-2,3,3-trimethyl-2-(propylamino)butyl]oxy]-1,6,6a,7,8,9,10,10a,10b,11,12,12a-dodecahydro-1,6a,8,10a-tetramethyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(3-fluor-4-pyridinyl)-1*H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2-cyclopropylpropyl]oxy]-8-[(1*R*)-1,2-dimethylpropyl]-14-[5-(3-fluor-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminotetrahydro-2*H*-pyran-4-yl)methoxyl-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(3-pyridinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminotetrahydro-2*H*-pyran-4-yl)methoxy]-8-[(1*R*)-1,2-dimethylpropyl]-14-[5-(3-fluor-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(3-fluor-4-pyridinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminotetrahydro-2*H*-pyran-4-yl)methoxy]-8-[(1*R*)-1,2-dimethylpropyl]-14-[5-(5-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[(4-Aminote-trahydro-2*H*-pyran-4-yl)methoxy]-8-[(1*R*)-1,2-dimethylpropyl]-14-[5-(4-pyrimidinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2H-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyrimidinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(5-pyrimidinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(1-oxido-4-pyridinyl)-1*H*-1,2,4-triazol-1-yl]-1,6,6a,7,8,9,10,10a, 10b,11,12,12a-dodecahydro-1,6a,8,10a-tetramethyl-4*H*-1,4a-propa-no-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(1-oxido-3-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(1-oxido-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
und pharmazeutisch annehmbare Salze davon.

12. Verbindung gemäß Anspruch 1, wobei die Verbindung ausgewählt ist aus der Gruppe bestehend aus:
(1*S*,4a*R*,6a*S*,7*R*,8*R*,10a*R*,10b*R*,12a*R*,14*R*,15*R*)-15-[[(2*R*)-2,3-Dimethyl-2-(methylamino)butyl]oxy]-8-[(1*R*)-1,2-dimethylpropyl]-14-[5-[[(1-methylethyl)amino]carbonyl]-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-14-[5-(Aminocarbonyl)-1*H*-1,2,4-triazol-1-yl]-15-[[(2*R*)-2-amino-2,3,3-trimethyl-butyl]oxy]-8-[(1*R*)-1,2-dimethylpropyl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1.,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[(2*R*)-2,3,3-trimethyl-2-(methylamino)butyl]oxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-8-[(1*R*)-1,2-Dimethylpropyl]-14-[5-(4-pyridinyl)-1*H*-1,2,4-triazol-1-yl]-15-[[tetrahydro-4-(methylamino)-2*H*-pyran-4-yl]methoxy]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(3-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3-dimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*, 4a*R*, 6a*S*, 7*R*, 8*R*, 10a*R*, 10b*R*, 12a*R*, 14*R*, 15*R*) -15- [[(2*R*)-2, 3-Di-methyl-2-(methylamino)butyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(3-fluor-4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
(1*S*,4a*R*,6a*S*,7*R*,8*R*,10a*R*,10b*R*,12a*R*,14*R*,15*R*)-15-[[(2*R*)-2-Amino-2-(1-methylcyclopropyl)propyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure;
und pharmazeutisch annehmbaren Salzen davon.

13. Verbindung gemäß Anspruch 1, wobei die Verbindung (1*S*,4a*R*,6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetra-methyl-4*H*-1,4a-propano-2*H*-phenanthro[1,2-c]pyran-7-carbonsäure oder ein pharmazeutisch annehmbares Salz davon ist.

14. Pharmazeutische Zusammensetzung, welche die Verbindung nach einem der Ansprüche 1 bis 13 und einen pharmazeutisch annehmbaren Träger, Adjuvans oder Vehikel umfasst.

15. Zusammensetzung gemäß Anspruch 14, welche ferner ein zweites therapeutisches Mittel umfasst.

16. Verbindung nach einem von Ansprüchen 1 bis 13 zur Verwendung in der Behandlung einer Pilzinfektion in einem Patienten, der dessen bedarf.

17. Verbindung zur Verwendung gemäß Anspruch 16, wobei besagte Pilzinfektion durch die Pilze *Cryptococcus spp., Candida spp.* oder *Aspergillus spp.* verursacht wird.

18. Verwendung einer Verbindung nach einem von Ansprüchen 1 bis 13 in der Herstellung eines Medikaments zum Behandeln einer Pilzinfektion.

19. Zusammensetzung nach Anspruch 15, worin das zweite therapeutische Mittel ein Azol, ein Polyen, ein Purin oder Pyrimidin-Nukleotid-Inhibitor, ein Pneumocandin oder Echinocandin-Derivat, ein Protein-Verlängerungsfaktor-Inhibitor, ein Chitin-Inhibitor, ein Mannan-Inhibitor, ein bakterizides oder Permeabilität herbeiführendes (BPI) Proteinprodukt oder ein immunmodulierendes Mittel ist.

20. Zusammensetzung nach Anspruch 19, worin das zweite therapeutische Mittel Itraconazol, Ketoconazol, Miconazol, Fluconazol, Voriconazol, Posaconazol, Amphotericin B, Flucytosin, Anidulafungin, Micafungin oder Caspofungin ist.

21. Verbindung zur Verwendung nach Anspruch 16, wobei die Verbindung nach einem von Ansprüchen 1 bis 13 in Kombination entweder aufeinander folgend oder gleichzeitig mit einem zweiten therapeutischen Mittel verabreicht wird, welches wirksam gegen Pilzinfektionen oder bakterielle Infektionen ist.

22. Verbindung zur Verwendung nach Anspruch 21, wobei das zweite therapeutische Mittel ein Azol, ein Polyen, ein Purin oder Pyrimidin-Nukleotid-Inhibitor, ein Pneumocandin oder Echinocandin-Derivat, ein Protein-Verlängerungsfaktor-Inhibitor, ein Chitin-Inhibitor, ein Mannan-Inhibitor, ein bakterizides oder Permeabilität herbeiführendes (BPI) Proteinprodukt oder ein immunmodulierendes Mittel ist.

23. Verbindung zur Verwendung nach Anspruch 22, wobei das zweite therapeutische Mittel Itraconazol, Ketoconazol, Miconazol, Fluconazol, Voriconazol, Posaconazol, Amphotericin B, Flucytosin, Anidulafungin, Micafungin oder Caspofungin ist.

## Revendications

1. Composé de formule (I) : 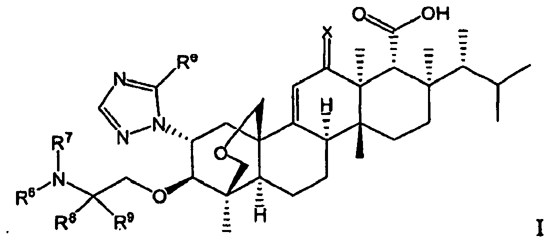 ou sel pharmaceutiquement acceptable de celui-ci, dans laquelle :
X est H, H ; ou O ;
R^{e} est C(O)NR^{f}R^{g} ou un groupe hétéroaryle à cycle à six chaînons contenant 1 ou 2 atomes d'azote où le groupe hétéroaryle est éventuellement mono-substitué sur l'atome de carbone du cycle par un groupe fluoro ou chloro ou sur un atome d'azote du cycle par un atome d'oxygène ;
R^{f}, R^{g}, R⁶ et R⁷ sont chacun indépendamment un atome d'hydrogène ou un groupe alkyle en C₁-C₃ ;
R⁸ est un groupe alkyle en C₁-C₄, cycloalkle en C₃-C₄ ou (cycloalkyl en C₄-C₅)-alkyle ;
R⁹ est un groupe méthyle ou éthyle ; et
R⁸ et R⁹ sont éventuellement pris conjointement pour former un cycle saturé à 6 chaînons contenant 1 atome d'oxygène.

2. Composé selon la revendication 1, dans lequel X est H, H.

3. Composé selon la revendication 1 ou la revendication 2, dans lequel R^{e} est un groupe pyridyle ou pyrimidinyle éventuellement mono-substitué sur un atome de carbone du cycle par un groupe fluoro ou chloro ou sur un atome d'azote du cycle par un atome d'oxygène.

4. Composé selon la revendication 3, dans lequel R^{e} est un groupe 4-pyridyle.

5. Composé selon la revendication 1 ou la revendication 2, dans lequel R^{e} est C(O)NH₂ ou C(O)NHalkyle en C₁-C₃.

6. Composé selon la revendication 5, dans lequel R^{e} est C(O)NH₂.

7. Composé selon l'une quelconque des revendications 1 à 6, dans lequel R⁸ est un groupe alkyle en C₁-C₄ et R⁹ est un groupe méthyle.

8. Composé selon la revendication 7, dans lequel R⁸ est un groupe t-butyle.

9. Composé selon l'une quelconque des revendications 1 à 8, dans lequel R⁶ et R⁷ sont chacun indépendamment un atome d'hydrogène ou un groupe méthyle.

10. Composé selon la revendication 1, dans lequel le composé est choisi dans le groupe constitué de :
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-[[(1-méthyléthyl)amino]carbonyl]-1H-1,2,4-triazol-1-yl]-1,6,6a,7,8,9,10,10a,10b,11,12, 12a-dodécahydro-1,6a,8,10a-tétraméthyl-4H-1,4a-propano-2H-phénanthro[12-c]pyran-7-carboxylique ;
l'acide (1,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[[(1-méthyléthyl)-amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[[(1-méthyléthyl)-amino]carbonyl]-1H-1,2,4-triazol-1-yl]-1,6,6a,7,8,9,10,10a,10b,11,12,12a-dodécahydro-1,6a,8,10a-tétraméthyl-6-oxo-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[[(1-méthyléthyl)-amino]carbonyl]-1H-1,2,4-triazol-1-yl]-1,6,6a,7,8,9,10,10a,10b,11,12,12a-dodécahydro-1,6a,8,10a-tétraméthyl-6-oxo-4H-1,4a-propano-2H-phénanthro[1,2-c]pyran-7-carboxylique ;
l'acide (1S,4aR,6aS,7R,8R,10aR,10bR,12aR,14R,15R)- 14-[5-(aminocarbonyl)-1H-1,2,4-triazol-1-yl]-15-[[(2R)-2-amino-2,3-diméthylbutyl]oxy]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-(aminocarbonyl)-1H-1,2,4-triazol-1-yl]-15-[[(2R)-2-(diméthylamino)-2,3-diméthylbutyl]oxy]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-(aminocarbonyl)-1H-1,2,4-triazol-1-yl]-8-[(1R)-1,2-diméthylpropyl]-15-[[(2R)-2-(éthylamino)-2,3-diméthylbutyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-(aminocarbonyl)-1H-1,2,4-triazol-1-yl]-15-[[(2R)-2-amino-2,3,3-triméthylbutyl]oxy]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-[(méthylamino)carbonyl]-1H-1,2,4-triazol-1-yl]-15-[[(2R)-2,2,3,-triméthyl-2-(méthylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-(aminocarbonyl)-1H-1,2,4-triazol-1-yl]-8-[(1R)-1,2-diméthylpropyl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-(aminocarbonyl)-1H-1,2,4-triazol-1-yl]-8-[(1R)-1,2-diméthylpropyl]-15-[[(2R)-2,3-diméthyl-2-(méthylamino)butyl]oxy] -1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-(aminocarbonyl)-1H-1,2,4-triazol-1-yl]-8-[(1R)-1,2-diméthylpropyl]-15-[[(2R)-2,3-diméthyl-2-(propylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-14-[5-[(diméthylamino)carbonyl]-1H-1,2,4-triazol-1-yl]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-(diméthylamino)-2,3-diméthylbutyl]oxy]-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-15-[[(2R)-2,3,-diméthyl-2-(propylamino)butyl]oxy]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-[(diméthylamino)carbonyl]-1H-1,2,4-triazol-1-yl]-15-[[(2R)-2,3-diméthyl-2-(méthylamino)butyl]oxy]-8-[(1R)-1,2-diméthylpropyl]- 1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-14-[5-[(diméthylamino)carbonyl]-1H-1,2,4-triazol-1-yl]-8-[(1R)-1,2-diméthylpropyl]-15-[[(2R)-2-(éthylamino)-2,3-diméthylbutyl]oxy]- 1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-[(éthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[(éthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-14-[5-[(diéthylamino)carbonyl]-1H-1,2,4-triazol-1-yl]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-[(1-propylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-(diméthylamino)-2,3-diméthylbutyl]oxy]-8-[(1R)-1,2-diméthylpropyl]-14-[5-[[(1-méthyléthyl)aminolcarbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-15-[[(2R)-2-(éthylamino)-2,3,-diméthylbutyl]oxy]-14-[5-[[(1-méthyléhyl)amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-15-[[(2R)-2,3-diméthyl-2-(propylamino)butyl]oxy]-14-[5-[[(1-méthyléhyl)amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)- 14-[5-(aminocarbonyl)-1H-1,2,4-triazol-1-yl]-8-[(1R)-1,2-diméthylpropyl]-15-[[(2R)-2,3,3-trimméthyl-2-(méthylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-14-[5-[(diméthylamino)carbonyl]-1H-1,2,4-triazol-1-yl]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[(éthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[[(1-méthyléthyl)amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-[[(1-méthyléthyl)amino]carbonyl]-1H-1,2,4-triazol-1-yl]-15-[[(2R)-2,2,3,-triméthyl-2-(méthylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)- 14-[5-(aminocarbonyl)-1H-1,2,4-triazol-1-yl]-15-(2-amino-2-éthylbutyloxy)-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-(2-amino-2-éthylbutyloxy)-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-(2-amino-2-éthylbutyloxy)-8-[(1R)-1,2-diméthylpropyl]-14-[5-[(1-méthyléthyl)amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)- 14-[5-(aminocarbonyl)-1H-1,2,4-triazol-1-yl]-15-[(4-aminotétrahydro-2H-pyran-4-yl)méthoxy]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-[(méthylamino)carbonyl]-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-[[(1-méthyléthyl)amino]carbonyl]-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[(2R)-2,3,3-triméthyl-2-(méthylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(5-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-(3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[(2-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-(5-pyrazinyl-1H-1,2,4-triazol-1-yl)-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(4-pyridazinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(3-pyridazinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(4-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(5-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(2-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-14-[5-(3-fluoro-4-pyridinyl)-1H-1,2,4-triazol-1-yl]-8-[(1R)-1,2-diméthylpropyl]- 1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(4-pyridazinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(4-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(3-fluoro-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-14-[5-(3-chloro-4-pyridinyl)-1H-1,2,4-triazol-1-yl]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-(2-amino-2-méthylpropoxy)-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-(2-amino-2-éthylbutoxy)-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-15-(2-éthyl-2-(méthylamino)butoxy]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2S)-2-amino-2,3-diméthylbutyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-clpyran-7-carboxylique ;
l'acide (1S,4aR,6aS,7R,8R,10aR,10bR,12aR,14R,15R)-15-[[(2R)-2-amino-2-(1-méthylcyclopropyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-(diméthylamino)-2,3,3-triméthylbutyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-15-[[(2R)-2-(éthylamino)-2,3,3-triméthylbutyl]oxy]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[(2R)-2,3,3-triméthyl-2-(propylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(3-fluoro-4-pyridinyl)-1H-1,2,4-triazol-1-yl]-15-[[(2R)-2,3,3-triméthyl-2-(méthylamino)butyl]oxy] - 1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2-cyclopropylpropyl]oxy]-8-[(1R)-1,2-diméthylpropyl]-14-[5-(3-fluoro-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(3-pyridinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(3-fluoro-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(3-fluoro-4-pyridinyl)-1H-1,2,4-triazol-1-yl]-15-[[tétrahydro-4-(méthylamino)-2H-pyran-4-yl]méthoxy] - 1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(4-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(5-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyrimidinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(5-pyrimidinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(1-oxydo-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(1-oxydo-3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(1-oxydo-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ; et
les sels pharmaceutiquement acceptables de celui-ci.

11. Composé selon la revendication 1, dans lequel le composé est choisi dans le groupe constitué de :
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[[(1-méthyléthyl)-amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)- 14-[5-(aminocarbonyl)-1H-1,2,4-triazol-1-yl]-15-[[(2R)-2-amino-2,3,3-triméthylbutyl]oxy]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[(méthylamino)carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[[(1-méthyléthyl)amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[(2R)-2,3,3-triméthyl-2-(méthylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(5-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutylloxyl-8-[(1R)-1,2-diméthylpropyll-14-[5-(3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-(3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(4-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(5-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-14-[5-(3-fluoro-4-pyridinyl)-1H-1,2,4-triazol-1-yl]-8-[(1R)-1,2-diméthylpropyl]- 1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(4-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(3-fluoro-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-14-[5-(3-chloro-4-pyridinyl)-1H-1,2,4-triazol-1-yl]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-(2-amino-2-méthylpropoxy)-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-(2-amino-2-éthylbutoxy)-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-15-(2-éthyl-2-(méthylamino)butoxy]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2-(1-méthylcyclopropyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-(diméthylamino)-2,3,3-triméthylbutyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-15-[[(2R)-2-(éthylamino)-2,3,3-triméthylbutyl]oxy]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[(2R)-2,3,3-triméthyl-2-(propylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(3-fluoro-4-pyridinyl)-1H-1,2,4-triazol-1-yl]-15-[[(2R)-2,3,3-triméthyl-2-(méthylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2-cyclopropylpropyl]oxy]-8-[(1R)-1,2-diméthylpropyl]-14-[5-(3-fluoro-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(3-pyridinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(3-fluoro-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(3-fluoro-4-pyridinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(5-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[(4-aminotétrahydro-2H-pyran-4-yl)méthoxy]-8-[(1R)-1,2-diméthylpropyl]-14-[5-(4-pyrimidinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyrimidinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-clpyran-7-carboxylique ;
l'acide (1S,4aR,6aS,7R,8R,10aR,10bR,12aR,14R,15R)-8-[(1R)-1,2-diméthylpropyl]-14-[5-(5-pyrimidinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(1-oxydo-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(1-oxydo-3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(1-oxydo-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ; et
les sels pharmaceutiquement acceptables de celui-ci.

12. Composé selon la revendication 1, dans lequel le composé est choisi dans le groupe constitué de :
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-[[(1-méthyléthyl)-amino]carbonyl]-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)- 14-[5-(aminocarbonyl)-1H-1,2,4-triazol-1-yl]-15-[[(2R)-2-amino-2,3,3-triméthylbutyl]oxy]-8-[(1R)-1,2-diméthylpropyl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-[(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[(2R)-2,3,3-triméthyl-2-(méthylamino)butyl]oxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-8-[(1R)-1,2-diméthylpropyl]-14-[5-(4-pyridinyl)-1H-1,2,4-triazol-1-yl]-15-[[tétrahydro-4-(méthylamino)-2H-pyran-4-yl]méthoxy]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(3-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3-diméthylbutyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2,3-diméthyl-2-(méthylamino)butyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2,3,3-triméthylbutyl]oxy]-8-[(1R)-1,2-diméthylpropyl]-14-[5-(3-fluoro-4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ;
l'acide (1S,4aR,6aS,7R,8R,10aR,10bR,12aR,14R,15R)-15-[[(2R)-2-amino-2-(1-méthylcyclopropyl]oxy]-8-[(1R)-1,2-diméthylpropyl]-14-[5-(4-pyridinyl)-1H-1,2,4-triazol-1-yl]-1,6,6a,7,8,9,10,10a,10b,11,12,12a-dodécahydro-1,6a,8,10a-tétraméthyl-4H-1,4a-propano-2H-phénanthro[1,2-c]pyran-7-carboxylique ; et
les sels pharmaceutiquement acceptables de celui-ci.

13. Composé selon la revendication 1, dans lequel le composé est l'acide (1S,4aR,6aS,7R,8R,10aR, 10bR,12aR,14R,15R)-15-[[(2R)-2-amino-2,3,3-triméthyl-butyl]-oxy]-8-[(1R)-1,2-diméthylpropyl]-14-[5-(4-pyridinyl)-1H-1,2,4-triazol-1-yl]-1,6,6a,7,8,9,10,10a, 10b,11,12,12a-dodécahydro-1,6a,8,10a-tétraméthyl-4H-1,4a-propano-2H-phénanthro[1,2-c]pyran-7-carboxylique ou un sel pharmaceutiquement acceptable de celui-ci.

14. Composition pharmaceutique comprenant le composé selon l'une quelconque des revendications 1 à 13 et un support, un adjuvant ou un véhicule pharmaceutiquement acceptable.

15. Composition selon la revendication 14 comprenant en outre un second agent thérapeutique.

16. Composé selon l'une quelconque des revendications 1 à 13, destiné à une utilisation dans le traitement d'une infection fongique chez un patient le nécessitant.

17. Composé destiné à une utilisation selon la revendication 16, dans lequel ladite infection fongique est provoquée par les champignons Cryptococcus spp., Candida spp. ou Aspergillus spp.

18. Utilisation d'un composé selon l'une quelconque des revendications 1 à 13 dans la fabrication d'un médicament pour traiter une infection fongique.

19. Composition selon la revendication 15, dans laquelle le second agent thérapeutique est un azole, un polyène, un inhibiteur de nucléotides de type purine ou pyrimidine, un dérivé de pneumocandine ou d'échinocandine, un inhibiteur du facteur d'allongement d'une protéine, un inhibiteur de chitine, un inhibiteur de mannane, un produit de type protéine bactéricide ou induisant une perméabilité (BPI) ou un agent immunomodulateur.

20. Composition selon la revendication 19, dans laquelle le second agent thérapeutique est l'itraconazole, le kétoconazole, le miconazole, le fluconazole, le voriconazole, le posaconazole, l'amphotéricine B, la flucytosine, l'anidulafungine, la micafungine ou la caspofungine.

21. Composé destiné à une utilisation selon la revendication 16, dans lequel le composé selon l'une quelconque des revendications 1 à 13 est administré en combinaison, séquentiellement ou conjointement, avec un second agent thérapeutique efficace contre des infections fongiques ou bactériennes.

22. Composé destiné à une utilisation selon la revendication 21, dans lequel le second agent thérapeutique est un azole, un polyène, un inhibiteur de nucléotides de type purine ou pyrimidine, un dérivé de pneumocandine ou d'échinocandine, un inhibiteur du facteur d'allongement d'une protéine, un inhibiteur de chitine, un inhibiteur de mannane, un produit de type protéine bactéricide ou induisant une perméabilité (BPI) ou un agent immunomodulateur.

23. Composé destiné à une utilisation selon la revendication 22, dans lequel le second agent thérapeutique est l'itraconazole, le kétoconazole, le miconazole, le fluconazole, le voriconazole, le posaconazole, l'amphotéricine B, la flucytosine, l'anidulafungine, la micafungine ou la caspofungine.
